# EUROPEAN PATENT SPECIFICATION

(11) **EP 2 257 569 B1**
(45) Date of publication and mention of the grant of the patent: **01.10.2014**
(21) Application number: 09720751.8
(22) Date of filing: 13.03.2009
(51) Int. Cl.: C07K 14/47, A61P 1/00, A61P 31/04, C12N 15/85, A01K 67/027, A61P 11/00, A61P 31/10, C12N 15/86, A23C 3/00, A61P 15/14, C07K 19/00, C12N 15/861, A23C 19/097, A61P 27/16, C12N 15/63, C12N 15/869, A61K 38/17, A61P 31/00, C12N 15/82

(54) **Vectors for expression of antimicrobial peptides in mammary gland**
Vektoren zur Expression von antimikrobiellen Peptiden in der Milchdrüsse
Vecteurs pour l'expression de peptides antimicrobiennes dans la glande mammaire

(30) Priority: 13.03.2008 AU 2008901249
(43) Date of publication of application: 08.12.2010
(73) Proprietor: Agriculture Victoria Services PTY Limited, Attwood, VIC 3049 (AU)
(72) Inventor: COCKS, Benjamin, Viewbank, Victoria 3084 (AU); SPANGENBERG, German, Bundoora, Victoria 3083 (AU); WANG, Jianghui, Bundoora, Victoria 3083 (AU)
(74) Representative: Marshall, Cameron John
(86) International application number: PCT/AU2009/000301
(87) International publication number: WO 2009/111838

(56) References cited:
- WO-A1-95/30751
- WO-A1-96/32482
- WO-A1-98/08534
- WO-A1-03/091437
- WO-A1-2007/106951
- WO-A2-2005/033274
- WO-A2-2005/033281
- WO-A2-2005/077046
- WO-A2-2007/142542
- DONOVAN D M ET AL: "Peptidoglycan hydrolase fusions maintain their parental specificities", APPLIED AND ENVIRONMENTAL MICROBIOLOGY, AMERICAN SOCIETY FOR MICROBIOLOGY, US, vol. 72, no. 4, 1 April 2006 (2006-04-01), pages 2988-2996, XP002582772, ISSN: 0099-2240, DOI: DOI:10.1128/AEM.72.4.2988-2996.2006
- BROADBENT J R ET AL: "NISIN INHIBITS SEVERAL GRAM-POSITIVE MASTITIS-CAUSING PATHOGENS", JOURNAL OF DAIRY SCIENCE, AMERICAN DAIRY SCIENCE ASSOCIATION, US, vol. 72, no. 12, 1 January 1989 (1989-01-01), pages 3342-3345, XP009149724, ISSN: 0022-0302
- HOEBEN D ET AL: "Effect of Bovine Somatotropin on Neutrophil Functions and Clinical Symptoms During Streptococcus uberis Mastitis", JOURNAL OF DAIRY SCIENCE, AMERICAN DAIRY SCIENCE ASSOCIATION, US, vol. 82, no. 7, 1 July 1999 (1999-07-01), pages 1465-1481, XP026993631, ISSN: 0022-0302 [retrieved on 1999-07-01]
- SPARO M D ET AL: "In vitro efficacy of the novel peptide CECT7121 against bacteria isolated from mastitic dairy cattle", LETTERS IN APPLIED MICROBIOLOGY, WILEY-BLACKWELL PUBLISHING LTD, GB, vol. 48, no. 2, 1 January 2009 (2009-01-01), pages 187-193, XP007918966, ISSN: 0266-8254, DOI: DOI:10.1111/J.1472-765X.2008.02507.X [retrieved on 2009-01-05]
- ROBERT J WALL ET AL: "Genetically enhanced cows resist intramammary Staphylococcus aureus infection", NATURE BIOTECHNOLOGY, NATURE PUBLISHING GROUP, NEW YORK, NY, US, vol. 23, no. 4, 1 July 2005 (2005-07-01), pages 445-451,897, XP007918965, ISSN: 1087-0156, DOI: DOI:10.1038/NBT1078 [retrieved on 2005-04-03]
- DALY ET AL: "Identification, characterization and expression of cathelicidin in the pouch young of tammar wallaby (Macropus eugenii)", COMPARATIVE BIOCHEMISTRY AND PHYSIOLOGY. PART B, BIOCHEMISTRYAND MOLECULAR BIOLOGY, ELSEVIER,OXFORD, GB, vol. 149, no. 3, 11 January 2008 (2008-01-11), pages 524-533, XP022453792, ISSN: 1096-4959, DOI: DOI:10.1016/J.CBPB.2007.12.002
- WANG J ET AL: "Mammary gland and innate immunity of the tammar wallaby", TISSUE ANTIGENS, MUNKSGAARD, COPENHAGEN, DK, vol. 66, no. 5, 1 January 2005 (2005-01-01), page 583, XP003023789, ISSN: 0001-2815
- RECIO I ET AL: "Protective effect of milk peptides: Antibacterial and antitumor properties", ADVANCES IN EXPERIMENTAL MEDICINE AND BIOLOGY, SPRINGER, US, vol. 606, 1 January 2008 (2008-01-01), pages 271-293, XP009149736, ISSN: 0065-2598
- DONOVAN, D. M. ET AL.: 'Peptidoglycan hydrolase fusions maintain their parental specificities' APPLIED AND ENVIRONMENTAL MICROBIOLOGY vol. 72, no. 4, 2006, pages 2988 - 2996
- BROADBENT, J. R. ET AL.: 'Nisin inhibits several gram-positive, mastitis-causing pathogens' JOURNAL OF DAIRY SCIENCE vol. 72, no. 12, 1989, pages 3342 - 3345
- HOEBEN, D. ET AL.: 'Effect of bovine somatotropin on neutrophil functions and clinical symptoms during Streptococcus uberis mastitis' JOURNAL OF DAIRY SCIENCE vol. 82, no. 7, 1999, pages 1465 - 1481
- SPARO, M. D. ET AL.: 'In vitro efficacy of the novel peptide CECT7121 against bacteria isolated from mastitic dairy cattle' LETTERS IN APPLIED MICROBIOLOGY vol. 48, no. 2, 2009, pages 187 - 192
- WALL, R.J. ET AL.: 'Genetically enhanced cows resist intramammary Staphylococcus aureus infection' NAT. BIOTECHNOL. vol. 23, no. 4, 2005, pages 445 - 451

## Description

### Field of the invention

The present invention relates to antibacterial peptide reagents and methods employing same for the treatment of microbial disease(s), in particular microbial disease(s) mediated in part of whole by one or more bacteria and/or fungi.

### Background of the invention

Human and animal health are valuable commercial sectors and bacterial and fungal pathogenic infections in humans, livestock and domestic pets represent a high cost to these sectors in terms of lost productivity and existing treatment costs. Many bacterial and fungal pathogens of diseases in humans, livestock animals and domestic pets are also recalcitrant to treatment with existing antibiotics, further exacerbating these adverse consequences of infection.

For example, the economic value of the dairy industry worldwide is significant. For example, the International Dairy Foods Association estimates that sales of cow milk in USA alone in 2006 was USD 23.9 billion. This value will be significantly increased when considered on a worldwide scale, and expanded to include all dairy products, e.g., cheese and butter, and to include sales of products from all major animal producers of dairy products, e.g., goats, sheep, camels and buffalo. The pharmaceutical and biotechnology industries have also developed an interest in dairy mammals as suitable bioreactors for the production of biological agents, particularly peptides and proteins. In this respect, the combination of large daily protein output, post-translational processing capabilities, ease of access to recombinant protein by milking and low capital cost of production plants, i.e., farms compared to high volume industrial fermenters makes dairy mammals excellent candidates for the production of recombinant peptides and proteins (Echelard, Curr. Opin. Biotechnol., 7: 536-540, 1996).

Mastitis is currently the most economically important disease of dairy mammals (Pyörälä Reprod. Dom Anim., 37: 211-216, 2002 and Bergonier et al., 34: 689-716, 2003). The annual costs of mastitis in dairy cattle alone is estimated to be about 10% of the total sales of farm milk, i.e., about USD 2 billion in USA alone *(*Radostits et al.,: Veterinary Medicine: A Textbook of the Diseases of Cattle, Sheep, Pigs, Goats and Horses, Ninth Ed., Elsevier Health Sciences, 2000). In this respect, the costs of mastitis extend beyond treatment and prevention costs and include losses in milk production, labor costs and loss of animals due to culling. Other effects of mastitis include the impact of agricultural use of antibiotics that are used to treat mastitis on the development of antibiotic resistant human pathogens (Smith et al., Proc. Natl. Acad. Sci., USA 99: 6434-6439, 2002) and the effect of residues of these antibiotics on human health (Clement Anim. Pharm., 407: 22-23, 1998). Moreover, the limited milk production resulting from mastitis limits the utility of mammals as bioreactors for producing pharmaceutical agents.

Mastitis is an inflammatory reaction of the mammary gland, usually to microbial infection. This condition is characterized by an influx of somatic cells, primarily polymorphonuclear neutrophils (PMN), into the mammary gland, and by an increase in milk protease content (Verdi et al., J. Dairy Sci., 70: 230-242, 1987). Mastitis is generally classified into clinical infections and non-clinical infections. Clinical infections are diagnosed by red, swollen appearance of a mammary gland and flakes or clots (protein aggregates) in the milk. Sub-clinical infections show no obvious symptoms. The majority of cases of mastitis are caused by one or more of *Staphylococcus aureus, Streptococcus dysgalactiae, Streptococcus agalactiae, Streptococcus uberis* or *Escherichia coli.* In this respect, *S. aureus, S. dysgalactiae* and *S. agalactiae* have a contagious route of transmission, whereas *S. uberis* and *E. coli* are environmental pathogens (Kerr and Wellnitz, J. Anim. Sci., 81: 38-47, 2003).

Susceptibility of a mammal to an intra-mammary infection leading to mastitis dramatically increases during early involution and during the periparturient period (Nickerson J. Dairy Sci., 72: 1665-1678, 1989; and Oliver and Sordillo, J. Dairy Sci., 71: 2584-2606, 1988). These infections are often associated with clinical mastitis during early lactation, and can have a marked detrimental effect on subsequent milk yield and/or quality. Susceptibility to mastitis is also high during the prepartum period in first-lactation bovine heifers (Nickerson et al., J. Dairy Sci., 78: 1607-1618, 1995). These infections are associated with a decrease in alveolar epithelial and luminal area and an increase in connective tissue in the mammary gland, potentially causing a lifelong reduction in milk yield in the infected mammal.

The incidence of contagious mastitis has declined over the last thirty years as a result of a five point control plan that recommends use of correctly maintained milking equipment, post-milking teat disinfection, both therapeutic and prophylactic use of antibiotics and culling of persistently infected animals (Bramley and Dodd, J. Dairy Res., 51: 481-512, 1984). Notwithstanding that implementation of this plan has almost eliminated *S. dysgalactiae* and *S*. *agalactiae* from many herds, as discussed *supra* the use of antibiotics is both expensive and may have a detrimental effect on human health. Moreover, *S*. *aureus,* which accounts for 15% to 30% of contagious infections has proven to be resistant to traditional management approaches. In this respect, the cure rate of treatment of *S. aureus* infection is often less than 15%. This reduced cure rate is attributed in part to antibiotic resistant strains of *S. aureus,* and to incomplete penetration of the antibiotics through a mammary gland thereby permitting bacteria to survive within the gland. Moreover, *S. aureus* is able to survive within mammary gland epithelial cells, within which antibiotic concentration is insufficient to cause bacterial cell death (Craven and Anderson J. Dairy Res., 51: 513-523, 1984, and Yancey et al., Eur. J. Clin. Microbiol. Infect. Dis., 10: 107-113, 2991).

Antibiotic treatment is used to treat mastitis caused by environmental pathogens, e.g., *S. uberis,* however these pathogens are often resistant to conventional antibiotics. Moreover, recurrence of infection from environmental reservoirs, e.g., within dairy barns, is a continuing problem (Kerr and Wellnitz, *supra*).

Current therapies for mastitis make use of conventional antibiotics, e.g., β-lactams including penicillins and cephalosporins. Notwithstanding that these antibiotics may be effective in the treatment of some pathogens that cause mastitis, some bacterial pathogens are resistant to these compounds. There is also a risk that ongoing use of these compounds may contribute to emergence of antibiotic resistant human pathogens *(*Smith et al., Proc. Natl. Acad. Sci. USA, 99: 6434-6439, 2002). Moreover, concern that accidental exposure of susceptible consumers of milk products containing traces of the antibiotic may produce drug-induced anaphylaxis has resulted in regulatory-bodies enforcing a post-treatment milk discard period and strict industry surveillance of all milk shipments (Kerr and Wellnitz, *supra*). Clearly, these additional regulatory requirements lead to increased cost in production of dairy products and loss of commercially valuable resources through wastage.

The disadvantages of conventional antibiotics in the treatment and/or prevention of mastitis has lead to the dairy industry investigating alternative sources of therapeutic and/or prophylactic compounds, e.g., using vaccines and immuno-regulatory agents. For example, researchers have immunized animals with live or attenuated bacteria, cell lysates or subunit vaccines comprising one or more cellular components of a bacterium. However, such approaches have met with limited success, e.g., because of a failure to protect against more than a single causative pathogen.

In a further example, *Clostridium difficile* is found in many hospital environments and is a major cause of diarrhoea, hemorrhagic enteritis and abomasitis in humans e.g., undergoing antibiotic therapy. In the US, more people die from *C*. *difficile* infections than all other intestinal infections combined, with most deaths involving patients aged 65 years or over. The disease is believed to have contributed to more than 8,000 deaths in the UK in 2007. The cost of managing the disease worldwide is high. The chronic bowel infection caused by *C*. *difficile* is also very difficult to treat.

In a further example, *Clostridium perfringens* causes clostridial necrotizing enteritis in humans e.g., subjects receiving TNF antagonist therapy (e.g., Enbrel, Humira) for chronic inflammation (essentially immunocompromised people). The disease comprises necrotizing inflammation of the jejunum and ileum. *C*. *perfringens* may also causes other severe inflammatory diseases in the small bowel e.g., the jejunum, wherein inflammation is segmental, involving small or large patches with varying degrees of haemorrhage and necrosis. Perforation of the intestine may also occur in severe cases.

Infections by *C*. *perfringens* are also the most common causes of clostridial hemorrhagic enteritis in neonatal ruminants, and in domestic animals such as felines and canines, especially in animals that have been overfed, fed on barely thawed or contaminated colostrum, or in animals that have decreased gut motility. The gram-positive bacilli are often found on affected mucosa and in sub-mucosa. For example, infected calves may exhibit tympany, hemorrhagic abomasitis, and abomasal ulceration. Infected animals develop pasty yellow and bloody diarrhea, and the abdomen becomes distended and painful. Known therapies are not highly effective and the disease has a high mortality rate.

In a further example, Respiratory Disease in livestock animals is a common cause of illness and death in pigs/swine and cattle, e.g., causing between 50% and 90% of all sickness and deaths in Australian feedlot cattle, and the disease is common in the first four weeks after cattle enter the feedlot. In addition to the costs associated with treatment, wasted feed and cattle deaths, Bovine Respiratory Disease (BRD) causes performance losses due to decreased weight gain and feed conversion efficiency of infected animals. During the transition from a paddock to a feedlot system, feedlot cattle are commonly exposed to a range of stress factors that may depress their immune system. Respiratory disease in livestock animals is caused by a plurality of viral and bacterial pathogens. Exemplary bacterial pathogens associated with respiratory disease in cattle include *Haemophilus somnus, Pasteurella multocida, Mannheimia (Pasteurella) hemolytica* and *Mycoplasma spp.* e.g., *M. bovis.* Exemplary bacterial pathogens associated with respiratory disease in swine (Swine Respiratory Dsease, SRD) include *Actinobacillus pleuropneumoniae, Actinobacillus suis, P. multicoda, Streptococcus suis, Haemophilus parasuis, Bordetella bronchiseptica, Salmonella choleraesuis* and *Mycoplasma hyopneumoniae.* Antimicrobial resistance among bacterial pathogens responsible for BRD and SRD is a serious global problem that complicates the management of infection. For example, resistance to treatment with known antibiotics e.g., tylosin, nalidixic acid, sulfamethozaxole trimethoprim and ampicillin, ranges from 35% to 55%.

In further examples, human and animal inflammatory diseases of the ear such as otitis externa and otitis media caused e.g., by *Proteus spp.,* and/or *Pseudomonas spp.* and/or the yeast/fungus *Malassezia pachydermatis* and/or *Staphylococcus spp.* result in alterations in the normal environment of the canal, including enlargement of the apocrine (cerumen) glands and/or reduced width of the ear canal and/or calcification of auricular cartilage and/or rupture of the tympanum and/or a painful inflammation of the vestibulocochlear nerve. Infections are often mixed with, or due entirely to *Malassezia pachydermatis.* Infection also results in the production of purulent and malodorous exudate. It is a common disease in humans and domestic animals such as felines and canines, especially pendulous-eared canines and animals with stenosis or hirsutism or the ear canal. Current treatments are costly, and include administration of systemic antibiotics e.g., trimethoprim-potentiated sulfonamides, cephalexin, enrofloxacin and clindamycin, and/or systemic antifungals e.g., ketoconazole. Systemic antiinflammatory corticosteroids may also be administered e.g., prednisone, to reduce swelling and/or pain associated with otitis externa. Resistance to such antibiotics may occur.

In a further example, the two most common cutaneous fungal infections in small animals are dermatophytosis and *Malassezia* dermatitis. Malassezia can be found in very large proportion on the skin of diseased animals, especially dogs. It often causes illness together with other pathogens (e.g. *Staphylococcus intermedius*). Some breeds are predisposed. *Malassezia pachydermatis* dermatitis is resistant to treatment with glucocorticoids. In cats, disease produced by *Malassezia* is most often seen as ear infections, severe acne, and generalized redness and scaling. Severe disease may be associated with underlying FIV infection. Dogs with corneal ulcer are more likely to develop *Malassezia pachydermatis* dermatitis which suggests its possible role at least as an aggravating factor in the disease. Current treatments are limited, including topical application of ketoconazole, and terbinafine has been used to decrease itchiness.

WO 2007/106951 describes antimicrobial compositions comprising peptide fragments of tammar wallaby milk proteins.

Wall et al. (Nat Biotechnol. 2005, 23(4):445-51) describes transgenic cows secreting lysostaphin in their milk and studies their susceptibility to *Staphylococcus aureus* infection.

Accordingly, there is a need in the art for suitable alternatives for the treatment (prophylactic and/or therapeutic) of organisms such as those referred to *supra* that are resistant to conventional antibiotic treatment and/or against which conventional antibiotics are not effective e.g., not bacteriostatic or bactericidal and/or for which treatment regimes are limited and there is a potential for resistance to develop and/or for which existing treamtnet regimes are otherwise poorly effective.

There is also a need in the art for suitable therapeutic and prophylactic peptide-based and/or protein-based reagents and methods for the treatment of microbial disease(s) such as one or more infections of the mammary gland (e.g., mastitis) and/or one or more infections of the respiratory system (e.g., respiratory disease in humans, and/or livestock such as pigs and/or cattle, and/or domestic animals such as dogs and/or cats) and/or one or more infections of the digestive system (e.g., clostridial intestinal disease(s) in humans, and/or livestock such as pigs and/or cattle, and/or domestic animals such as dogs and/or cats, including diarrhoea and/or hemorrhagic enteritis and/or abomasitis), and/or one or more infections of the ear(s) (e.g., otitis externa or otitis media) and/or one or more infections of the skin (e.g., dermatophytosis or *Malassezia* dermatitis). In the case of applications in the dairy industry, there is also a desire for such peptide and/or protein-based reagents to have low activity against one or more gut-friendly bacteria e.g., lactobacilli.

There is also a need in the art for suitable therapeutic and prophylactic peptide-based and/or protein-based reagents and methods for the treatment of microbial disease(s) in humans such as one or more infections of the mammary gland (e.g., mastitis) and/or one or more infections of the digestive system (e.g., clostridial intestinal disease(s) such as diarrhoea and/or hemorrhagic enteritis and/or nectrotising enteritis and/or abomasitis), and/or one or more infections of the ear(s) (e.g., otitis externa or otitis media).

There is also a need in the art for suitable therapeutic and prophylactic peptide-based and/or protein-based reagents and methods for the treatment of microbial disease(s) in mammals that produce food products for human consumption such as livestock animals and/or animals that act as bioreactors e.g., pigs and/or cattle and/or poultry, such as one or more infections of the mammary gland (e.g., mastitis) and/or one or more infections of the respiratory system (e.g., bovine respiratory disease and/or swine respiratory disease) and/or one or more infections of the digestive system (e.g., clostridial intestinal disease(s) such as diarrhoea and/or hemorrhagic enteritis and/or abomasitis). In the case of applications in the dairy industry, there is also a desire for such peptide and/or protein-based reagents to have low activity against one or more gut-friendly bacteria e.g., lactobacilli.

There is also a need in the art for suitable therapeutic and prophylactic peptide-based and/or protein-based reagents and methods for the treatment of microbial disease(s) in domesticated mammals such as dogs and/or cats such as one or more infections of the respiratory system (e.g., feline respiratory disease) and/or one or more infections of the ear(s) (e.g., otitis externa or otitis media) or one or more infections of the skin (e.g., dermatophytosis or *Malassezia* dermatitis).

There is also a need in the art for suitable therapeutic and prophylactic peptide-based and/or protein-based reagents and methods for the treatment of microbial disease(s) mediated by *Streptococcus spp.* e.g., S. *uberis* and/or *S. suis* and/or *S. agalactiae.*

There is also a need in the art for suitable therapeutic and prophylactic peptide-based and/or protein-based reagents and methods for the treatment of microbial disease(s) mediated by enterobacteria e.g., *Escherichia coli* and/or *Clostridium difficile* and/or *Clostridium perfringens.*

There is also a need in the art for suitable therapeutic and prophylactic peptide-based and/or protein-based reagents and methods for the treatment of microbial disease(s) mediated by *Staphylococcus spp.* e.g., *S. aureus* and/or *S. schleifen* subsp. coagulans and/or *S*. *schleiferi* and/or *S*. *intermedius* and/or *S*. *epidermis* and/or *S*. *pseudointermedin.*

There is also a need in the art for suitable therapeutic and prophylactic peptide-based and/or protein-based reagents and methods for the treatment of microbial disease(s) mediated by *Pasteurella spp.* e.g., *Mannheimia haemolytica (P. haemolytica)* and/or *P*. *multocida.*

There is also a need in the art for suitable therapeutic and prophylactic peptide-based and/or protein-based reagents and methods for the treatment of microbial disease(s) mediated by *Actinobacillus spp.* e.g., *A. pleuropneumoniae* (APP) and/or *A. suis*

There is also a need in the art for suitable therapeutic and prophylactic peptide-based and/or protein-based reagents and methods for the treatment of microbial disease(s) mediated by *Salmonella choleraesuis.*

There is also a need in the art for suitable therapeutic and prophylactic peptide-based and/or protein-based reagents and methods for the treatment of microbial disease(s) mediated by *Mycoplasma spp.* e.g., *Mycoplasma hyopneumoniae.*

There is also a need in the art for suitable therapeutic and prophylactic peptide-based and/or protein-based reagents and methods for the treatment of microbial disease(s) mediated by *Proteus spp.* e.g., *Proteus mirabili.*

There is also a need in the art for suitable therapeutic and prophylactic peptide-based and/or protein-based reagents and methods for the treatment of microbial disease(s) mediated by *Haemophilus spp.* e.g., *H. parasuis* and/or or *H*. *somnus.*

There is also a need in the art for suitable therapeutic and prophylactic peptide-based and/or protein-based reagents and methods for the treatment of microbial disease(s) mediated by *Pseudomonas spp.*

There is also a need in the art for suitable therapeutic and prophylactic peptide-based and/or protein-based reagents and methods for the treatment of microbial disease(s) mediated by *Malassezia pachydermatis.*

There is also a need in the art for suitable therapeutic and prophylactic peptide-based and/or protein-based reagents and methods for the treatment of microbial disease(s) mediated by *Bordetella spp.* e.g., *B. bronchiseptica.*

The following publications provide conventional techniques of molecular biology. Such procedures are described, for example, in the following texts:
1. Sambrook, Fritsch & Maniatis, Molecular Cloning: A Laboratory Manual, Cold Spring Harbor Laboratories, New York, Third Edition (2001), whole of Vols I, II, and III;
2. DNA Cloning: A Practical Approach, Vols. I and II (D. N. Glover, ed., 1985), IRL Press, Oxford, whole of text;
3. Ausubel et al., Current Protocols in Molecular Biology. Wiley Interscience, ISBN 047 150338, 1987, whole of text;
4. Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; Sproat *et al.,* pp 83-115; and Wu *et al.,* pp 135-151;
5. Nucleic Acid Hybridization: A Practical Approach (B. D. Hames & S. J. Higgins, eds., 1985) IRL Press, Oxford, whole of text;
6. Perbal, B., A Practical Guide to Molecular Cloning (1984);
7. Animal Cell Culture: Practical Approach, Third Edition (John R.W. Masters, ed., 2000), ISBN 0199637970, whole of text;
8. J.F. Ramalho Ortigão, "The Chemistry of Peptide Synthesis" In: Knowledge database of Access to Virtual Laboratory website (Interactiva, Germany);
9. Sakakibara, D., Teichman, J., Lien, E. Land Fenichel, R.L. (1976). Biochern. Biophys. Res. Commun. 73 336-342;
10. Merrifield, R.B. (1963). J. Am. Chem. Soc. 85, 2149-2154.
11. Barany, G. and Merrifield, R.B. (1979) in The Peptides (Gross, E. and Meienhofer, J. eds.), vol. 2, pp. 1-284, Academic Press, New York.
12. Wünsch, E., ed. (1974) Synthese von Peptiden in Houben-Weyls Metoden der Organischen Chemise (Müler, E., ed.), vol. 15, 4th edn., Parts 1 and 2, Thieme, Stuttgart.
13. Bodanszky, M. (1984) Principles of Peptide Synthesis, Springer-Verlag, Heidelberg.
14. Bodanszky, M. & Bodanszky, A. (1984) The Practice of Peptide Synthesis, Springer-Verlag, Heidelberg.
15. Nagy et al eds. "Manipulating the Mouse Embryo", Cold Spring Harbor Laboratory, 3rd Edition, 2002, ISBN 0879695749; and
16. Methods in Molecular Biology: Transgenic Mouse Methods and Protocols (Hofker and Deursen, eds., 2002), Humana Press, NJ, USA.

### Summary of invention

(A) The invention provides an expression construct, or an expression vector or a virus particle comprising said expression construct, wherein said expression construct comprises a nucleic acid encoding a peptide, said peptide comprises an amino acid sequence selected from SEQ ID NOs: 7, 8, 10 to 32 or 36 to 58 and having antimicrobial activity at least against *Streptococcus uberis,* and wherein said nucleic acid is operably linked to a promoter that confers expression on said nucleic acid in a mammary gland or cell or tissue thereof, wherein the promoter is selected from the group consisting of a β-casein gene promoter, a prolactin-inducible mammary specific promoter, an α-lactalbumin gene promoter, a whey acidic protein (WAP) gene promoter and a nuclear factor-κB (NF-κB) responsive promoter.
(B) The invention also provides an isolated genetically-modified non-human cell comprising the expression construct or the expression vector or the virus particle according to paragraph (A) above, preferably wherein the cell is a non-human mammalian cell.
(C) The invention also provides an *ex vivo* method of producing a genetically-modified non-human mammalian cell, said method comprising introducing into a non-human mammalian cell the expression construct or the expression vector according to paragraph (A) above.
(D) The invention also provides a genetically-modified non-human mammal comprising the expression construct or expression vector or virus particle according to paragraph (A) above or a zygote, embryo, offspring or reproductive material thereof comprising said expression construct or expression vector or virus particle.
(E) The invention also provides a method for producing an antimicrobial peptide, said method comprising:
   (i) producing or obtaining a genetically-modified non-human mammal capable of expressing the peptide and wherein said mammal comprises the expression construct, vector or viral particle according to paragraph (A) above; and
   (ii) maintaining the genetically-modified non-human mammal for a time and under conditions sufficient for the antimicrobial peptide to be expressed,
   thereby producing the antimicrobial peptide.
(F) The invention also provides the expression construct or the expression vector or a virus particle according to paragraph (A) above, for use in the treatment or prevention of mastitis and/or an infection in a mammary gland or cell or tissue thereof by:
   (i) *S. uberis;* or
   (ii) *S. uberis* and at least one or more further organism(s) selected from the group consisting of *S*. *aureus, S. agalactiae* and *E. coli.*

In work leading up to the present invention, the inventors sought to identify peptide-based and/or protein-based therapeutic and prophylactic reagents for treatment of bacterial and fungal diseases in humans and animals, especially livestock animals and domestic pets. The inventors were particularly interested in developing peptide-based and protein-based therapeutics for treatment of infections by gram-negative and/or gram-positive bacteria in humans and such animals.

The inventors also sought to produce peptide-based and/or protein-based reagents for the treatment of microbial disease(s) such as one or more infections of the mammary gland (e.g., mastitis) and/or one or more infections of the respiratory system (e.g., respiratory disease in humans, and/or livestock such as pigs and/or cattle, and/or domestic animals such as dogs and/or cats) and/or one or more infections of the digestive system (e.g., clostridial intestinal disease(s) in humans, and/or livestock such as pigs and/or cattle, and/or domestic animals such as dogs and/or cats, including diarrhoea and/or hemorrhagic enteritis and/or abomasitis), and/or one or more infections of the ear(s) (e.g., otitis externa or otitis media) and/or one or more infections of the skin (e.g., dermatophytosis or *Malassezia* dermatitis). For applications in the dairy industry, the inventors sought to produce peptide and/or protein-based reagents having low activity against one or more gut-friendly bacteria e.g., lactobacilli.

The inventors also sought to produce peptide-based and/or protein-based reagents for the treatment of microbial disease(s) in humans such as one or more infections of the mammary gland (e.g., mastitis) and/or one or more infections of the digestive system (e.g., clostridial intestinal disease(s) such as diarrhoea and/or hemorrhagic enteritis and/or nectrotising enteritis and/or abomasitis), and/or one or more infections of the ear(s) (e.g., otitis externa or otitis media).

The inventors also sought to produce peptide-based and/or protein-based reagents for the treatment of microbial disease(s) in mammals that produce food products for human consumption such as livestock animals and/or animals that act as bioreactors e.g., pigs and/or cattle and/or poultry, such as one or more infections of the mammary gland (e.g., mastitis) and/or one or more infections of the respiratory system (e.g., bovine respiratory disease and/or swine respiratory disease) and/or one or more infections of the digestive system (e.g., clostridial intestinal disease(s) such as diarrhoea and/or hemorrhagic enteritis and/or abomasitis). In the case of applications in the dairy industry, there is also a desire for such peptide and/or protein-based reagents to have low activity against one or more gut-friendly bacteria e.g., lactobacilli.

The inventors also sought to produce peptide-based and/or protein-based reagents for the treatment of microbial disease(s) in domesticated mammals such as dogs and/or cats such as one or more infections of the respiratory system (e.g., feline respiratory disease) and/or one or more infections of the ear(s) (e.g., otitis externa or otitis media) or one or more infections of the skin (e.g., dermatophytosis or *Malassezia* dermatitis).

The inventors also sought to produce peptide-based and/or protein-based reagents for the treatment of microbial disease(s) mediated by *Streptococcus spp.* e.g., *S. uberis* and/or *S*. *suis* and/or *S*. *agalactiae.*

The inventors also sought to produce peptide-based and/or protein-based reagents for the treatment of microbial disease(s) mediated by enterobacteria e.g., *Escherichia coli* and/or *Clostridium difficile* and/or *Clostridium perfringens.*

The inventors also sought to produce peptide-based and/or protein-based reagents for the treatment of microbial disease(s) mediated by *Staphylococcus spp.* e.g., *S. aureus* and/or *S. schleifen* subsp. coagulans and/or *S*. *schleiferi* and/or *S. intermedius* and/or *S*. *epidermis* and/or *S*. *pseudointermedin.*

The inventors also sought to produce peptide-based and/or protein-based reagents for the treatment of microbial disease(s) mediated by *Pasteurella spp.* e.g., *Mannheimia haemolytica (P. haemolytica)* and/or *P. multocida.*

The inventors also sought to produce peptide-based and/or protein-based reagents for the treatment of microbial disease(s) mediated by *Actinobacillus spp.* e.g., *A. pleuropneumoniae* (APP) and/or *A. suis*

The inventors also sought to produce peptide-based and/or protein-based reagents for the treatment of microbial disease(s) mediated by *Salmonella choleraesuis.*

The inventors also sought to produce peptide-based and/or protein-based reagents for the treatment of microbial disease(s) mediated by *Mycoplasma spp.* e.g., *Mycoplasma hyopneumoniae.*

The inventors also sought to produce peptide-based and/or protein-based reagents for the treatment of microbial disease(s) mediated by *Proteus spp.* e.g., *Proteus mirabili.*

The inventors also sought to produce peptide-based and/or protein-based reagents for the treatment of microbial disease(s) mediated by *Haemophilus spp.* e.g., *H. parasuis* and/or or *H. somnus.*

The inventors also sought to produce peptide-based and/or protein-based reagents for the treatment of microbial disease(s) mediated by *Pseudomonas spp.*

The inventors also sought to produce peptide-based and/or protein-based reagents for the treatment of microbial disease(s) mediated by *Malassezia pachydermatis.*

The inventors also sought to produce peptide-based and/or protein-based reagents for the treatment of microbial disease(s) mediated by *Bordetella spp.* e.g., *B. bronchiseptica.*

More particularly, the inventors have isolated and/or produced several antimicrobial peptides having activity against at least one agent of mastitis and/or at least one agent of respiratory disease e.g., Bovine Respiratory Disease and/or Swine Respiratory and/or at least one agent of a clostridial intestinal disease and/or at least one agent of otitis externa and/or at least one agent of dermatophytosis and/or at least one agent of *Malassezia* dermatitis.

The peptides of the invention are useful in formulations for administration directly to humans and animals e.g., milk-producing animals and/or for ectopic expression in animals that can be used as bioreactors for their production and/or for ectopic expression in animals to protect them against infection. For example, the peptides may be expressed in one or more cells of the mammary gland (e.g., mammary cells), for example in a non-secretory or secretory form, to thereby provide prophylactic and/or therapeutic protection against the bacterium e.g., by virtue of being secreted into a mammary structure e.g., alveoli and/or lobule and/or duct and/or gland and/or teat and/or alveolar lumen and/or teat canal and/or teat cisterna and/or Furstenberg's rosette and/or by virtue of being secreted in the vicinity of a tissue such as alveolar secretory tissue (e.g., comprising cuboidal cells) and/or alveolar epithelium (e.g., basal membrane) and/or myoepithelium (e.g., comprising contractile myoepithelial cells). By way of example, the antimicrobial peptides of the invention have activity against a plurality of agents of mastitis comprising *S*. *uberis* and a further organism selected from *Escherichia coli, Staphylococcus aureus, Streptococcus agalactiae,* and combinations thereof, including one or two or three or all four of said organisms.

As used herein, the term "ectopic expression" shall be taken to mean expression of a peptide by means of transgenics or transient expression, the only requirement being that the peptide being expressed ectopically is not expressed in or by the cell or tissue in nature.

By "non-secretory form" is meant that an antimicrobial peptide is expressed in a cell such that it is not secreted by the cell and therefore has antimicrobial activity in the cell e.g., to provide a prophylactic or therapeutic benefit against at least one causative agent of mastitis.

By "secretory form" is meant that an antimicrobial peptide is expressed in a cell such that it is secreted by the cell in which it is expressed and therefore has antimicrobial activity outside that cell e.g., to provide a prophylactic or therapeutic benefit against at least one causative agent of mastitis. For example, a protein may be secreted to a structure of the mammary gland in the vicinity of a cell in which it is expressed, or alternatively, outside that vicinity if it is capable of being transported from a site at which it is expressed to a structure of the mammary gland e.g., by the vasculature. The term "vicinity" shall be construed broadly to mean that a secreted peptide is found in sufficient physical proximity to a stated tissue or cell to have antimicrobial bioactivity in a therapeutic and/or prophylactic context.

Structural and functional characterization of the antimicrobial peptides of the present invention identifies sub-classes of peptides suitable for specific formulations or modes of administration to achieve a therapeutic or prophylactic benefit.

For example, the class of antimicrobial peptides described herein are active against one or more mastitis agents at salt concentrations normally found in milk products (e.g., milk, buttermilk, cream, butter and derivatives thereof) and in milk (e.g., fresh milk, pasteurised milk, homogenized milk and combinations and variants thereof such as those variants differing in milk fat composition). Such antimicrobial peptides are particularly useful for a wide range of formulations and for expression in a non-secretory or secretory form to animals at various developmental phases e.g., during prenatal development, prepubertal development, postpubertal development, pregnancy or early or late lactation.

In one example, one or more peptides of the invention has low activity against lactobacilli, especially one or more *Lactis spp.,* in particular one or more organisms selected individually or collectively from the group consisting of: *L. helveticus, L. acidophilus, L. lactis, L. bugaricus* and *L. citrovorum,* and especially *L. acidophilus.* This low activity against lactobacilli suggests utility of the peptides in dairy applications e.g., dairy starter cultures, cheese production or yoghurt production, etc.

By way of example, peptides that retain their antimicrobial activity in milk are generally heat-resistant under conditions used to pasteurise milk and milk products. For example, the peptides can retain their antimicrobial activity following incubation at approximately 56°C for 30 minutes or approximately 70°C for 15 minutes, i.e., temperatures used for pasteurisation of milk and milk products. Such peptides are clearly suitable for being expressed ectopically in mammary glands in a secretable form for subsequent extraction from the milk e.g., prior to, during or following pasteurisation, as a bioactive agent.

As used herein, the term "milk" shall be taken to include fresh milk, pasteurised milk, homogenized milk and combinations and variants thereof such as those variants differing in milk fat composition, and milk products derived from fresh milk, pasteurised milk, homogenized milk and combinations and variants thereof such as those variants differing in milk fat composition.

The term "fresh milk" as used herein means milk that has not been pasteurised so as to kill bacteria normally present in milk.

Bioactive antimicrobial peptides extracted or purified from milk are used in pharmaceutical formulations e.g., for therapeutic and/or prophylactic treatment of infection by any one of a number of organisms against which the peptide is active i.e., not merely mastitis agents. For example, in addition to bioactivity against one or more mastitis agents, the antimicrobial peptide may have activity against any number of gram positive and/or gram negative bacteria and/or a fungus such as, for example one or more *Streptococcus spp.* e.g., *S*. *uberis* and/or *S. suis* and/or *S. agalactiae* and/or one or more enterobacteria e.g., *Escherichia coli* and/or *Clostridium difficile* and/or *Clostridium perfringens* and/or one or more *Staphylococcus spp.* e.g., *S. aureus* and/or *S. schleifen* subsp. coagulans and/or *S*. *schleiferi* and/or *S. intermedius* and/or *S*. *epidermis* and/or *S. pseudointermedin* and/or one or more *Pasteurella spp.* e.g., *Mannheimia haemolytica (P. haemolytica)* and/or *P. multocida* and/or one or more *Actinobacillus spp.* e.g., *A. pleuropneumoniae* (APP) *and*/*or A. suis* and/or one or more *Mycoplasma spp.* e.g., *Mycoplasma hyopneumoniae* and/or *M. bovis* and/or one or more *Proteus spp.* e.g., *Proteus vulgaris* and/or *Proteus mirabili* and/or one or more *Haemophilus spp.* e.g., *H. parasuis* and/or or *H. somnus* and/or one or more *Pseudomonas spp.* e.g., *P. aeruginosa,* and/or *Malassezia pachydermatis* and/or *Salmonella choleraesuis* and/or *B. bronchiseptica.* The accompanying examples also demonstrate efficacy of these peptides against one or more bacteria selected from the group consisting *of S. uberis, S. suis, S. agalactiae, P. aeruginosa, E. coli, S. aureus, S. schleifen* subsp. coagulans, *S. schleiferi, S. epidermis, S. pseudointermedin, Mannheimia haemolytica (P. haemolytica), P. multocida, A. pleuropneumoniae* (APP), *H. somnus, Salmonella choleraesuis* and *B. bronchiseptica.*

The inventors also identified a class of antimicrobial peptides having conditional bioactivity against one or more of the disease agents referred to herein, especially those effective against an agent of mastitis. By "conditional bioactivity" is meant that the antimicrobial activity of the peptide against one or more organisms is reduced or antagonized or partially or completely inhibited when contacted with milk as hereinbefore defined. Such antimicrobial peptides are particularly useful for non-milk formulations and for expression in a non-secretory or secretory form to non-lactating animals e.g., during prenatal development, prepubertal development or postpubertal development or early pregnancy. Such peptides provide the added advantage of safety in humans, because they do not require a separate inactivation process, e.g., (pasteurisation or filtration) prior to human consumption. Accordingly, milk products treated with such peptides are generally less expensive to produce.

The inventors also identified a class of antimicrobial peptides comprising an amino acid sequence selected from the group consisting of:
a) the consensus sequence TKFRNSIX₁X₂RLKNFN (SEQ ID NO: 1), wherein X₁ is a basic amino acid e.g., K or R, and wherein X₂ is N or K; and
b) a sequence having at least about 65% identity to SEQ ID NO: 7.
By way of example, peptides falling within this structural group comprise a sequence selected from the group consisting of SEQ ID NO: 10, SEQ ID NO: 14, SEQ ID NO: 15, SEQ ID NO: 16, SEQ ID NO: 17 and SEQ ID NO: 18. For the purposes of nomenclature, each of SEQ ID NO: 10, SEQ ID NO: 14, SEQ ID NO: 15, SEQ ID NO: 16, SEQ ID NO: 17 and SEQ ID NO: 18 is derived from a base peptide consisting of SEQ ID NO: 7 by mutagenesis as described herein.

The inventors also identified a class of antimicrobial peptides comprising an amino acid sequence selected from the group consisting of:
a) the consensus sequence KRGXG (SEQ ID NO: 2), wherein X is a basic amino acid e.g., R or K, or a non-polar amino acid e.g., L or F; and
b) a sequence having at least about 55% identity to SEQ ID NO: 7 or SEQ ID NO: 8.
By way of example, peptides falling within this structural group comprise a sequence selected from the group consisting of SEQ ID NO: 19, SEQ ID NO: 20, SEQ ID NO: 22 and SEQ ID NO: 31. For the purposes of nomenclature, each of SEQ ID NO: 19, SEQ ID NO: 20 and SEQ ID NO: 22 is derived from a base peptide consisting of SEQ ID NO: 8 by mutagenesis, and SEQ ID NO: 31 is derived from a consensus sequence for antimicrobial C-terminal fragments of cathelicidin proteins as described herein.

The inventors also identified a class of antimicrobial peptides comprising an amino acid sequence selected from the group consisting of:
a) the consensus sequence MVKRGXGE (SEQ ID NO: 3), wherein X is a non-polar amino acid e.g., L or F; and
b) a sequence having at least about 55% identity to SEQ ID NO: 7 or SEQ ID NO: 8.
By way of example, peptides falling within this structural group comprise a sequence selected from the group consisting of SEQ ID NO: 19, SEQ ID NO: 20 and SEQ ID NO: 22.

The inventors also identified a class of antimicrobial peptides comprising an amino acid sequence selected from the group consisting of:
a) the consensus sequence IX₁X₂TLX₃NFX₄X₅, (SEQ ID NO: 4) wherein X₁ and X₃ are each a basic amino acid, e.g., K or R, X₂ and X₄ are each K or N, and X₅ is a non-polar amino acid e.g., F or L; and
b) a sequence having at least about 54% identity to SEQ ID NO: 8.
By way of example, peptides falling within this structural group comprise a sequence selected from the group consisting of SEQ ID NO: 11, SEQ ID NO: 12, SEQ ID NO: 15, SEQ ID NO: 16, SEQ ID NO: 19, SEQ ID NO: 20 and SEQ ID NO: 21. For the purposes of nomenclature, each of SEQ ID NO: 11, SEQ ID NO: 12, SEQ ID NO: 15, SEQ ID NO: 16, SEQ ID NO: 19, SEQ ID NO: 20 is derived from a base peptide consisting of SEQ ID NO: 8 by mutagenesis as described herein.

The inventors also identified a class of antimicrobial peptides comprising an amino acid consensus sequence set forth in SEQ ID NO: 88. For the purposes of nomenclature, the sequence set forth in SEQ ID NO: 88 is derived by alignment of the sequences of antimicrobial peptides set forth in SEQ ID NO: 7 and 8 and derivatives thereof having enhanced antimicrobial activity.

Peptides falling within the parameters of each of these structural classes are produced without undue experimentation, e.g., using standard synthetic techniques for producing peptides, and determining antimicrobial activity e.g., by growth inhibition assay.

The inventors also produced the antimicrobial exemplified by amino acid sequences set forth in SEQ ID Nos: 23-32 by mutagenesis based on a consensus sequence for antimicrobial C-terminal fragments of cathelicidin proteins as described herein.

Of the sequences set forth in SEQ ID Nos: 10-32, sequences set forth in SEQ ID Nos: 10-13 and 23-32 were shown to have enhanced growth inhibition relative to their parent base peptide(s) i.e., SEQ ID NO: 7 and/or SEQ ID NO: 8.

The inventors have also derived retro-analogs and retro-inverso analogs of the sequences set forth in each of SEQ ID Nos: 7-22.

### Specific embodiments

Disclosed herein is an antimicrobial peptide or an analog or derivative thereof, said peptide, analog or derivative having antimicrobial activity against one or more *Streptococcus spp.* e.g., *S. uberis* and/or *S. suis* and/or *S. agalactiae* and/or one or more enterobacteria e.g., *Escherichia coli* and/or *Clostridium difficile* and/or *Clostridium perfringens* and/or one or more *Staphylococcus spp.* e.g., *S. aureus* and/or *S. schleifen* subsp. coagulans and/or *S. schleiferi* and/or *S. intermedius* and/or *S. epidermis* and/or *S*. *pseudointermedin* and/or one or more *Pasteurella spp.* e.g., *Mannheimia haemolytica (P. haemolytica)* and/or *P. multocida* and/or one or more *Actinobacillus spp.* e.g., *A. pleuropneumoniae* (APP) and/or *A. suis* and/or one or more *Mycoplasma spp.* e.g., *Mycoplasma hyopneumoniae* and/or *M. bovis* and/or one or more *Proteus spp.* e.g., *Proteus mirabili* and/or one or more *Haemophilus spp.* e.g., *H. parasuis* and/or or *H. somnus* and/or one or more *Pseudomonas spp.* e.g., *P. aeruginosa,* and/or *Malassezia pachydermatis* and/or *Salmonella choleraesuis* and/or *B. bronchiseptica.*

Also disclosed herein is an antimicrobial peptide or an analog or derivative thereof, said peptide, analog or derivative having antimicrobial activity against one or more bacteria selected from the group consisting of *S. uberis, S. suis, S. agalactiae, P. aeruginosa*, *E. coli, S. aureus, S. schleifen* subsp. coagulans, S. *schleiferi, S. epidermis, S. pseudointermedin, Mannheimia haemolytica (P. haemolytica*), *P. multocida, A. pleuropneumoniae* (APP), *H. somnus, Salmonella choleraesuis* and *B. bronchiseptica.* Peptides having activity against combinations of said bacteria, including peptides having activity against one or two or three or four or five or six or seven or eight or nine or ten or eleven or twelve or thirteen or fourteen or fifteen or sixteen of said organisms in any combination are clearly encompassed by the present disclosure.

Also disclosed herein is an antimicrobial peptide or an analog or derivative thereof, said peptide, analog or derivative having antimicrobial activity against *Streptococcus uberis,* and preferably a plurality of agents of mastitis comprising *S. uberis* and a further organism selected from *S. suis, S. agalactiae, P. aeruginosa, E. coli, S. aureus, S. schleifen* subsp. coagulans, S. *schleiferi, S. epidermis, S. pseudointermedin, Mannheimia haemolytica (P. haemolytica*), *P. multocida, A. pleuropneumoniae* (APP), *H. somnus, Satmonella choleraesuis* and *B. bronchiseptica* and any combinations thereof, including one or two or three or four or five or six or seven or eight or nine or ten or eleven or twelve or thirteen or fourteen or fifteen or all sixteen of said organisms

In another example, one or more antimicrobial peptides of the invention has low activity against lactobacilli, especially one or more *Lactis spp.,* in particular one or more organisms selected individually or collectively from the group consisting of: *L. helveticus, L. acidophilus, L. lactis, L. bugaricus* and *L. citrovorum,* and especially *L. acidophilus.*

As used herein, the term "antimicrobial" shall be taken to mean that the peptide or analog or derivative thereof is capable of killing a microorganism and/or preventing growth of a microorganism, i.e., the peptide has microbicidal activity and/or microbiostatic activity. Methods for determining the antimicrobial activity of a peptide or analog or derivative thereof will be apparent to the skilled artisan and/or described herein. For example, the peptide or analog or derivative is applied to a substrate upon which a microorganism has been previously grown and, after a suitable period of time, the level of growth inhibition and/or cell death of the microorganism is determined. The term "microorganism" encompasses any microscopic organism, preferably S. *uberis* and, optionally includes any other microscopic organism, preferably a microorganism that causes mastitis and/or respiratory disease e.g., Bovine Respiratory Disease and/or Swine Respiratory Disease, and/or clostridial intestinal disease and/or otitis externa and/or dermatophytosis and/or *Malassezia* dermatitis, and preferably a bacterium that causes mastitis and/or respiratory disease e.g., Bovine Respiratory Disease and/or Swine Respiratory Disease, and/or clostridial intestinal disease and/or otitis externa. Exemplary microorganisms in this context are bacteria e.g., gram-positive bacteria or gram-negative bacteria e.g., *S*. *uberis* and/or *S. aureus* and/or *S*. *agalactiae* and/or *E. coli* and/or *S. suis* and/or *P. aeruginosa* and/or *S. schleifen* subsp. coagulans and/or *S*. *schleiferi* and/or *S. epidermis* and/or *S*. *pseudointermedin* and/or *Mannheimia haemolytica (P. haemolytica)* and/or *P. multocida* and/or *A. pleuropneumoniae* (APP) and/or *H. somnus* and/or *Salmonella choleraesuis* and/or *B. bronchiseptica* and any combinations thereof, including one or two or three or four or five or six or seven or eight or nine or ten or eleven or twelve or thirteen or fourteen or fifteen or all sixteen of said organisms.

As used herein, the term "analog" includes a peptide modified by varying the amino acid sequence, e.g., by substituting an amino acid with a naturally-occurring amino acid and/or by substituting an amino acid with a non-naturally occurring amino acid and/or by addition of a naturally-occurring amino acid and/or by addition of a non-naturally occurring amino acid and/or by deleting one or more amino acids. Analogs also include peptidomimetics, e.g., in which one or more peptide bonds have been modified. Preferred analogs include an analog of a peptide as described herein comprising one or more non-naturally-occurring amino acid analogs; an analog of a peptide described herein comprising one or more D-amino acids; an analog of a peptide described herein in which at least one peptide bond is replaced by a non-peptide bond (i.e., a peptidomimetic); an isostere of a peptide as described herein; a retro-peptide analog of a peptide as described herein; and a retro-inverted peptide analog of a peptide as described herein.

As used herein the term "derivative" with reference to a stated peptide shall be taken to include e.g., a fragment or processed form of the stated peptide, a variant or mutant comprising one or more amino acid substitutions, deletions of additions relative to the stated peptide, a fusion protein comprising the stated peptide or a peptide comprising one or more additional non-peptide components relative to the stated peptide e.g., a chemical component, e.g., polyethylene glycol (PEG).

Preferred fusion proteins encompassed by the present disclosure include a fusion protein comprising a peptide, analog or derivative as described herein fused or linked to another peptide, polypeptide or protein, e.g., a fusion protein comprising a plurality of peptides, polypeptides or analogs of the present invention, optionally separated by a protease cleavage site. A further example of a fusion protein comprises one or more peptide, analogs or derivatives fused to a heterologous protein to thereby display the peptide(s) analog(s) and/or derivative(s) within said heterologous protein. Another example of a fusion protein comprises a peptide, analog or derivative as described herein fused or linked to an epitope to facilitate detection or isolation of the peptide. An exemplary epitope is a FLAG epitope or a V5 epitope or an HA epitope. In another example, a fusion protein comprises a plurality of peptides and/or analogs and/or derivatives as described herein.

In one example, the antimicrobial peptide or analog or derivative thereof retains its antimicrobial activity at salt concentrations normally found in a milk product (e.g., milk, buttermilk, cream, butter and derivatives thereof) and/or in milk (e.g., fresh milk, pasteurised milk, homogenized milk and combinations and variants thereof such as those variants differing in milk fat composition). For example, the antimicrobial peptide or analog or derivative thereof has antimicrobial activity at a salt concentration between about 20 mM NaCl and about 100 mM NaCl. Preferably the antimicrobial peptide or analog or derivative thereof has antimicrobial activity at a salt concentration between about 25mM NaCl and about 75mM NaCl. More preferably the antimicrobial peptide or analog or derivative thereof has antimicrobial activity at a salt concentration between about 25mM NaCl and about 50mM NaCl. Even more preferably the antimicrobial peptide or analog or derivative thereof has antimicrobial activity at a salt concentration of about 30mM NaCl.

Alternatively, or in addition, the antimicrobial peptide or analog or derivative thereof has antimicrobial activity following heating to a temperature at which milk or a milk product is generally pasteurized. For example, the antimicrobial peptide or analog or derivative thereof has antimicrobial activity following incubation at approximately 56°C for 30 minutes or approximately 70°C for 15 minutes. In this respect, the level of antimicrobial activity of the peptide or analog or derivative thereof following heating need not be the same as the level of activity before treatment. For example, the level of activity can be enhanced, reduced or approximately the same following heating.

Alternatively, or in addition, the antimicrobial activity of said peptide or analog or derivative thereof is completely or partially reduced or inhibited following heating to a temperature at which milk or a milk product is generally pasteurized. For example, the antimicrobial activity of said peptide or analog or derivative thereof is completely or partially reduced or inhibited following incubation at approximately 56°C for 30 minutes or approximately 70°C for 15 minutes.

Alternatively, or in addition, the antimicrobial activity of the antimicrobial peptide and/or analog and/or derivative is reduced or antagonized or partially or completely inhibited when contacted with milk.

Alternatively or in addition, the antimicrobial peptide or analog or derivative is resistant to a protease expressed and/or active in milk or a mammary gland or cell or tissue thereof.

Alternatively or in addition, the antimicrobial peptide or analog or derivative thereof comprises an amino acid sequence selected individually or collectively from the group consisting of:
a) the consensus sequence TKFRNSIX₁X₂RLKNFN (SEQ ID NO: 1), wherein X₁ is a basic amino acid e.g., K or R, and wherein X₂ is N or K; and/or
b) the consensus sequence KRGXG (SEQ ID NO: 2), wherein X is a basic amino acid e.g., R or K, or a non-polar amino acid e.g., L or F; and/or
c) the consensus sequence MVKRGXGE (SEQ ID NO: 3), wherein X is a non-polar amino acid e.g., L or F; and/or
d) the consensus sequence IX₁X₂TLX₃NFX₄X₅, (SEQ ID NO: 4) wherein X₁ and X₃ are each a basic amino acid, e.g., K or R, X₂ and X₄ are each K or N, and X₅ is a non-polar amino acid e.g., F or L; and/or
e) the consensus sequence set forth in SEQ ID NO: 88,
wherein the amino acid sequence of said antimicrobial peptide is at least about 50% identical to SEQ ID NO: 7 and/or SEQ ID NO: 8.

Alternatively or in addition, the antimicrobial peptide, derivative or analog possesses enhanced antimicrobial activity against one or more bacterial agents, e.g., one or more bacterial agents described herein, compared to an antimicrobial peptide comprising the sequence of SEQ ID NO: 7 or SEQ ID NO: 8 under the same conditions.

Alternatively or in addition, the antimicrobial peptide, derivative or analog comprises an amino acid sequence other than SEQ ID NO: 7 or SEQ ID NO: 8 or SEQ ID NO: 9 hereof.

Alternatively or in addition, the antimicrobial peptide comprises a sequence set forth in any one of SEQ ID NOs: 10-32 or an analog or derivative thereof having antimicrobial activity.

Preferred peptides, and analogs and derivatives comprise a sequence selected from SEQ ID Nos: 10-13 and 23-32, more preferably SEQ ID Nos: 10-13, 24, 25, 28 and 30 and analogs and derivatives thereof.

In one example, the analog is a retro-peptide analog. The skilled artisan will be aware that a retro-peptide analog is a peptide analog in which the sequence of at least two amino acids is reversed. Preferably a retro-peptide analog as described herein is a peptide analog in which the sequence of all amino acids in the analog is reversed. In one example, a retro-peptide analog as described herein comprises a sequence set forth in any one of SEQ ID NOs: 33-58, preferably 36-58, more preferably 36-39 and 49-58, and still more preferably SEQ ID Nos: 36-39, 50, 51, 54 and 56.

In another example, the analog is a retro-inverted peptide analog. The skilled artisan will also be aware that a retro-inverted peptide analog is a peptide analog in which the sequence of at least two amino acids is reversed and those amino acids are D-amino acids. Preferably a retro-inverted peptide analog as described herein is a peptide analog in which the sequence of all amino acids in the analog is reversed and all amino acids in the analog other than glycine are D-amino acids. In one example, a retroinverted-peptide analog as described herein comprises a sequence set forth in any one of SEQ ID NOs: 59-83, preferably 61-83, more preferably 61-64 and 74-83, and still more preferably SEQ ID Nos: 61-64, 75, 76, 79 and 81.

Also disclosed herein is a complex of peptides and/or analogs and/or derivatives of the present invention. Without being bound by theory or mode of action or suggesting that a complex is necessary for performance of the present invention, such a complex is useful for enhancing the antimicrobial activity of an antimicrobial peptide or analog or derivative of the invention. For example, disclosed herein is a complex of the same peptide, analog or derivative. Alternatively, disclosed herein is a complex or aggregate comprising a plurality of different peptides and/or analogs and/or derivatives of the invention. Such complex or aggregate may comprise additional antimicrobial peptides known in the art. Synergistic combinations of two or more peptides, analogs or derivatives of the present invention are alsodisclosed.

Also disclosed herein is a composition comprising an amount of a peptide, analog derivative, fusion protein or complex as described herein and a suitable carrier or excipient. Such compositions may also comprise a plurality of peptides, analogs or derivatives described according to any example hereof, including combinations of peptides, analogs or derivatives wherein the combination provides an additive or non-additive i.e., synergistic, effect against one or more microbial agents compared to the individual peptide, analog or derivate components of the combination. For example, the present invention provides a pharmaceutical composition. Such a composition may take any of a number of forms, such as, for example, a solution (e.g., a spray solution or a pharmaceutical solution, e.g., a nasal spray solution or syrup), an aerosol, a cream, a lotion, a gel or a powder. Suitable compositions will be apparent to the skilled artisan based on the description herein.

Preferably the composition comprises an amount of a peptide and/or analog and/or derivative and/or complex sufficient to kill and/or prevent growth of *S. uberis* and, optionally, further organism selected from *S. aureus* and/or *S. agalactiae* and/or *E. coli* and/or *S*. *suis* and/or *P. aeruginosa* and/or *S. schleifen* subsp. coagulans and/or *S*. *schleiferi* and/or *S. epidermis* and/or *S. pseudointermedin* and/or *Mannheimia haemolytica (P. haemolytica*) and/or *P. multocida* and/or *A. pleuropneumoniae* (APP) and/or *H. somnus* and/or *Salmonella choleraesuis* and/or *B. bronchiseptica* and any combinations thereof, including one or two or three or four or five or six or seven or eight or nine or ten or eleven or twelve or thirteen or fourteen or fifteen or all sixteen of said organisms. Preferably the composition comprises an amount of a peptide and/or analog and/or derivative and/or complex to treat or prevent mastitis and/or respiratory disease e.g., Bovine Respiratory Disease and/or Swine Respiratory Disease, and/or clostridial intestinal disease and/or otitis externa and/or dermatophytosis and/or *Malassezia* dermatitis, and preferably to treat mastitis and/or respiratory disease e.g., Bovine Respiratory Disease and/or Swine Respiratory Disease, and/or clostridial intestinal disease and/or otitis externa. Again, pluralities of peptides, analogs or derivative aredisclosed, including synergistic combinations.

As used herein, the term "suitable carrier or excipient" shall be taken to mean a compound or mixture thereof that is suitable for use in a formulation for administration to a cell, tissue, organ or subject. In one example, the carrier or excipient is suitable for administration to a mammary gland of cell or tissue thereof albeit not necessarily limited in use to that context.

A suitable carrier or excipient may be an "intra-mammary carrier or excipient". In this respect, an "intra-mammary carrier or excipient " is compound or mixture thereof that is described in the art only with reference to a use in a composition for administration to a mammary gland or tissue or cell thereof. Such a carrier or excipient for production of a composition for treatment or prophylaxis of mastitis and/or respiratory disease e.g., Bovine Respiratory Disease and/or Swine Respiratory Disease, and/or clostridial intestinal disease and/or otitis externa and/or dermatophytosis and/or *Malassezia* dermatitis, and preferably mastitis and/or respiratory disease e.g., Bovine Respiratory Disease and/or Swine Respiratory Disease, and/or clostridial intestinal disease and/or otitis externa.

Alternatively, the carrier or excipient is a carrier or excipient for intramammary application". The term "carrier or excipient for intramammary application" shall be taken to mean a compound or mixture thereof that is suitable for application to a mammary gland or cell or tissue thereof, and which may be suitable for use in other contexts.

Also disclosed herein is a solid surface coated with or having adsorbed thereto the peptide and/or analog and/or derivative, complex or composition as described herein. For example, disclosed herein is a bead or implant coated with the peptide and/or analog and/or derivative and/or complex of the invention, e.g., an automatic milking device and/or an intramammary device.

As discussed herein above an antimicrobial peptide and/or analog and/or derivative of the present invention is suitable for ectopic expression in one or more cells of a mammary gland, e.g., to provide prophylactic and/or therapeutic protection against *S*. *uberis* and/or to treat or prevent mastitis and/or to produce the peptide and/or analog and/or derivative, e.g., for therapeutic or prophylactic purposes. Accordingly, the present invention provides means for expressing a peptide and/or analog and/or derivative as described herein in a mammary gland or cell or tissue thereof. An exemplary expression construct comprises nucleic acid encoding a peptide and/or analog and/or derivative as described herein operably linked to a promoter that confers expression on said nucleic acid in a mammary gland or cell or tissue thereof. In one example, the expression construct of the present invention comprises a nucleic acid comprising a sequence set forth in any one of SEQ ID NOs: 84-88, 90 or 91, or alternatively, a sequence capable of encoding any one or more peptides or retro-peptides comprising an amino acid sequence selected from SEQ ID NOs: 7, 8, 10 to 32 or 36 to 58.

As used herein, the term "promoter" is to be taken in its broadest context and includes transcriptional regulatory sequences of a classical genomic gene, including the TATA box which is required for transcription initiation, with or without a CCAAT box sequence and additional regulatory elements (e.g., upstream activating sequences, enhancers and silencers) which confer gene expression in a mammary gland or cell or tissue thereof. A promoter is usually, but not necessarily, positioned upstream, or 5', of a structural gene, upon which it confers expression. Furthermore, the regulatory elements comprising a promoter are usually positioned within 2 kb of the start site of transcription of a gene. Preferred promoters can contain additional copies of one or more specific regulatory elements to further enhance expression and/or alter the spatial expression and/or temporal expression of said nucleic acid. Preferably the promoter preferentially or specifically confers expression on the nucleic acid in a mammary gland or cell or tissue thereof.

As used herein, the term "operably linked to" means positioning a promoter relative to a nucleic acid, e.g., a transgene such that expression of the nucleic acid is controlled by the promoter. For example, a promoter is generally positioned 5' (upstream) to the nucleic acid, the expression of which it controls. To construct heterologous promoter/nucleic acid combinations (i.e., an expression construct of the present invention), it is generally preferred to position the promoter at a distance from the gene transcription start site that is approximately the same as the distance between that promoter and the nucleic acid it controls in its natural setting, i.e., the gene from which the promoter is derived. As is known in the art, some variation in this distance can be accommodated without loss of promoter function.

Suitable methods for linking nucleic acids will be apparent to the skilled artisan and/or described herein and include enzymatic ligation, e.g., T4 DNA ligase, topoisomerase-mediated ligation e.g., using *Vaccinia* DNA topoisomerase I, recombination in *cis* or *trans,* e.g., using a recombinase or by random integration, amplification from one or more primer sequences including primer extension means, amplification from a vector, or chemical ligation, e.g., cyanogen bromide-mediated condensation of nucleic acids.

As used herein, and unless the context requires otherwise, the word "confer" and variations thereof such as "conferring" shall be taken to mean the ability of a promoter, for example in the context of other factors such as DNA conformation and/or cis-acting DNA sequence(s) and/or trans-acting factor(s) and/or signalling pathway(s) and/or transcript structure and/or transcript processing, to produce expression or a pattern of expression of nucleic acid to which the promoter is operably-linked in a mammary gland or cell or tissue thereof, e.g., in response to one or more developmental and/or environmental and/or hormonal and/or other stimuli that would normally elicit the expression or pattern of expression for nucleic acid to which the promoter is operably-connected in its native context.

Preferably the promoter confers expression on the nucleic acid operably-linked thereto at a time at which a mammal is at risk of being infected by a microorganism that causes mastitis and/or is at risk of developing mastitis, e.g., during pregnancy and before lactation commences and/or at the time of or following giving birth, and/or during involution and/or during lactation.

Suitable promoters will be apparent to the skilled artisan and include, for example, a β-casein gene promoter (e.g., comprising a sequence set forth in SEQ ID NO: 92) or a prolactin-inducible mammary specific promoter (e.g., comprising a sequence set forth in SEQ ID NO: 93) or a α-lactalbumin gene promoter (e.g., comprising a sequence set forth in SEQ ID NO: 94) or a whey acidic protein (WAP) gene promoter (e.g., comprising a sequence set forth in SEQ ID NO: 95 or 96 or 97) or a β-lactoglobulin gene promoter (e.g., comprising a sequence set forth in SEQ ID NO: 98 or 99 or 100). Each of these promoters confers expression on a nucleic acid operably linked thereto at least in a mammary epithelial cell at least during lactation. A lactalbumin promoter also confers expression on a nucleic acid linked thereto in at least mammary epithelial cells during pregnancy. In another example, the promoter is a nuclear factor-κB (NF-κB) responsive promoter. In this respect, NF-κB is expressed at increased levels in mammary epithelial cells during mastitis and/or during involution. For example, the promoter is derived from a lactoferrin gene, e.g., a bovine lactoferrin gene. Such a promoter confers expression on a nucleic acid operably-linked thereto in a mammary cell, and this expression is increased during infection, e.g., by a microorganism that causes mastitis.

The expression construct may also comprise one or more intron sequences positioned downstream of the promoter and transcription start site and optionally downstream of a translation start site for the encoded protein to be expressed. The intron sequence may enhance transcript stability. Preferred introns must be capable of being processed form a primary transcript in the target cell or tissue in which the fusion protein is to be expressed and will generally be employed for expressing an antimicrobial peptide, analog or derivative of the invention or a fusion protein comprising same in a eukaryotic cell or tissue. Preferred introns are intron 1 sequences derived from native genomic genes. For example, preferred intron 1 sequences for expression in bovine cells and tissues are described by Mossallam et al., J. App. Sci. 3(11), 1400-1406 (2007).

In one example, an expression construct of the present invention further comprises a sequence encoding a signal peptide that provides for expression of a fusion protein comprising an N-terminal signal peptide and C-terminal antimicrobial peptide, analog or derivative. Preferably, the signal peptide directs secretion of the peptide and/or analog and/or derivative of the invention into milk. Accordingly, the encoded antimicrobial peptide or analog or derivative is in secretable form. Suitable signal peptides will be apparent to the skilled artisan and/or described herein and include an α-1 lactalbumin signal peptide (e.g., comprising a sequence set forth in SEQ ID NO: 101) or a αS-1 casein signal peptide (e.g., comprising a sequence set forth in SEQ ID NO: 102) or a β-lactoglobulin signal peptide (e.g., comprising a sequence set forth in SEQ ID NO: 103). Generally, a sequence encoding a signal peptide is positioned between the promoter sequence and sequence encoding the antimicrobial peptide, analog or derivative.

Alternatively, or in addition, an expression construct of the present invention comprises a sequence encoding a prepro sequence of a cathelicidin protein that when linked in the same reading frame to a sequence encoding an antimicrobial peptide, analog or derivative described according to example hereof provides for expression of a fusion protein comprising an N-terminal prepro sequence and C-terminal antimicrobial peptide, analog or derivative. Preferably, the fusion protein is subsequently processed by cellular protease(s) to release the mature and bioactive antimicrobial peptide, analog or derivative. By expressing the antimicrobial peptide, analog or derivative as a prepro protein, the stability of the encoded peptide, analog or derivative and/or resistance of the encoded peptide, analog or derivative to proteolysis may be enhanced. Suitable prepro sequences of cathelicidin proteins are readily available to the skilled artisan and include milk-expressed or mammary gland expressed proteins from any one of a number of mammals, including the *Macropus eugenii* sequences set forth in SEQ ID Nos: 104 and 105. Any one of the bovine sequences set forth in SEQ ID Nos: 106-111 may also be employed. Generally, a sequence encoding a cathelicidin prepro sequence is positioned between the promoter sequence and sequence encoding the antimicrobial peptide, analog or derivative. When a signal sequence is also present in such a construct, it is preferred to position the protein-encoding elements downstream of a promoter sequence and in a configuration that provides for expression of a fusion protein comprising N-terminal signal peptide and C-terminal antimicrobial peptide, analog or derivative with an intervening cathelicidin prepro sequence. Alternatively, the protein-encoding elements are positioned downstream of a promoter sequence and in a configuration that provides for expression of a fusion protein comprising N-terminal cathelicidin prepro sequence and C-terminal antimicrobial peptide, analog or derivative with an intervening signal peptide. Alternatively, the protein-encoding elements are positioned downstream of a promoter sequence and in a configuration that provides for expression of a fusion protein comprising N-terminal cathelicidin prepro sequence and C-terminal signal peptide with an intervening antimicrobial peptide, analog or derivative.

In another example, the expression construct of the present invention comprises a sequence encoding a fusion protein comprising a plurality of antimicrobial peptides and/or a plurality of analogs thereof and/or a plurality of derivatives thereof. For example, the fusion protein comprises at least about 2 or 3 or 4 or 5 or 6 or 7 or 8 or 9 or 10 antimicrobial peptides and/or analogs and/or derivatives thereof. As with constructs comprising a single copy of an antimicrobial peptide, analog or derivative of the invention, constructs comprising a plurality of peptides, analogs or derivatives may also include one or more sequences encoding signal sequence(s) and/or one or more sequences encoding prepro sequence(s) of cathelicidin protein(s).

It is also within the scope of the present invention for an expression construct to include one or more sequences encoding recognition sites for protease(s) e.g., a serine protease(s) or cysteine protease(s), such as positioned between a sequence encoding a prepro sequence and a sequence encoding the antimicrobial peptide, analog or derivative, or alternatively, positioned between a sequence encoding a signal sequence and a sequence encoding the antimicrobial peptide, analog or derivative, or alternatively, positioned between each sequence encoding an antimicrobial peptide, analog or derivative in a construct comprising a plurality of peptides, analogs or derivatives. Preferred recognition sequences for proteases will not be present in the antimicrobial peptide, analog or derivative moiety of the fusion protein, or if present in that moiety, will not be high affinity site(s) for proteases present in a cell, tissue or other protease-containing environment in which the fusion protein is expressed. For example, a sequence encoding the enterokinase recognition sequence (D-D-D-D-K; SEQ ID NO: 112) and/or a sequence encoding a recognition sequence for a protease selected from furin (R-V-R-R), filaggrin (R-K-R-R), HGF-SF (K-Q-L-R), MT-SP1/matriptase (R-Q-A-R), PAR2 (S-K-G-R) or uPA/urokinase (P-R-F-K) may be employed. For fusion proteins expressed as prepro proteins in milk or with signal sequences targeting the fusion protein or its processed form to milk, it is preferred to employ a sequence encoding a recognition sequence for a protease present in milk e.g., plasmin, plasminogen, plasminogen activator, thrombin, cathepsin D, acid milk protease or aminopeptidase. High affinity recognition sequences for the human serine proteases plasmin, thrombin, factor Xa, plasmin and urokinase plasminogen activator are known in the art e.g., Gosalia et al., Proteomics 5, 1292-1298 (2005). High affinity recognition sequences for the human MT-SP1/ matriptases proteases plasmin, thrombin, factor Xa, plasmin and urokinase plasminogen are also known in the art e.g., Proc. Natl Acas. Sci (USA) 104(14), 5771-5776 (2007). In this manner, following expression of the fusion protein, individual peptides and/or analogs and/or derivatives are released by protease cleavage to thereby exert an antimicrobial activity, e.g., to reduce or prevent or treat mastitis.

Alternatively, each peptide or analog or derivative is separated from another peptide or analog or derivative by a cleavage site of a protease that is not expressed or active in a target cell, tissue or other protease-containing environment in which the fusion protein is expressed e.g., mammary gland or cell or tissue thereof or secretion thereof. In this example, a fusion protein comprising the antimicrobial peptide, analog or derivative and other protein elements e.g., signal sequence and/or prepro sequence separated by protease recognition and cleavage site(s) is isolated from the cell or tissue in which it is expressed milk, and cleaved with the relevant protease recognizing the protease recognition and cleavage site to thereby release bioactive antimicrobial peptides and/or analogs and/or derivatives, e.g., for therapeutic or prophylactic use. For example, TEV protease may be employed in such applications.

In another example, the present invention provides an expression vector comprising an expression construct as described according to any embodiment hereof. The skilled artisan will be aware that in addition to the expression construct of the present invention, an expression vector generally comprises one or more sequences to permit it to be maintained in a cell e.g., one or more selectable marker genes e.g., to confer antibiotic resistance on a cell comprising the expression vector, and one or more origins of replication e.g., for replication in bacterial cells and/or yeast cells. An expression vector may also include one or more recombinase site sequences to permit excision of a portion of its DNA in a cell and/or to facilitate integration into host cell DNA. Expression vectors encompassed by the present invention include a plasmid, bacteriophage, phagemid, cosmid, virus, sub-genomic or genomic fragment, bacterial artificial chromosome, yeast artificial chromosome or other nucleic acid capable of maintaining and or replicating heterologous DNA in an expressible format. Selection of appropriate vectors is within the knowledge of those having skill in the art.

Expression constructs and/or expression vectors as described according to any embodiment hereof are also useful for producing a recombinant cell comprising said expression vector or expression construct. Accordingly, the present invention provides a cell comprising an expression vector or expression vector as described according to any embodiment hereof. In one example, the cell is a zygote or an oocyte or an embryonic cell or a stem cell, e.g., a pluripotent cell.

In another example, the present invention also provides a genetically-modified non-human mammal comprising an expression construct or an expression vector as described according to any embodiment hereof. Preferably said genetically-modified non-human mammal expresses a peptide and/or analog and/or derivative as described herein in a mammary gland or a cell or tissue thereof and/or a secretion of a mammary gland or cell or tissue thereof, e.g., milk.

As used herein, the term "genetically-modified" shall be taken to mean a non-human mammal that comprises genetic material additional to the naturally-occurring nucleic acid within said non-human mammal, i.e., nucleic acid encoding an antimicrobial peptide and/or analog and/or derivative as described herein.

In this respect, the present invention is not to be limited to a non-human mammal comprising the expression construct or expression vector within their genome. Rather, the present invention also encompasses a genetically-modified non-human mammal comprising an episomal expression construct, e.g., within an artificial chromosome or expression vector or viral nucleic acid. The present invention also encompasses a genetically-modified non-human mammal in which a transgene is only within one or more cells within a mammary gland, i.e., either integrated into the genome of the cell within the mammary gland or episomal within the cell within the mammary gland. A genetically-modified non-human mammal comprising an episomal expression construct within a cell of the mammary gland is produced, for example, using a virus and/or by administration of naked DNA to a mammary gland cell. Suitable methods for such administration are described herein.

Preferably the genetically-modified non-human mammal comprises a genetic construct as described according to any embodiment hereof within its genome. However, the present invention is not to be limited by the means of integration of the nucleic acid into the genome of the non-human mammal. For example, the nucleic acid can be randomly integrated into the animal's genome or integrated at a pre-determined site by homologous recombination (e.g., "knocked-in").

As used herein, the term "non-human mammal" shall be taken to refer to any endothermic animal comprising at least one mammary gland, other than a human. Preferably the non-human mammal produces milk that is consumed by humans, e.g., that is collected and distributed for human consumption. For example, the non-human animal is a ruminant mammal. By "ruminant mammal" is meant any artiodactyl mammal (i.e., of the order Artiodactyla) that digests its food in two steps, first by eating raw material and regurgitating a semi-digested form known as cud from within their first stomach (known as the rumen) and again chewing the cud to break down plant matter therein and stimulate digestion. Ruminants include cattle, goats, sheep, camels, alpacas, llamas, giraffes, American Bison, European bison, yaks, water buffalo, deer, wildebeest and antelope. Preferably the non-human mammal is of the suborder Ruminantia.

Preferably a non-human mammal is a bovine mammal, preferably *Bos taurus* or a cattle cross, an ovine mammal, preferably *Ovis aries,* a caprine mammal, preferably *Capra hircus,* a camel, preferably *Camelus dromedarius,* a Bubalis mammal, preferably Bubalus bubalis or a Rangifer mammal, preferably *Rangifer tarandus.* Preferably the non-human mammal is a bovine mammal, preferably *Bos taurus,* an ovine mammal, preferably *Ovis aries,* a caprine mammal, preferably *Capra hircus.* More preferably the non-human mammal is a bovine mammal, preferably *Bos taurus.*

Preferably the genetically-modified non-human mammal expresses a sufficient level of an antimicrobial peptide or an analog or derivative thereof encoded by the expression construct or expression vector in a mammary gland or cell or tissue thereof to kill or prevent growth of *S. uberis* and, optionally one or more additional microorganisms selected *from S. aureus, S. agalactiae* and *E. coli* and combinations thereof in the mammary gland or cell or tissue thereof, and/or to treat or prevent mastitis.

In another example, the present invention additionally provides a zygote or an embryo or an offspring of a genetically-modified non-human mammal as described according to any embodiment hereof, wherein said zygote or embryo or offspring comprises an expression construct or expression vector as described according to any embodiment hereof. In this respect, the present invention contemplates a zygote or embryo or offspring that is homozygous or heterozygous for an expression construct as described according to any embodiment hereof. Furthermore, the present invention contemplates zygotes, embryos or offspring that are inbred (and, as a consequence, substantially isogenic compared to their parents) or outbred. Methods for producing zygotes, embryos or offspring of a genetically-modified non-human mammal, e.g., by breeding or *in vitro* fertilization or intracellular sperm injection will be apparent to the skilled artisan.

In another example, the present invention provides reproductive material from a genetically-modified non-human mammal as described according to any embodiment hereof, wherein said reproductive material comprises an expression construct or expression vector as described according to any embodiment hereof. For example, the reproductive material is an ovary or a part thereof comprising an oocyte or precursor thereof, or an oocyte, or a precursor of an oocyte, or a testis or a part thereof comprising a sperm cell or precursor thereof, or an epididymis comprising a sperm cell, or a sperm cell, or a precursor of a sperm cell.

In another example, the present invention also provides a cell or a tissue derived or isolated from a transgenic animal as described according to any embodiment hereof wherein said cell comprises an expression construct or expression vector of the present invention. For example, the cell is a pluripotent cell or a totipotent cell or a mammary gland cell or a precursor cell of a mammary gland cell.

In another example, the present invention also provides a method for producing a genetically-modified non-human mammal expressing an antimicrobial peptide an/or analog and/or derivative of the present invention in a mammary gland or cell or tissue thereof, said method comprising introducing an expression construct or expression vector as described according to any embodiment hereof into a non-human mammal cell and regenerating or otherwise producing a non-human mammal there from. Preferably the nucleic acid construct is introduced into the genome of a pronucleus of a fertilized oocyte and the oocyte is maintained under conditions sufficient for an animal to be regenerated there from. Alternatively, the cell is a stem cell and said stem cell is maintained under conditions sufficient to regenerate a non-human mammal, e.g., introduced into a non-human mammal blastocyst, which is in turn introduced into a pregnant or pseudo-pregnant non-human mammal and permitted to develop into a non-human mammal. Alternatively, the cell is a somatic cell and the nucleus of said cell or said cell is introduced into an oocyte under conditions for de-differentiation to occur and the resulting cell maintained under conditions sufficient for an embryo to develop, which is in turn introduced into a pregnant or pseudo-pregnant non-human mammal and permitted to develop into a non-human mammal.

In another example, the present invention provides a method for producing a genetically-modified non-human mammal expressing an antimicrobial peptide and/or analog and/or derivative as described herein in a mammary gland or cell or tissue thereof, said method comprising:
(i) providing or obtaining an oocyte or sperm cell comprising an expression construct or expression vector as described according to any embodiment hereof; and
(ii) fertilizing said oocyte with a sperm cell or fertilizing an oocyte with said sperm cell to thereby produce a zygote and maintaining the zygote under conditions sufficient for development of a genetically-modified non-human mammal expressing an antimicrobial peptide and/or analog and/or derivative as described herein in a mammary gland or cell or tissue thereof,
thereby producing a genetically-modified non-human mammal expressing the antimicrobial peptide and/or analog and/or derivative as described herein in a mammary gland or cell or tissue thereof.

For example, the zygote is maintained under conditions sufficient for an embryo to form and the embryo administered or implanted into a uterus of a non-human mammal and permitted to develop into a genetically-modified non-human mammal.

In another example, the present invention provides a method for producing a genetically-modified non-human mammal expressing an antimicrobial peptide and/or analog and/or derivative as described herein in a mammary gland or cell or tissue thereof, said method comprising:
(i) providing or obtaining a zygote or embryo comprising an expression construct or expression vector as described according to any embodiment hereof; and
(ii) maintaining the zygote or embryo under conditions sufficient for development of a genetically-modified non-human mammal expressing an antimicrobial peptide and/or analog and/or derivative as described herein in a mammary gland or cell or tissue thereof,
thereby producing genetically-modified non-human mammal expressing the antimicrobial peptide and/or analog and/or derivative as described herein in a mammary gland or cell or tissue thereof.

In another example, the present invention additionally provides a method for producing a genetically-modified non-human mammal expressing an antimicrobial peptide and/or analog and/or derivative as described herein in a mammary gland or cell or tissue thereof, said method comprising:
(i) providing or obtaining a genetically-modified non-human mammal as described according to any embodiment hereof;
(ii) mating the genetically-modified non-human mammal at (i) with a non-human mammal of the same species to thereby produce offspring of the genetically-modified non-human mammal; and
(iii) identifying or selecting an offspring at (ii) comprising an expression construct or expression vector of the present invention,
thereby producing a genetically-modified non-human mammal expressing the antimicrobial peptide and/or analog and/or derivative as described herein in a mammary gland or cell or tissue thereof.

The method may additionally comprises identifying or selecting an offspring at (ii) and/or (iii) that expresses the antimicrobial peptide and/or analog and/or derivative as described herein in a mammary gland or cell or tissue thereof

As will be apparent from the foregoing, the present invention also provides for use of an expression construct or expression vector or genetically-modified non-human mammal as described according to any embodiment hereof to produce a genetically modified non-human mammal expressing an antimicrobial peptide and/or analog and/or derivative thereof as described herein in a mammary gland or cell or tissue thereof.

In another example, the antimicrobial peptides and/or analogs and/or complex and/or expression constructs and/or expression vectors are useful for preventing or treating bacterial infection in a human or non-human mammal.

In one example, the present invention provides an expression construct and/or expression vector of the present invention for use in a method for treating or preventing mastitis in a mammal e.g., a non-human mammal, said method comprising administering to mammal in need of treatment or prophylaxis an expression construct and/or expression vector of the present invention. Preferably the expression construct and/or expression vector of the present invention is administered for a time and under conditions sufficient to kill and/or prevent growth of *S. uberis* and, optionally, a microorganism selected from *S. aureus, S. agalactiae* and *E. coli* and combinations thereof in a mammary gland or cell or tissue thereof.

In one example, a subject in need of treatment suffers from mastitis and/or is infected with *S. uberis* and/or *S. aureus* and/or *S. agalactiae* and/or *E. coli.* For example, the subject has an increased number of somatic cells e.g., polymorphonuclear neutrophils (PMN) in a mammary gland or secretion thereof e.g., milk, and/or has a red, swollen mammary gland and/or flakes or clots (protein aggregates) in milk compared to a normal and/or healthy subject.

In one example, a subject in need of treatment or prophylaxis is a mammalian subject at risk of developing mastitis, e.g., a mammal in a prepartum period, e.g., in a non-human mammal that is not lactating and/or a lactating human female or other pregnant or lactating mammalian subject.

In a preferred example, the present invention provides an expression construct, expression vector or virus particle of the present invention for use in a method for treating or preventing mastitis in a mammal e.g., a non-human mammal, said method comprising expressing in a mammary gland or a cell or tissue thereof a peptide and/or analog and/or derivative of the present invention in the mammalian subject in need of treatment or prophylaxis. For example, the method comprises administering an expression construct or expression vector of the present invention to a mammary gland or cell or tissue of the subject to thereby express the peptide and/or analog and/or derivative of the present invention. Suitable methods of administration of an expression construct or expression vector will be apparent to the skilled artisan and/or described herein. For example, the expression construct or expression vector is administered by high-pressure jet injection, or a virus, e.g., an adenovirus, comprising an expression construct is administered to a mammary gland of the subject or a or cell or tissue thereof.

In one example, a peptide and/or analog and/or derivative of the present invention is expressed in a non-human mammal by producing or obtaining a genetically-modified non-human mammal as described according to any embodiment hereof, wherein said genetically-modified non-human mammal expresses the antimicrobial peptide or analog or derivative in a mammary gland or cell or tissue thereof. For example, the genetically modified non-human mammal is produced or obtained by performing a method described according to any embodiment hereof.

Preferably the genetically-modified non-human mammal expresses an amount of an antimicrobial peptide or analog or derivative sufficient to kill and/or prevent growth of *S. uberis* and, optionally a microorganism selected from *S. aureus, S. agalactiae, E. coli* and combinations thereof in a mammary gland or a cell or tissue thereof.

In one example, the antimicrobial activity of the antimicrobial peptide and/or analog and/or derivative is reduced or antagonized or partially or completely inhibited when contacted with milk, and said peptide and/or analog and/or derivative is expressed in a cell prior to lactation. For example, nucleic acid encoding such a peptide is operably-linked to a lactalbumin gene promoter or a lactoferrin gene promoter, which confer expression in a mammary gland or cell or tissue thereof prior to lactation. Expression of such a peptide is useful e.g., to prevent mastitis and/or prevent infection by *S. uberis* and/or *S. aureus* and/or *S. agalactiae* and/or *E. coli* prior to commencement of a first lactation. For example, the peptide comprises a sequence set forth in SEQ ID NO: 8.

In another example, the antimicrobial peptide and/or analog and/or derivative retains its activity in milk and/or a milk product and/or other dairy product e.g., cheese starter culture, yoghurt starter culture, cheese or yoghurt.

Expression of such a peptide prior to lactation and/or during lactation is useful for preventing or treating mastitis and/or treating or preventing infection by *S. uberis* and/or *S. aureus* and/or *S. agalactiae* and/or *E. coli.* Such a peptide can also be secreted into milk to treat or prevent mastitis and/or said infection. For example, the peptide comprises a sequence set forth in SEQ ID NO: 7.

In another example, the present invention also provides for use of an expression construct or expression vector as described according to any embodiment hereof to treat or prevent mastitis and/or to treat or prevent an infection in a mammary gland or cell or tissue thereof by *S. uberis* and/or *S. aureus* and/or *S. agalactiae* and/or *E. coli.*

In another example, the present invention also provides for use of an expression construct or expression vector as described according to any embodiment hereof in the manufacture of a medicament to treat or prevent mastitis and/or to treat or prevent an infection in a mammary gland or cell or tissue thereof by *S. uberis* and/or *S. aureus* and/or *S. agalactiae* and/or *E. coli.*

As will be apparent to the skilled artisan based on the description herein, mastitis is associated with reduced milk production in mammals. Because the present invention provides expression constructs, expression vectors and virus particles for use in methods for treating or preventing mastitis, the present invention also provides the same for use in methods for improving milk production in a non-human mammal. Accordingly, the present invention also provides expression constructs, expression vectors and virus particles for use in a process for improving milk production in a non-human mammal, said process comprising performing a method as described according to any embodiment hereof to prevent or treat mastitis in a non-human mammal thereby improving milk production in the genetically-modified non-human mammal.

In another example, the present invention further provides expression constructs, expression vectors and virus particles for use in a process for improving production of a recombinant polypeptide in milk of a genetically-modified non-human mammal, said process comprising performing a method as described according to any embodiment hereof to prevent or treat mastitis in a genetically-modified non-human mammal, wherein said genetically-modified non-human mammal secretes a recombinant peptide or polypeptide into milk produced from its mammary gland(s), thereby improving production of the recombinant polypeptide in milk of a genetically-modified non-human mammal. In this respect, the recombinant peptide or polypeptide can be an antimicrobial peptide or analog or derivative of the present invention.

In another example, the present invention also provides a process for producing an antimicrobial peptide and/or analog and/or derivative as described herein, said process comprising:
(i) producing or obtaining a genetically-modified non-human mammal as described according to any embodiment hereof or producing a genetically-modified non-human mammal by performing a method as described according to any embodiment hereof;
(ii) maintaining the genetically-modified non-human mammal for a time and under conditions sufficient for the antimicrobial peptide and/or analog and/or derivative to be expressed,
thereby producing the antimicrobial peptide and/or analog and/or derivative.

Preferably, the process comprises obtaining or producing a genetically-modified non-human mammal that secretes the antimicrobial peptide and/or analog and/or derivative into milk, and maintaining the genetically-modified non-human mammal for a time and under conditions sufficient for lactation to occur to thereby produce milk comprising said antimicrobial peptide and/or analog and/or derivative.

In another example, the process additionally comprises isolating the antimicrobial peptide and/or derivative and/or analog e.g., from milk. Methods for isolating the antimicrobial peptide and/or analog and/or derivative will be apparent to the skilled artisan and/or described herein.

In another example, the present invention also provides for use of an expression construct or expression vector or genetically-modified non-human mammal as described according to any embodiment hereof to produce the antimicrobial peptide and/or derivative and/or analog of the present invention.

In another example, disclosed herein is a method of producing a dairy product e.g., a fermented dairy product comprising providing an antimicrobial peptide, analog or derivative as described herein to a fermentation culture comprising one or more lactobacilli against which the peptide, analog or derivative has low activity to thereby prevent contamination or infection by a microbe against which the peptide has significant antimicrobial activity. The dairy product may be any dairy product comprising lactobacilli or for which lactobacilli are used in production, e.g., cheese starter culture, yoghurt starter culture, cheese or yoghurt. Other dairy products are not excluded.

As will be apparent to the skilled artisan, an antimicrobial peptide and/or analog and/or derivative as described herein and/or produced by performing a method as described according to any embodiment hereof and/or milk comprising an antimicrobial peptide having bioactivity therein is useful for a variety of purposes in addition to treatment or prevention of mastitis, e.g., to treat or prevent microbial growth and/or to treat or prevent and infection and/or to prolong storage life of a perishable product. Suitable uses for the antimicrobial peptide and/or analog and/or derivative will be apparent to the skilled artisan based on the description herein.

In another example, disclosed herein is a method for treating or preventing respiratory disease e.g., BRD or SRD, in a human or non-human mammal e.g., a bovine or porcine animal, said method comprising administering to mammal in need of treatment or prophylaxis a peptide and/or analog and/or derivative and/or complex and/or expression construct and/or expression vector of the present invention. Preferably the peptide and/or analog and/or derivative and/or complex and/or expression construct and/or expression vector of the present invention is administered for a time and under conditions sufficient to kill and/or prevent growth of *E. coli* and/or *S. suis* and/or *P. aeruginosa* and/or *Mannheimia haemolytica (P. haemolytica*) and/or *P. multocida* and/or *A. pleuropneumoniae* (APP) and/or *H. somnus* and/or *Salmonella choleraesuis* and/or *B. bronchiseptica* and combinations thereof, including one or two or three or four or five or six or seven or eight or nine of said organisms.

In another example, disclosed herein is a method for treating or preventing clostridial intestinal disease in a human or non-human mammal e.g., an immune-compromized subject or subject receiving anti-TNF therapy, said method comprising administering to mammal in need of treatment or prophylaxis a peptide and/or analog and/or derivative and/or complex and/or expression construct and/or expression vector of the present invention. Preferably the peptide and/or analog and/or derivative and/or complex and/or expression construct and/or expression vector of the present invention is administered for a time and under conditions sufficient to kill and/or prevent growth of at least one bacterium of *Clostridium spp.,* e.g., *C*. *difficile* and/or *C*. *perfringens* and combinations thereof

In another example, disclosed herein is a method for treating or preventing otitis externa in a human or non-human mammal e.g., a domestic animal such as a feline or canine, said method comprising administering to mammal in need of treatment or prophylaxis a peptide and/or analog and/or derivative and/or complex and/or expression construct and/or expression vector of the present invention. Preferably the peptide and/or analog and/or derivative and/or complex and/or expression construct and/or expression vector of the present invention is administered for a time and under conditions sufficient to kill and/or prevent growth of *S. aureus* and/or *S*. *schleifen* subsp. coagulans and/or *S. schleiferi* and/or *S. epidermis* and/or *S. pseudointermedin* and/or *P. aeruginosa* and any combinations thereof.

### Definitions

This specification contains nucleotide and amino acid sequence information prepared using PatentIn Version 3.4, presented herein after the claims. Each nucleotide sequence is identified in the sequence listing by the numeric indicator <210> followed by the sequence identifier (e.g. <210>1, <210>2, <210>3, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (e.g. SEQ ID NO: 1 refers to the sequence in the sequence listing designated as <400>1).

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue.

As used herein the term "derived from" shall be taken to indicate that a specified integer may be obtained from a particular source albeit not necessarily directly from that source.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each embodiment described herein is to be applied *mutatis mutandis* to each and every other embodiment unless specifically stated otherwise.

Those skilled in the art will appreciate that the invention described herein is susceptible to variations and modifications other than those specifically described. It is to be understood that the invention includes all such variations and modifications.

The present invention is not to be limited in scope by the specific embodiments described herein, which are intended for the purpose of exemplification only.

### Brief description of the drawings

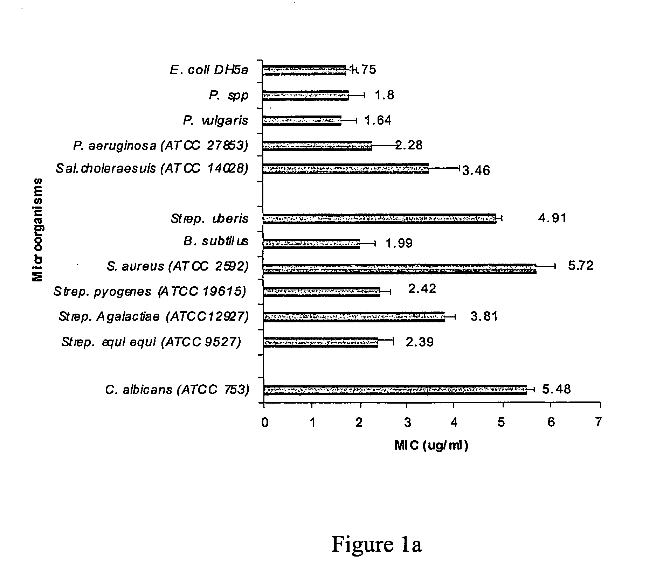

Figure 1a is a graphical representation of the minimum inhibitory concentration (MIC) of an antimicrobial peptide comprising an amino acid sequence set forth in SEQ ID NO: 7 as determined using a radial diffusion assay. The MIC (µg/ml) is indicated on the X- axis. The MIC is defined as the χ intercept of the least mean square regression lines through the respective data points. Results are means ± standard error of the mean (SEM) from two experiments. The microorganism being tested is indicated on the Y axis.

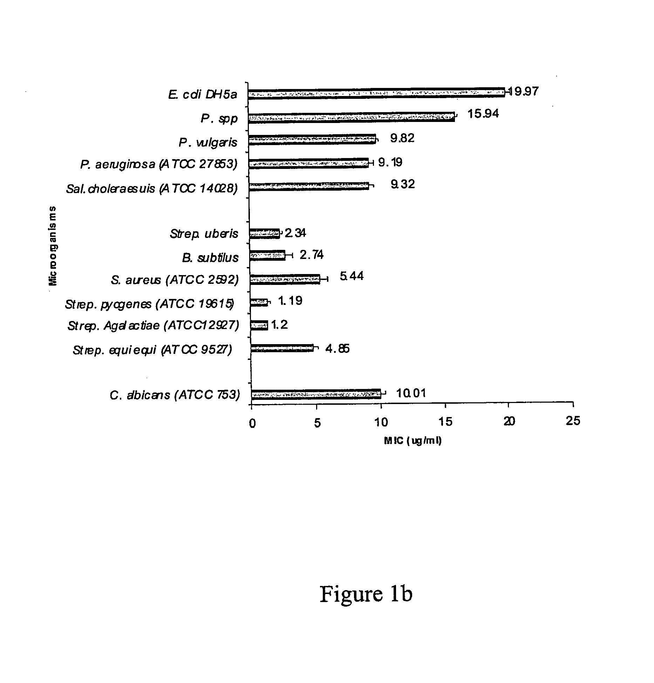

Figure 1b is a graphical representation of the minimum inhibitory concentration (MIC) of an antimicrobial peptide comprising an amino acid sequence set forth in SEQ ID NO: 8 as determined using a radial diffusion assay. The MIC (µg/ml) is indicated on the X- axis. The MIC is defined as the χ intercept of the least mean square regression lines through the respective data points. Results are means ± standard error of the mean (SEM) from two experiments. The microorganism being tested is indicated on the Y axis.

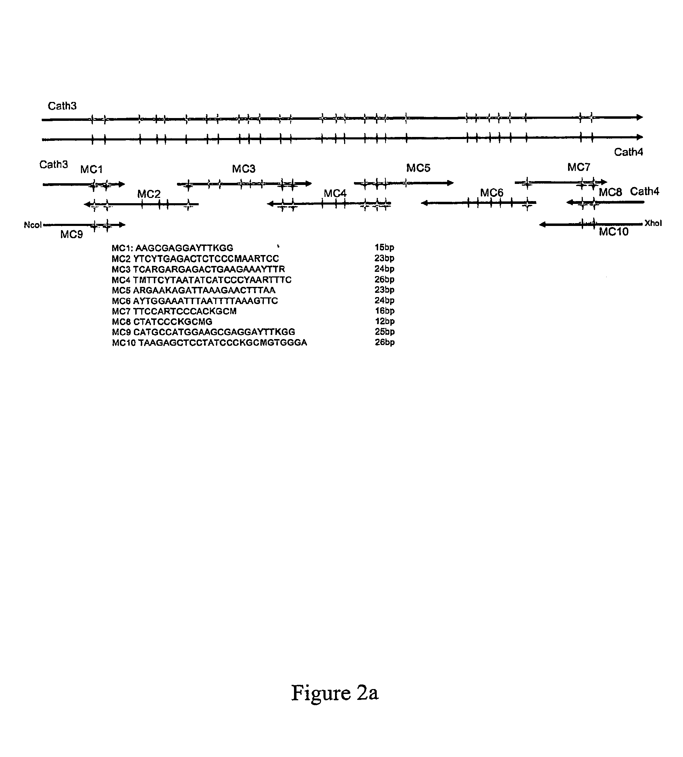

Figure 2a is a schematic representation showing the alignment of degenerate overlapping oligonucleotides (SEQ ID Nos: 113-122) to the sequences of AGG01 (Cath3; SEQ ID NO: 7) and AGG02 (Cath 4; SEQ ID NO: 8) peptides, showing the positions of variable residues as cross-bars. The schematic shows how the positions of variable sequences are conserved in the alignment overlapping oligonucleotides used for producing assembled mutated peptides as described in Example 2 hereof. Sequences of the oligonucleotides are presented below the aligned sequences.

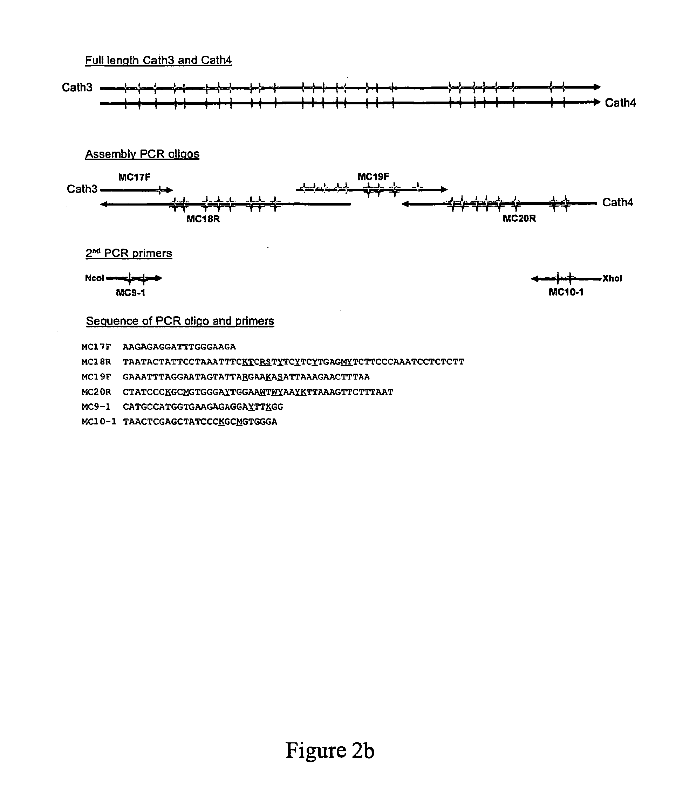

Figure 2b is a schematic representation showing the alignment of degenerate overlapping oligonucleotides (SEQ ID Nos: 123-128) to the sequences of AGG01 (Cath3; SEQ ID NO: 7) and AGG02 (Cath 4; SEQ ID NO: 8) peptides, showing the positions of variable residues as cross-bars. The schematic shows how the positions of variable sequences are conserved in the alignment overlapping oligonucleotides used for producing assembled mutated peptides as described in Example 2 hereof. Sequences of the oligonucleotides are presented below the aligned sequences.

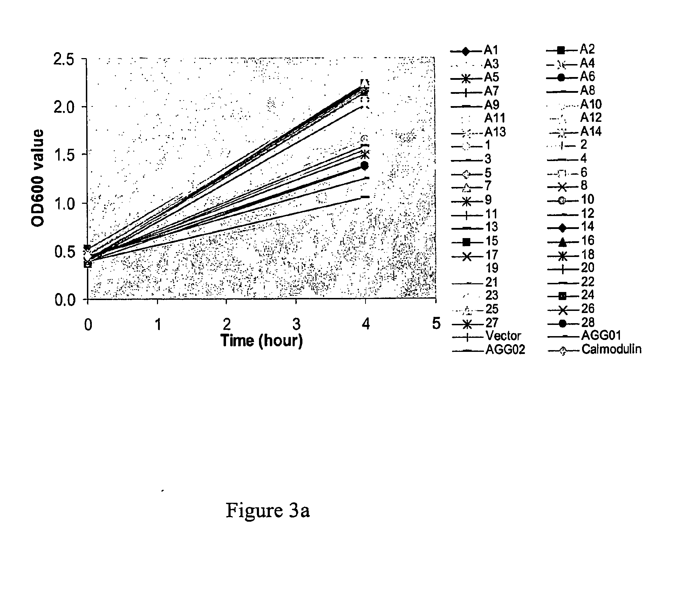

Figure 3a provides a graphical representation showing growth inhibition of antimicrobial peptides produced as described in Figure 2a and 2b (Example 2) over a time course of up to about 4 hours. Peptides are shown to the right. Growth was determined by optical density measurement at 600nm. Data demonstrate that peptides designated A12, 5, 6 and 13 have enhanced growth inhibition activity compared to SEQ ID NO: 7 (AGG01) or SEQ ID NO: 8 (AGG02), or calmodulin and vector controls.

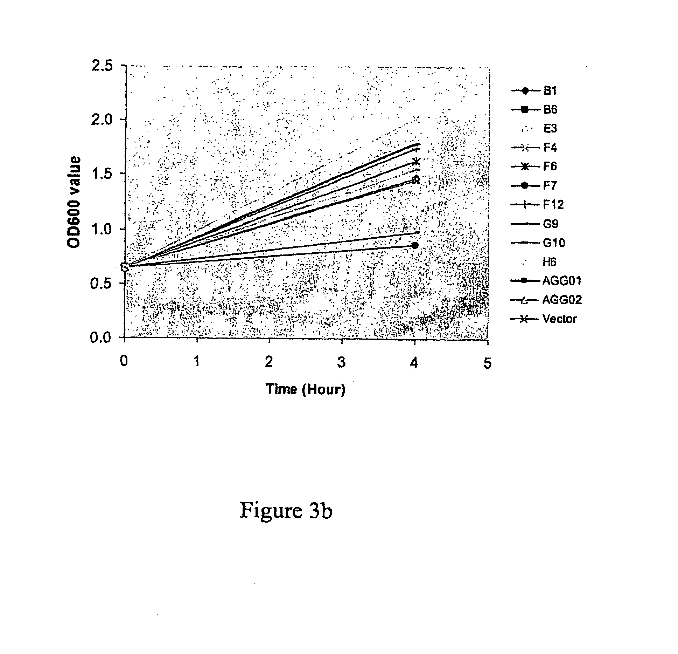

Figure 3b provides a graphical representation showing growth inhibition of antimicrobial peptides produced as described in Figure 2a and 2b (Example 2) over a time course of up to about 4 hours. Peptides are shown to the right. Growth was determined by optical density measurement at 600nm. Data demonstrate that peptides designated B6, E3, F7 and G9 have enhanced growth inhibition activity compared to SEQ ID NO: 7 (AGG01) or SEQ ID NO: 8 (AGG02).

### Detailed description of the preferred embodiments

### Peptides, derivatives and analogs

Disclosed herein is an antimicrobial peptide encoded by a nucleic acid comprising a sequence set forth in SEQ ID NO: 5 or an analog of said peptide or a derivative of said peptide. Preferably said peptide has antimicrobial activity against *S. uberis* and optionally, one or more microorganisms selected from *S. aureus,* or *S. agalactiae* and/or *E. coli.* Suitable antimicrobial peptides will be apparent to the skilled artisan based on the description herein.

As disclosed herein above, preferred antimicrobial peptides have a sequence having a degree of percentage identity to a reference sequence. In determining whether or not two amino acid sequences fall within the defined percentage identity limits *supra,* those skilled in the art will be aware that it is possible to conduct a side-by-side comparison of the amino acid sequences. In such comparisons or alignments, differences will arise in the positioning of non-identical residues depending upon the algorithm used to perform the alignment. In the present context, references to percentage identities and similarities between two or more amino acid sequences shall be taken to refer to the number of identical and similar residues respectively, between said sequences as determined using any standard algorithm known to those skilled in the art. In particular, amino acid identities and similarities are calculated using software of the Computer Genetics Group, Inc., University Research Park, Madison, Wisconsin, United States of America, e.g., using the GAP program of Devereaux et al., Nucl. Acids Res. 12, 387-395, 1984, which utilizes the algorithm of Needleman and Wunsch, J. Mol. Biol. 48, 443-453, 1970. Alternatively, the CLUSTAL W algorithm of Thompson et al., Nucl. Acids Res. 22, 4673-4680, 1994, is used to obtain an alignment of multiple sequences, wherein it is necessary or desirable to maximize the number of identical/similar residues and to minimize the number and/or length of sequence gaps in the alignment.

Alternatively, a suite of commonly used and freely available sequence comparison algorithms is provided by the National Center for Biotechnology Information (NCBI) Basic Local Alignment Search Tool (BLAST) (Altschul et al. J. Mol. Biol. 215: 403-410, 1990), which is available from several sources, including the NCBI, Bethesda, Md.. The BLAST software suite includes various sequence analysis programs including "blastn," that is used to align a known nucleotide sequence with other polynucleotide sequences from a variety of databases and "blastp" used to align a known amino acid sequence with one or more sequences from one or more databases. Also available is a tool called "BLAST 2 Sequences" that is used for direct pairwise comparison of two nucleotide sequences.

As used herein the term "NCBI" shall be taken to mean the database of the National Center for Biotechnology Information at the National Library of Medicine at the National Institutes of Health of the Government of the United States of America, Bethesda, MD, 20894.

In this respect, non-natural amino acids shall be considered to be identical to their natural counterparts. Accordingly, a peptide comprising only non-natural amino acids (e.g., D-amino acids) equivalent to those set forth in SEQ ID NO: 7 shall be considered to have an amino acid sequence 100% identical to SEQ ID NO: 7.

Preferably an antimicrobial peptide or analog or derivative thereof is between about 6 to about 100 residues long (or any value there between), preferably from about 15 to 75 residues (or any value there between), preferably from about 20 to about 50 residues (or any value there between), and even more preferably from about 24 to about 40 residues (or any value there between).

### Peptide analogs

Suitable peptide analogs include, for example, an antimicrobial peptide comprising one or more conservative amino acid substitutions. A "conservative amino acid substitution" is one in which the amino acid residue is replaced with an amino acid residue having a similar side chain.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), β-branched side chains (e.g., threonine, valine, isoleucine) and aromatic side chains (e.g., tyrosine, phenylalanine, tryptophan, histidine).

The importance of the hydropathic amino acid index in conferring interactive biological function on a protein is generally understood in the art (Kyte & Doolittle, J. Mol. Biol. 157, 105-132, 1982). It is known that certain amino acids may be substituted for other amino acids having a similar hydropathic index or score and still retain a similar biological activity, for example, the ability to bind to a membrane of a microorganism and/or kill the microorganism. The hydropathic index of amino acids also may be considered in determining a conservative substitution that produces a functionally equivalent molecule. Each amino acid has been assigned a hydropathic index on the basis of their hydrophobicity and charge characteristics, as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). In making changes based upon the hydropathic index, the substitution of amino acids whose hydropathic indices are within +/- 0.2 is preferred. More preferably the substitution will involve amino acids having hydropathic indices within +/- 0.1, and more preferably within about +/- 0.05.

It is also understood in the art that the substitution of like amino acids is made effectively on the basis of hydrophilicity. As detailed in US Patent No. 4,554,101, the following hydrophilicity values have been assigned to amino acid residues: arginine (+3.0); lysine (+3.0); aspartate (+3.0 +/- 0.1); glutamate (+3.0 +/- 0.1); serine (+0.3); asparagine (+0.2); glutamine (+0.2); glycine (0); threonine (-0.4); proline (-0.5 +/- 0.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, it is preferred to substitute amino acids having hydrophilicity values within about +/- 0.2 of each other, more preferably within about +/- 0.1, and even more preferably within about +/- 0.05

Non-conservative amino acid changes are also contemplated. For example, of particular interest are substitutions of charged amino acids with another charged amino acid and with neutral or positively charged amino acids. The latter of these substitutions results in an antimicrobial peptide analog having reduced positive charge, thereby improving the characteristics of the antimicrobial peptide.

Additional preferred peptide analogs have reduced immunogenicity compared to an antimicrobial peptide of the invention. Alternatively, or in addition, a preferred peptide analog has enhanced stability compared to an antimicrobial peptide of the invention.

It also is contemplated that other sterically similar compounds may be formulated to mimic the key portions of the peptide structure. Such compounds, which may be termed peptidomimetics, may be used in the same manner as the peptides of the invention and hence are also analogs of a peptide of the invention. The generation of such an analog may be achieved by the techniques of modeling and chemical design known to those of skill in the art. It will be understood that all such sterically similar antimicrobial peptide analogs fall within the scope of the present invention.

Another method for determining the "equivalence" of modified peptides involves a functional approach. For example, a given peptide analog is tested for its antimicrobial activity e.g., using any screening method described herein.

Particularly preferred analogs of a peptide of the invention will comprise one or more non-naturally occurring amino acids or amino acid analogs. For example, an antimicrobial peptide analog of the invention comprises one or more naturally-occurring non-genetically encoded L-amino acids, synthetic L-amino acids or D-enantiomers of an amino acid. For example, the peptide comprises only D-amino acids. In another example, the analog comprises one or more residues selected from the group consisting of: hydroxyproline, β-alanine, 2,3-diaminopropionic acid, α-amino isobutyric acid, N-methylglycine (sarcosine), ornithine, citrulline, t-butylalanine, t-butylglycine, N-methylisoleucine, phenylglycine, cyclohexylalanine, norleucine, naphthylalanine, pyridylananine 3-benzothienyl alanine 4-chlorophenylalanine, 2-fluorophenylalanine, 3-fluorophenylalanine, 4-fluorophenylalanine, penicillamine, 1,2,3,4-tetrahydrotic isoquinoline-3-carboxylic acid β-2-thienylalanine, methionine sulfoxide, homoarginine, N-acetyl lysine, 2,4-diamino butyric acid, p-aminophenylalanine , N-methylvaline, homocysteine, homoserine, ε-amino hexanoic acid, δ-amino valeric acid, 2,3-diaminobutyric acid and mixtures thereof.

Commonly-encountered amino acids that are not genetically encoded and which can be present, or substituted for an amino acid in an analog of an antimicrobial peptide of the invention include, but are not limited to, β-alanine (β-Ala) and other omega-amino acids such as 3-aminopropionic acid (Dap), 2,3-diaminopropionic acid (Dpr), 4-aminobutyric acid and so forth; α-aminoisobutyric acid (Aib); ε-aminohexanoic acid (Aha); δ-aminovaleric acid (Ava); methylglycine (MeGly); omithine (Orn); citrulline (Cit); t-butylalanine (t-BuA); t-butylglycine (t-BuG); N-methylisoleucine (MeIle); phenylglycine (Phg); cyclohexylalanine (Cha); norleucine (Nle); 2-naphthylalanine (2-Nal); 4-chlorophenylalanine (Phe(4-C1)); 2-fluorophenylalanine (Phe(2-F)); 3-fluorophenylalanine (Phe(3-F)); 4-fluorophenylalanine (Phe(4-F)); penicillamine (Pen); 1,2,3,4-tetrahydroisoquinoline-3-carboxylic acid (Tic); .beta.-2-thienylalanine (Thi); methionine sulfoxide (MSO); homoarginine (hArg); N-acetyl lysine (AcLys); 2,3-diaminobutyric acid (Dab); 2,3-diaminobutyric acid (Dbu); p-aminophenylalanine (Phe(pNH₂)); N-methyl valine (MeVal); homocysteine (hCys) and homoserine (hSer).

Other amino acid residues that are useful for making the peptides and peptide analogs described herein can be found, e.g., in Fasman, 1989, CRC Practical Handbook of Biochemistry and Molecular Biology, CRC Press, Inc., and the references cited therein.

Also disclosed herein is an isostere of a peptide described herein. The term "isostere" as used herein is intended to include a chemical structure that can be substituted for a second chemical structure because the steric conformation of the first structure fits a binding site specific for the second structure. The term specifically includes peptide back-bone modifications (i.e., amide bond mimetics) known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[CH₂S], ψ[CH₂NH], ψ[CSNH₂], ψ[NHCO], ψ[COCH₂], and ψ[(E) or (Z) CH=CH]. In the nomenclature used above, ψ indicates the absence of an amide bond. The structure that replaces the amide group is specified within the brackets.

Other modifications include, for example, an N-alkyl (or aryl) substitution (ψ [CONR]), or backbone cross-linking to construct lactams and other cyclic structures. Other derivatives of the modulator compounds of the invention include C-terminal hydroxymethyl derivatives, O-modified derivatives (e.g., C-terminal hydroxymethyl benzyl ether), N-terminally modified derivatives including substituted amides such as alkylamides and hydrazides.

The peptide analog may be a retro peptide analog *(*Goodman et al., Accounts of Chemical Research, 12:1-7, 1979). A retro peptide analog comprises a reversed amino acid sequence of an antimicrobial peptide of the present invention. For example, a retro peptide analog of an antimicrobial peptide of the present comprises an amino acid sequence set forth in any one of SEQ ID NOs: 33-58.

An analog of an antimicrobial peptide of the invention may preferably be a retro-inverted peptide (Sela and Zisman, FASEB J. 11:449, 1997). Evolution has ensured the almost exclusive occurrence of L-amino acids in naturally occurring proteins. As a consequence, virtually all proteases cleave peptide bonds between adjacent L- amino acids. Accordingly, artificial proteins or peptides composed of D-amino acids are preferably resistant to proteolytic breakdown. Retro-inverted peptide analogs are isomers of linear peptides in which the direction of the amino acid sequence is reversed (retro) and the chirality, D- or L-, of one or more amino acids therein is inverted (inverso) e.g., using D-amino acids rather than L-amino acids, e.g., Jameson et al., Nature, 368, 744-746 (1994); Brady et al., Nature, 368, 692-693 (1994). The net result of combining D-enantiomers and reverse synthesis is that the positions of carbonyl and amino groups in each amide bond are exchanged, while the position of the side-chain groups at each alpha carbon is preserved.

An advantage of retro-inverted peptides is their enhanced activity *in vivo* due to improved resistance to proteolytic degradation, i.e., the peptide has enhanced stability. (e.g., Chorev et al., Trends Biotech. 13, 438-445, 1995).

Retro-inverted peptide analogs may be complete or partial. Complete retro-inverted peptides are those in which a complete sequence of an antimicrobial peptide of the invention is reversed and the chirality of each amino acid in a sequence is inverted e.g., a peptide comprising an amino acid sequence set forth in any one of SEQ ID Nos:59-83. Partial retro-inverted peptide analogs are those in which some or all of the peptide bonds are reversed (i.e., completely reversed sequence) and the chirality of some, but not all, amino acid residues is inverted. Partial retro-inverted peptide analogs can also have only some of the peptide bonds are reversed and the chirality of only those amino acid residues in the reversed portion inverted. For example, one or two or three or four or five or six or seven or eight or nine or ten or eleven or twelve or thirteen or fourteen or fifteen or sixteen or seventeen or eighteen or nineteen or twenty or twenty one or twenty two or twenty three or twenty four or twenty five or twenty six or twenty seven or twenty eight or twenty nine or thirty or thirty one or thirty two or thirty three or thirty four or thirty five or thirty six or thirty seven or thirty eight amino acid residues are D-amino acids. Disclosed herein are both partial and complete retro-inverted peptide analogs.

In another example, an analog of a peptide is modified to reduce the immunogenicity of said analog. Such reduced immunogenicity is useful for a peptide that is to be injected into a subject. Methods for reducing the immunogenicity of a peptide will be apparent to the skilled artisan. For example, an antigenic region of a peptide is predicted using a method known in the art and described, for example, in Kolaskar and Tongaonkar FEBS Letters, 276: 172-174, 1990. Any identified antigenic region may then be modified to reduce the immunogenicity of a peptide analog, provided that said analog is an antimicrobial peptide analog.

### Peptide derivatives,

Preferred derivatives include, for example, a fragment or processed form of an antimicrobial peptide of the invention. Preferred derivatives have reduced immunogenicity. For example, by deleting an antigenic determinant from an antimicrobial peptide of the invention, a derivative is produced having reduced immunogenicity.

Alternatively, or in addition, a preferred derivative of an antimicrobial peptide of the invention has enhanced antimicrobial activity.

Alternatively, or in addition, a preferred derivative of an antimicrobial peptide of the invention has enhanced stability. For example, a cleavage site of a protease active in a subject to which a peptide is to be administered is mutated and/or deleted to produce a stable derivative of an antimicrobial peptide of the invention.

Methods for producing additional derivatives of an antimicrobial peptide of the invention will be apparent to the skilled artisan and include recombinant methods, e.g., as exemplified herein. For example, the sequences encoding two antimicrobial peptides are aligned, and a consensus sequence produced that is capable of encoding either peptide. Such a consensus sequence is capable of encoding an amino acid that occurs at each position in either peptide, in addition to encoding amino acids that do not occur in either peptide. In this manner peptides that are hybrids of both base peptides are produced.

In another example, a nucleic acid encoding an antimicrobial peptide of the invention or an analog thereof is amplified using mutagenic PCR and the resulting nucleic acid expressed to produce a peptide using a method known in the art and/or described herein.

In a preferred embodiment, the nucleic acid fragments are modified by amplifying a nucleic acid fragment using mutagenic PCR. Such methods include a process selected from the group consisting of: (i) performing the PCR reaction in the presence of manganese; and (ii) performing the PCR in the presence of a concentration of dNTPs sufficient to result in mis-incorporation of nucleotides.

Methods of inducing random mutations using PCR are known in the art and are described, for example, in Dieffenbach (ed) and Dveksler (ed) (In: PCR Primer: A Laboratory Manual, Cold Spring Harbour Laboratories, NY, 1995). Furthermore, commercially available kits for use in mutagenic PCR are obtainable, such as, for example, the Diversify PCR Random Mutagenesis Kit (Clontech) or the GeneMorph Random Mutagenesis Kit (Stratagene).

Peptide derivatives of the present invention also include an antimicrobial peptide or an analog thereof as described herein that is modified to contain one or more-chemical moieties other than an amino acid. The chemical moiety may be linked covalently to the peptide or analog e.g., via an amino terminal amino acid residue, a carboxy terminal amino acid residue, or at an internal amino acid residue. Such modifications include the addition of a protective or capping group on a reactive moiety in the peptide, addition of a detectable label, and other changes that do not adversely destroy the activity of the peptide compound (e.g., its antimicrobial activity).

An "amino-terminal capping group" of a peptide derivative described herein is any chemical compound or moiety that is covalently linked to or conjugated to the amino terminal amino acid residue of a peptide or analog. An amino-terminal capping group may be useful to inhibit or prevent intramolecular cyclization or intermolecular polymerization, to protect the amino terminus from an undesirable reaction with other molecules, or to provide a combination of these properties. A peptide derivative of this invention that possesses an amino-terminal capping group may possess other beneficial activities as compared with the uncapped peptide, such as enhanced efficacy or reduced side effects. Examples of amino terminal capping groups that are useful in preparing peptide derivatives according to the invention include, but are not limited to, 1 to 6 naturally occurring L-amino acid residues, preferably 1-6 lysine residues, 1-6 arginine residues, or a combination of lysine and arginine residues; urethanes; urea compounds; lipoic acid ("Lip"); glucose-3-O-glycolic acid moiety ("Gga"); or an acyl group that is covalently linked to the amino terminal amino acid residue of a peptide, wherein such acyl groups useful in the compositions of the invention may have a carbonyl group and a hydrocarbon chain that ranges from one carbon atom (e.g., as in an acetyl moiety) to up to 25 carbons (e.g., palmitoyl group, "Palm" (16:0) and docosahexaenoyl group, "DHA" (C22:6-3)). Furthermore, the carbon chain of the acyl group may be saturated, as in Palm, or unsaturated, as in DHA. It is understood that when an acid, such as docosahexaenoic acid, palmitic acid, or lipoic acid is designated as an amino terminal capping group, the resultant peptide compound is the condensed product of the uncapped peptide and the acid.

A "carboxy-terminal capping group" of a peptide derivative described herein is any chemical compound or moiety that is covalently linked or conjugated to the carboxy terminal amino acid residue of a peptide or analog. The primary purpose of such a carboxy-terminal capping group is to inhibit or prevent intramolecular cyclization or intermolecular polymerization or to provide a combination of these properties. A peptide derivative of this invention possessing a carboxy-terminal capping group may also possess other beneficial activities as compared with an uncapped peptide, such as enhanced efficacy, reduced side effects, enhanced hydrophilicity, enhanced hydrophobicity. Carboxy-terminal capping groups that are particularly useful in the peptide derivatives described herein include primary or secondary amines that are linked by an amide bond to the α-carboxyl group of the carboxy terminal amino acid of the peptide compound. Other carboxy terminal capping groups useful in the invention include aliphatic primary and secondary alcohols and aromatic phenolic derivatives, including flavenoids, with 1 to 26 carbon atoms, which form esters when linked to the carboxylic acid group of the carboxy-terminal amino acid residue of a peptide described herein.

Other chemical modifications of a peptide or analog, include, for example, glycosylation, acetylation (including N-terminal acetylation), carboxylation, carbonylation, phosphorylation, PEGylation, amidation, addition of trans olefin, substitution of α-hydrogens with methyl groups, derivatization by known protecting/blocking groups, circularization, linkage to an antibody or other cellular ligand, etc. Any of numerous chemical modifications may be carried out by known techniques, including but not limited to specific chemical cleavage by cyanogen bromide, trypsin, chymotrypsin, papain, V8 protease, NaBH₄, acetylation, formylation, oxidation, reduction, etc.

### Fusion proteins and complexes

Also disclosed herein is an additional derivative of an antimicrobial peptide of the invention, such as, for example a fusion protein comprising one or more of the antimicrobial peptides and/or analogs of the invention. For example, the antimicrobial peptide or analog is fused to a tag or label. Such a tag or label facilitates purification or isolation of the antimicrobial peptide and/or analog and/or derivative or detection of the peptide, analog or derivative. Suitable tags will be apparent to the skilled artisan and include, for example, influenza virus hemagglutinin (HA), Simian Virus 5 (V5), polyhistidine, c-myc or FLAG.

In another example, a fusion protein of the present invention comprises a plurality of antimicrobial peptides of the invention and/or analogs thereof. In this respect, the fusion protein may comprise multiple copies of the same antimicrobial peptide or analog and/or a plurality of antimicrobial peptides and/or analogs (whether present in a single copy or a plurality of copies).

Such a fusion protein may comprise one or more additional components, such as, for example, a tag or label and/or an additional antimicrobial peptide or analog or derivative thereof.

In a further example, a fusion protein as described herein comprises an antimicrobial peptide or analog or derivative thereof fused to another protein or a fragment thereof so as to constrain and/or display said antimicrobial peptide or analog within said other protein. Polypeptides used for such purposes are capable of reducing the flexibility of another protein's amino and/or carboxyl termini. Preferably such proteins provide a rigid scaffold or platform for the protein. In addition, such proteins preferably are capable of providing protection from proteolytic degradation and the like, and/or are capable of enhancing solubility. In one example, the antimicrobial peptide or analog thereof is fused to a protein or fragment thereof that is expressed in a non-human mall in nature. In one example, the antimicrobial peptide or analog is fused to a naturally-occurring antimicrobial peptide, e.g., a peptide comprising a sequence set forth in any one of SEQ ID NOs: 104-111, or a fragment thereof, e.g., a prepro region of a sequence set forth in any one of SEQ ID NOs: 104-111.

Each of the components of a derivative of an antimicrobial peptide of the invention may optionally be separated by a linker that facilitates the independent folding of each of said components. A suitable linker will be apparent to the skilled artisan. For example, it is often unfavourable to have a linker sequence with high propensity to adopt α-helix or β-strand structures, which could limit the flexibility of the protein and consequently its functional activity. Rather, a more desirable linker is a sequence with a preference to adopt extended conformation. In practice, most currently designed linker sequences have a high content of glycine residues that force the linker to adopt loop conformation. Glycine is generally used in designed linkers because the absence of a β-carbon permits the polypeptide backbone to access dihedral angles that are energetically forbidden for other amino acids.

Preferably the linker is hydrophilic, i.e. the residues in the linker are hydrophilic.

Linkers comprising glycine and/or serine have a high freedom degree for linking of two proteins, i.e., they enable the fused proteins to fold and produce functional proteins. Robinson and Sauer Proc. Natl. Acad. Sci. 95: 5929-5934, 1998 found that it is the composition of a linker peptide that is important for stability and folding of a fusion protein rather than a specific sequence. For example, the authors found that a fusion protein comprising a linker consisting almost entirely of glycine was unstable. Accordingly, the use of amino acid residues other than glycine, such as, for example, alanine or serine, is also useful for the production of a linker.

The linker may be a glycine rich linker. Preferably the linker is a glycine linker that additionally comprises alanine and/or serine.

### Peptide synthesis

In one example, an antimicrobial peptide of the invention or an analog or derivative thereof synthesized using a chemical method known to the skilled artisan. For example, synthetic peptides are prepared using known techniques of solid phase, liquid phase, or peptide condensation, or any combination thereof, and can include natural and/or unnatural amino acids. Amino acids used for peptide synthesis may be standard Boc (Nα-amino protected Nα-t-butyloxycarbonyl) amino acid resin with the deprotecting, neutralization, coupling and wash protocols of the original solid phase procedure of Merrifield, J. Am. Chem. Soc., 85:2149-2154, 1963, or the base-labile Nα-amino protected 9-fluorenylmethoxycarbonyl (Fmoc) amino acids described by Carpino and Han, J. Org. Chem., 37:3403-3409, 1972. Both Fmoc and Boc Nα-amino protected amino acids can be obtained from various commercial sources, such as, for example, Fluka, Bachem, Advanced Chemtech, Sigma, Cambridge Research Biochemical, Bachem, or Peninsula Labs.

Generally, chemical synthesis methods comprise the sequential addition of one or more amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions that allow for the formation of an amide linkage. The protecting group is then removed from the newly added amino acid residue and the next amino acid (suitably protected) is then added, and so forth. After the desired amino acids have been linked in the proper sequence, any remaining protecting groups (and any solid support, if solid phase synthesis techniques are used) are removed sequentially or concurrently, to render the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide. See, e.g., J. M. Stewart and J. D. Young, Solid Phase Peptide Synthesis (Pierce Chemical Co., Rockford, IL 1984) and G. Barany and R. B.Merrifield, The Peptides : Analysis, Synthesis, Biology, editors E. Gross and J. Meienhofer, Vol. 2, (Academic Press, New York, 1980), pp. 3-254, for solid phase peptide synthesis techniques; and M. Bodansky, Principles of Peptide Synthesis, (Springer-Verlag, Berlin 1984)and E. Gross and J. Meienhofer, Eds. , The Peptides : Analysis. Synthesis. Biology, Vol.1, for classical solution synthesis. These methods are suitable for synthesis of an antimicrobial peptide of the present invention or an analog or derivative thereof.

Typical protecting groups include t-butyloxycarbonyl (Boc), 9-fluorenylmethoxycarbonyl (Fmoc) benzyloxycarbonyl (Cbz); p-toluenesulfonyl (Tx); 2,4-dinitrophenyl ; benzyl (Bzl); biphenylisopropyloxycarboxy-carbonyl, t-amyloxycarbonyl, isobornyloxycarbonyl, o-bromobenzyloxycarbonyl, cyclohexyl, isopropyl, acetyl, o-nitrophenylsulfonyl and the like.

Typical solid supports are cross-linked polymeric supports. These can include divinylbenzene cross-linked-styrene-based polymers, for example, divinylbenzene-hydroxymethylstyrene copolymers, divinylbenzene- chloromethylstyrene copolymers and divinylbenzene-benzhydrylaminopolystyrene copolymers.

The antimicrobial peptide, analog or derivative of the present invention can also be chemically prepared by other methods such as by the method of simultaneous multiple peptide synthesis. See, e. g. , Houghten Proc. Natl. Acad. Sci. USA 82: 5131-5135, 1985 or U. S. Patent No. 4,631,211.

As will be apparent to the skilled artisan based on the description herein, an analog or derivative of an antimicrobial of the invention may comprise D-amino acids, a combination of D- and L-amino acids, and various unnatural amino acids (e.g., α-methyl amino acids, Cα-methyl amino acids, and Nα-methyl amino acids, etc) to convey special properties. Synthetic amino acids include ornithine for lysine, fluorophenylalanine for phenylalanine, and norleucine for leucine or isoleucine. Methods for the synthesis of such peptides will be apparent to the skilled artisan based on the foregoing.

### Recombinant peptide production

In another example, an antimicrobial peptide or analog or derivative thereof is produced as a recombinant protein. To facilitate the production of a recombinant peptide or fusion protein nucleic acid encoding same is preferably isolated or synthesized. Typically the nucleic acid encoding the constituent components of the fusion protein is/are isolated using a known method, such as, for example, amplification (e.g., using PCR or splice overlap extension) or isolated from nucleic acid from an organism using one or more restriction enzymes or isolated from a library of nucleic acids. Methods for such isolation will be apparent to the ordinary skilled artisan and/or described in Ausubel et al (In: Current Protocols in Molecular Biology. Wiley Interscience, ISBN 047 150338, 1987), Sambrook et al (In: Molecular Cloning: Molecular Cloning: A Laboratory Manual, Cold Spring Harbor Laboratories, New York, Third Edition 2001).

For example, nucleic acid (e.g.., genomic DNA or RNA that is then reverse transcribed to form cDNA) from a cell or organism capable of expressing an antimicrobial peptide of the invention is isolated using a method known in the art and cloned into a suitable vector. The vector is then introduced into a suitable organism, such as, for example, a bacterial cell. Using a nucleic acid probe from a known antimicrobial peptide encoding gene a cell comprising the nucleic acid of interest is isolated using methods known in the art and described, for example, in Ausubel et al (In: Current Protocols in Molecular Biology. Wiley Interscience, ISBN 047 150338, 1987), Sambrook et al (In: Molecular Cloning: Molecular Cloning: A Laboratory Manual, Cold Spring Harbor Laboratories, New York, Third Edition 2001).

Alternatively, nucleic acid encoding an antimicrobial peptide of the invention is isolated using polymerase chain reaction (PCR). Methods of PCR are known in the art and described, for example, in Dieffenbach (ed) and Dveksler (ed) (In: PCR Primer: A Laboratory Manual, Cold Spring Harbour Laboratories, NY, 1995). Generally, for PCR two non-complementary nucleic acid primer molecules comprising at least about 20 nucleotides in length, and more preferably at least 25 nucleotides in length are hybridized to different strands of a nucleic acid template molecule, and specific nucleic acid molecule copies of the template are amplified enzymatically. Preferably the primers hybridize to nucleic acid adjacent to a nucleic acid encoding an antimicrobial peptide of the invention, thereby facilitating amplification of the nucleic acid that encodes the subunit. Following amplification, the amplified nucleic acid is isolated using a method known in the art and, preferably cloned into a suitable vector.

Other methods for the production of a nucleic acid of the invention will be apparent to the skilled artisan and are encompassed by the present invention.

For expressing protein by recombinant means, a protein-encoding nucleotide sequence is placed in operable connection with a promoter or other regulatory sequence capable of regulating expression in a cell-free system or cellular system. For example, nucleic acid comprising a sequence that encodes an antimicrobial peptide of the present invention in operable connection with a suitable promoter is expressed in a suitable cell for a time and under conditions sufficient for expression to occur. Nucleic acid encoding an antimicrobial protein of the present invention is readily derived from the publicly available amino acid sequence.

Should it be preferred that a peptide or fusion protein of the invention is expressed *in vitro* a suitable promoter includes, but is not limited to a T3 or a T7 bacteriophage promoter (Hanes and Plückthun Proc. Natl. Acad. Sci. USA, 94 4937-4942 1997).

Typical expression vectors for *in vitro* expression or cell-free expression have been described and include, but are not limited to the TNT T7 and TNT T3 systems (Promega), the pEXP1-DEST and pEXP2-DEST vectors (Invitrogen).

Typical promoters suitable for expression in bacterial cells include, but are not limited to, the lacz promoter, the Ipp promoter, temperature-sensitive λL or λR promoters, T7 promoter, T3 promoter, SP6 promoter or semi-artificial promoters such as the IPTG-inducible tac promoter or lacUV5 promoter. A number of other gene construct systems for expressing the nucleic acid fragment of the invention in bacterial cells are well-known in the art and are described for example, in Ausubel et al (In: Current Protocols in Molecular Biology. Wiley Interscience, ISBN 047 150338, 1987), US Patent No. 5,763,239 (Diversa Corporation) and Sambrook et al (In: Molecular Cloning: Molecular Cloning: A Laboratory Manual, Cold Spring Harbor Laboratories, New York, Third Edition 2001).

Numerous expression vectors for expression of recombinant polypeptides in bacterial cells and efficient ribosome binding sites have been described, and include, for example, PKC30 (Shimatake and Rosenberg, Nature 292, 128, 1981); pKK173-3 (Amann and Brosius, Gene 40, 183, 1985), pET-3 (Studier and Moffat, J. Mol. Biol. 189, 113, 1986); the pCR vector suite (Invitrogen), pGEM-T Easy vectors (Promega), the pL expression vector suite (Invitrogen) the pBAD/TOPO or pBAD/thio - TOPO series of vectors containing an arabinose-inducible promoter (Invitrogen, Carlsbad, CA), the latter of which is designed to also produce fusion proteins with a Trx loop for conformational constraint of the expressed protein; the pFLEX series of expression vectors (Pfizer Inc., CT,USA); the pQE series of expression vectors (QIAGEN, CA, USA), or the pL series of expression vectors (Invitrogen), amongst others.

Typical promoters suitable for expression in viruses of eukaryotic cells and eukaryotic cells include the SV40 late promoter, SV40 early promoter and cytomegalovirus (CMV) promoter, CMV IE (cytomegalovirus immediate early) promoter amongst others. Preferred vectors for expression in mammalian cells (e.g., 293, COS, CHO, 10T cells, 293T cells) include, but are not limited to, the pcDNA vector suite supplied by Invitrogen, in particular pcDNA 3.1 myc-His-tag comprising the CMV promoter and encoding a C-terminal 6xHis and MYC tag; and the retrovirus vector pSRatkneo *(*Muller et al., Mol. Cell. Biol., 11, 1785, 1991).

A wide range of additional host/vector systems suitable for expressing an antimicrobial peptide or fusion protein of the present invention are available publicly, and described, for example, in Sambrook et al (In: Molecular cloning, A laboratory manual, second edition, Cold Spring Harbor Laboratory, Cold Spring Harbor, N.Y., 1989).

Means for introducing the isolated nucleic acid molecule or a gene construct comprising same into a cell for expression are well-known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA-coated tungsten or gold particles (Agracetus Inc., WI, USA) amongst others.

### Peptide/analog/derivative/fusion protein isolation

Following production/expression/synthesis, an antimicrobial peptide of the invention or derivative or analog thereof is purified using a method known in the art. Such purification preferably provides a peptide of the invention substantially free of conspecific protein, acids, lipids, carbohydrates, and the like. Antibodies and other affinity ligands are particularly preferred for producing isolated protein. Preferably the protein will be in a preparation wherein more than about 90% (e.g. 95%, 98% or 99%) of the protein in the preparation is an antimicrobial peptide of the invention or derivative or analog thereof or fusion protein comprising same.

Standard methods of peptide purification are employed to obtain an isolated peptide of the invention, including but not limited to various high-pressure (or performance) liquid chromatography (HPLC) and non-HPLC peptide isolation protocols, such as size exclusion chromatography, ion exchange chromatography, phase separation methods, electrophoretic separations, precipitation methods, salting in/out methods, immunochromatography, and/or other methods.

A preferred method of isolating peptide compounds useful in compositions and methods of the invention employs reversed-phase HPLC using an alkylated silica column such as C₄-, C₈- or C₁₈-silica. A gradient mobile phase of increasing organic content is generally used to achieve purification, for example, acetonitrile in an aqueous buffer, usually containing a small amount of trifluoroacetic acid. Ion-exchange chromatography can also be used to separate a peptide based on its charge.

Alternatively, affinity purification is useful for isolating a fusion protein comprising a label. Methods for isolating a protein using affinity chromatography are known in the art and described, for example, in Scopes (In: Protein purification: principles and practice, Third Edition, Springer Verlag, 1994). For example, an antibody or compound that binds to the label (in the case of a polyhistidine tag this may be, for example, nickel-NTA) is preferably immobilized on a solid support. A sample comprising a fusion protein is then contacted to the immobilized antibody or compound for a time and under conditions sufficient for binding to occur. Following washing to remove any unbound or non-specifically bound protein, the fusion protein is eluted.

The degree of purity of the peptide compound may be determined by various methods, including identification of a major large peak on HPLC. A peptide compound that produces a single peak that is at least 95% of the input material on an HPLC column is preferred. Even more preferable is a polypeptide that produces a single peak that is at least 97%, at least 98%, at least 99% or even 99.5% of the input material on an HPLC column.

To ensure that a peptide obtained using-any of the techniques described above is the desired peptide for use in compositions and methods of the present invention, analysis of the composition of the peptide is determined by any of a variety of analytical methods known in the art. Such composition analysis may be conducted using high resolution mass spectrometry to determine the molecular weight of the peptide. Alternatively, the amino acid content of a peptide can be confirmed by hydrolyzing the peptide in aqueous acid, and separating, identifying and quantifying the components of the mixture using HPLC, or an amino acid analyzer. Protein sequenators, which sequentially degrade the peptide and identify the amino acids in order, may also be used to determine the sequence of the peptide. Since some of the peptide compounds contain amino and/or carboxy terminal capping groups, it may be necessary to remove the capping group or the capped amino acid residue prior to a sequence analysis. Thin-layer chromatographic methods may also be used to authenticate one or more constituent groups or residues of a desired peptide.

### Expression constructs

### Nucleic acid encoding an antimicrobial peptide or analog or derivative

Nucleic acids that encode one or more antimicrobial peptides or analogs or derivatives of the present invention will be apparent to the skilled artisan based on the description herein.

For example, the nucleotide sequence of suitable nucleic acids encode an antimicrobial peptide or analog or derivative thereof individually or collectively selected from the group consisting of:
(i) a peptide comprising a sequence set forth in any one of SEQ ID NOs: 7-32;
(ii) an analog of (i) comprising a sequence set forth in any one of SEQ ID NOs: 33-83; and
(iii) a derivative of (i) having at least about 90% or 95% sequence identity thereto and comprising a sequence that differs from a sequence set forth in (i) or (ii) by one or more conservative amino acid substitutions, wherein said peptide has antimicrobial activity.

In determining whether or not two sequences fall within these defined percentage identity limits, those skilled in the art will be aware that it is possible to conduct a side-by-side comparison of the sequences. In such comparisons or alignments, differences will arise in the positioning of non-identical residues depending upon the algorithm used to perform the alignment. In the present context, references to percentage identities and similarities between two or more sequences shall be taken to refer to the number of identical and similar residues respectively, between said sequences as determined using any standard algorithm known to those skilled in the art. For example, nucleotide identities and similarities are calculated using software of the Computer Genetics Group, Inc., University Research Park, Maddison, Wis., United States of America, e.g., using the GAP program of Devereaux et al., Nucl. Acids Res. 12, 387-395, 1984, which utilizes the algorithm of Needleman and Wunsch, J. Mol. Biol. 48, 443-453, 1970. Alternatively, the CLUSTAL W algorithm of Thompson et a/., Nucl. Acids Res. 22, 4673-4680, 1994, is used to obtain an alignment of multiple sequences, wherein it is necessary or desirable to maximize the number of identical/similar residues and to minimize the number and/or length of sequence gaps in the alignment. Sequence alignments can also be performed using a variety of other commercially available sequence analysis programs, such as, for example, the BLAST program available at NCBI.

Preferably the expression construct comprises a sequence encoding a peptide comprising a sequence set forth in any one of SEQ ID NOs: 7-32, more preferably any one of SEQ ID NOs: 10-13 and 23-32, still more preferably any one of SEQ ID NOs: 10-13, 24, 25, 28 or 30.

For example, the genetically-modified non-human mammal comprises an expression construct comprising a sequence set forth in any one of SEQ ID NOs: 84-88.90 or 91, preferably any one of SEQ ID Nos: 84-86 including SEQ ID NO: 84 or 85 or 86.

The expression construct can also optionally comprise a transcriptional terminator that is operative in the animal to which the construct is to be introduced. Furthermore, the gene construct may comprise a nucleic acid comprising the sequence of a polyadenylation signal operative in animal to which the construct is to be introduced.

### Promoters

Suitable promoters will be apparent to the skilled artisan. For example, a variety of transcriptional promoters that preferentially activate transcription in mammary epithelial cells are available. These include the promoters that control the genes encoding milk proteins such as caseins (αS1-, αS2-, β-, γ-, and κ-casein), β-lactoglobulin (Clark et al., 1989, Bio/Technology 7: 487-492), whey acid protein (Gordon et al., 1987, Bio/Technology 5: 1183-1187), and α-lactalbumin (Soulier et al., 1992, FEBS Letters 297: 13).

Nucleotide sequences of many promoters are publicly available, e.g., in GenBank and in scientific publications such as:
1) rat α-lactalbumin (Richards et al., 1981, J. Biol. Chem. 256: 526-532);
2) rat WAP (Campbell et al., 1984, Nucleic Acids Res. 12: 8685-8697);
3) rat α-casein (Jones et al., 1985, J. Biol. Chem. 260: 7042-7050);
4) rat α-casein (Lee and Rosen, 1983, J. Biol. Chem. 258: 10794-10804);
5) human alpha-lactalbumin (Hall, 1987, Biochem. J. 242: 735-742);
6) bovine α-S1 casein (Stewart, 1984, Nucleic Acids Res. 12: 389);
7) bovine α-casein (Gorodetsky et al., 1988, Gene 66: 87-96);
8) bovine α-casein (Alexander et al., 1988, Eur. J. Biochem. 178: 395-401);
9) bovine α-S2 casein (Brignon et al., 1977, FEBS Letters 188: 48-55);
10) bovine α-lactoglobulin (Jamieson et al., 1987, Gene 61: 85-90; Alexander et al., 1989, Nucleic Acids Res. 17: 6739).

In one example, a suitable promoters is a bovine β-casein gene promoter comprising a sequence set forth in SEQ ID NO: 92. In another example, a suitable promoter is a murine prolactin-inducible mammary specific promoter comprising a sequence set forth in SEQ ID NO: 93. In another example, a suitable promoter is a water buffalo α-lactalbumin gene promoter comprising a sequence set forth in SEQ ID NO: 94. In another example, a suitable promoter is a murine whey acidic protein (WAP) gene promoter comprising a sequence set forth in SEQ ID NO: 95 or a camel WAP gene promoter comprising a sequence set forth in SEQ ID NO: 96 or a rat WAP gene promoter comprising a sequence set forth in SEQ ID NO: 97. In another example, a suitable promoter is a caprine β-lactoglobulin gene promoter comprising a sequence set forth in SEQ ID NO: 98 or an ovine β-lactoglobulin gene promoter comprising a sequence set forth in SEQ ID NO: 99 or a caprine β-lactoglobulin gene promoter comprising a sequence set forth in SEQ ID NO: 100.

In another example, the promoter is a NF-κB promoter. For example, the promoter is derived from a lactoferrin gene. For example, the promoter comprises 4.4kb of nucleic acid 5' to a bovine lactoferrin gene, e.g., as described in Zheng et al., Gene, 353: 107-117,2005.

Whilst it is preferable that a promoter used in an expression construct is derived from a non-human mammal into which the expression construct is to be introduced to increase the likelihood of correct expression, this is not essential. For example, rat and murine WAP gene promoters have been shown to be effective at inducing expression of proteins in other non-human mammals.

In the case of an expression construct to be administered to a mammary gland or cell or tissue thereof a suitable promoter also includes a promoter that expresses in a variety of cells in a non-human mammal including a mammary cell. For example, the promoter is a human cytomegalovirus (hCMV) immediate early (IE) promoter or a simian virus (SV)-40 promoter and/or enhancer.

### Signal Peptide Sequences

In one example, an expression construct encodes an antimicrobial peptide or analog or derivative fused to signal peptide, particularly a signal peptide of a milk specific gene, e.g., to induce or enhance secretion of an encoded peptide or analog or derivative into milk. For optimal secretion efficiency, the milk-specific signal peptide sequence is preferably derived from the same gene as the promoter used in the transgene.

Exemplary signal sequences are from genes coding for caseins, e.g., αS1-, αS2-, β-, γ-, and κ-casein, β-lactoglobulin, whey acid protein, or lactalbumin. For example, the sequence of a signal peptide from an α-1 lactalbumin is set forth in SEQ ID NO: 101. The sequence of a signal peptide from an αS1-casein protein is set forth in SEQ ID NO: 102. the sequence of a signal peptide from a β-lactoglobulin protein is set forth in SEQ ID NO: 103.

### Selectable and/or detectable markers

In one embodiment, an expression construct comprises a nucleic acid encoding a detectable and/or selectable marker operably linked to a promoter. Such a marker facilitates the detection and/or selection of a genetically-modified cell or animal.

As used herein the term "selectable marker" shall be taken to mean a protein or peptide that confers a phenotype on a cell expressing said selectable marker that is not shown by those cells that do not carry said selectable marker. Examples of selectable markers include, but are not limited to the *dhfr* resistance gene, which confers resistance to methotrexate (Wigler, et al., Proc. Natl. Acad. Sci. USA 77:3567, 1980); the *gpt* resistance gene, which confers resistance to mycophenolic acid (Mulligan & Berg, Proc. Natl. Acad. Sci. USA 78:2072, 1981); the neomycin phosphotransferase gene, which confers resistance to the aminoglycoside G-418 (Colberre-Garapin, et al., J. Mol. Biol. 150:1, 1981); and the hygromycin resistance gene (Santerre, et al., Gene 30:147, 1984).

Alternatively, the nucleic acid construct comprises a detectable marker gene. Suitable detectable marker gene include, for example, a bacterial luciferase gene; a firefly luciferase gene; or a β-galactosidase gene (the expression of which is detected by the metabolism of 5-bromo-4-chloro-3-indolyl-β-D-galactopyranoside to produce a blue precipitate) or a fluorescent marker, such as, for example, a green fluorescent protein *(gfp),* a monomeric discosoma red fluorescent protein (dsRED) or a monomeric GFP from *Aequorea coerulescens.*

### Producing an expression construct

Methods for producing expression constructs are known in the art and/or described in Ausubel et al (In: Current Protocols in Molecular Biology. Wiley Interscience, ISBN 047 150338, 1987), Sambrook et al (In: Molecular Cloning: Molecular Cloning: A Laboratory Manual, Cold Spring Harbor Laboratories, New York, Third Edition 2001).

Typically, the nucleic acid encoding the constituent components of the expression construct is/are isolated using a known method, such as, for example, amplification (e.g., using PCR or splice overlap extension) or isolated from nucleic acid from an organism using one or more restriction enzymes or isolated from a library of nucleic acids. Methods for such isolation will be apparent to the ordinary skilled artisan. For example, the present inventors have used splice overlap extension to produce nucleic acid encoding an antimicrobial peptide variant or have synthesized nucleic acid encoding an antimicrobial peptide variant.

Alternatively, nucleic acid encoding a nucleic acid constituent of a construct for use in the method of the present invention is isolated using polymerase chain reaction (PCR). Methods of PCR are known in the art and described, for example, in Dieffenbach (ed) and Dveksler (ed) (In: PCR Primer: A Laboratory Manual, Cold Spring Harbour Laboratories, NY, 1995). Generally, for PCR two non-complementary nucleic acid primer molecules comprising at least about 20 nucleotides in length, and more preferably at least 25 nucleotides in length are hybridized to different strands of a nucleic acid template molecule, and specific nucleic acid copies of the template are amplified enzymatically. Preferably the primers hybridize to nucleic acid adjacent to the nucleic acid of interest (e.g., a nucleic acid encoding an antimicrobial peptide and/or analog and/or derivative, a promoter and/or a nucleic acid encoding a detectable marker or selectable marker), thereby facilitating amplification of the nucleic acid. Following amplification, the amplified nucleic acid is isolated using a method known in the art and, preferably cloned into a suitable vector, e.g., a vector described herein.

Other methods for the production of an expression construct of the invention will be apparent to the skilled artisan and are encompassed by the present invention. For example, a nucleic acid encoding a antimicrobial peptide is introduced into a publicly available expression vector in operable connection with a promoter that expresses a peptide in a mammary gland or cell or tissue thereof. Suitable vectors include, for example, a pRβH vector described in USSN 10/952,376 in which a nucleic acid encoding an antimicrobial peptide analog or derivative is operably linked to a β-casein promoter and a β-lactamase selectable marker gene, or a pVEβcash vector also described in USSN 10/952,376 in which a nucleic acid encoding an antimicrobial peptide or analog or derivative is operably linked to a truncated β-casein promoter and a neomycin resistance gene.

Prior to introduction of the expression construct into a cell to produce a genetically-modified cell, said construct is preferably linearized, for example, by restriction endonuclease digestion to facilitate integration into the genome of the cell. Optionally, regions that are not required for expression of an antimicrobial peptide and/or analog and/or derivative and/or selectable/detectable marker are removed at this stage, e.g., by restriction endonuclease digestion.

### Production of a knock-in construct

In one embodiment, the expression construct is a so-called "knock-in" construct. Such a construct facilitates the insertion of an expression construct at a predetermined site in the genome of a non-human mammal by homologous recombination. Accordingly, such a construct is useful for replacing an endogenous antimicrobial peptide encoding gene with a nucleic acid encoding, for example, an antimicrobial peptide or analog or derivative described herein. For example, a nucleic acid encoding an antimicrobial peptide or variant or fusion protein described herein is inserted into the coding region of a gene expressed in milk in nature, e.g., αS1-, αS2-, β-, γ-, and κ-casein, β-lactoglobulin, whey acid protein, or lactalbumin.

One of two configurations of construct is generally used for a vector for homologous recombination, i.e., an insertion construct or a replacement construct. An insertion construct comprises a region of homology to the target nucleic acid cloned as a single continuous sequence. The insertion construct additionally comprises a nucleic acid that is to be inserted into the target nucleic acid, e.g., a antimicrobial peptide encoding nucleic acid, positioned adjacent to and, if required, in-frame with the region of homology. The insertion vector is then linearized, e.g., by cleavage of a unique restriction site within the region of homology with the target sequence. Homologous recombination introduces the insertion construct sequences and any adjacent nucleic acid into the homologous site of the target nucleic acid, interrupting normal target nucleic acid structure by adding an additional sequence. Such a vector is useful for, for example, introducing one or more antimicrobial peptide encoding nucleic acids

A replacement construct is also useful for knocking-in a nucleic acid of interest. This form of construct contains two regions of homology to the target nucleic acid located on either side of a heterologous nucleic acid, for example, encoding one or more antimicrobial peptides or analogs or derivatives and/or selectable/detectable markers. Homologous recombination proceeds by at least two recombination events, i.e., a double cross-over event that leads to the replacement of target nucleic acid with the replacement construct sequences. More specifically, each region of homology in the vector induces at least one recombination event that leads to the heterologous nucleic acid in the vector replacing the nucleic acid located between the regions of homology in the target nucleic acid. A replacement construct is useful for, for example, replacing an endogenous antimicrobial peptide encoding gene with a nucleic acid encoding a specific antimicrobial peptide described herein.

The present invention clearly contemplates an expression construct designed to introduce a nucleic acid encoding a antimicrobial peptide at a predetermined site in the genome of a non-human mammal. In general terms, such an expression construct comprises a nucleic acid comprising a nucleotide sequence that is effective for homologous recombination with the target nucleic acid. For example, a replacement targeting vector comprises at least two regions of nucleic acid that are substantially identical to a genomic sequence of the target sequence. As will be apparent to the skilled artisan, some degree of non-identity does not significantly adversely affect the gene targeting capability of a construct of the invention. However, a higher the degree of identity between the regions of homology in the vector and the target sequence increases the likelihood of effective homologous recombination. Accordingly, it is preferred that a region of a vector homologous to a target nucleic acid comprises a nucleotide sequence that is at least about 80% identical to the target sequence, more preferably 90% or 95% identical.

Longer regions of homology are useful for inducing homologous recombination at the target nucleic acid. However, this region need not be so long so as to be unwieldy. Preferably each region of homology comprises at least about 1500bp that is substantially identical to a target sequence, more preferably 2000bp and even more preferably at least about 3000bp.

Guidelines for the selection and use of sequences are described, for example, in Deng and Cappecchi, Mol. Cell. Biol., 12:3365-3371, 1992 and Bollag, et al., Ann. Rev. Genet., 23:199-225, 1989.

Suitable targeting constructs of the invention are prepared using standard molecular biology techniques known to those of skill in the art. For example, techniques useful for the preparation of suitable vectors are described by Maniatis, et al., Molecular Cloning: A Laboratory Manual, Cold Spring Harbor Laboratory, Cold Spring Harbor, N.Y. An appropriate vector includes, for example, an insertion vector such as the insertion vector described by Capecchi, M. R., Science, 244:1288-92, 1989; or a vector based on a promoter trap strategy or a polyadenylation trap, or "tag-and-exchange" strategy described by Bradley, et al., Biotechnology, 10:543-539, 1992; or Askew, et al., Mol. Cell. Biol., 13:4115-5124, 199.

### Production of a genetically-modified cell

Following production of a suitable expression construct, said expression construct is introduced into a suitable cell. Depending on the type of construct produced the type of cell and method of introduction will vary.

For example, should the expression construct randomly integrate into the genome of a cell and not require homologous recombination, such a construct is preferably introduced into the pronucleus of a fertilized oocyte isolated from an animal to which the construct is to be introduced. Methods for isolating and/or producing a fertilized oocyte will be apparent to the skilled artisan. For example, one or more oocytes are harvested from a female animal and fertilized *in vitro.* Following a suitable time for the pronucleus to form, the expression construct is injected into said pronucleus. The resulting zygote is then maintained for a time and under conditions sufficient for an embryo to form, e.g., for a blastocyst to form. Optionally, the embryo or a cell thereof is screened to detect presence of the expression construct, e.g., by detecting expression of a selectable or detectable marker gene or by PCR or *in situ* hybridization. The embryo is then administered to or implanted into a uterus of a non-human mammal and maintained under conditions for a non-human mammal to develop and be born. Such methods for producing a genetically-modified cell and/or animal are known in the art and/or described in USSN 10/820,777, WO93/14200, WO91/19810, WO93/02189, WO89/00689, WO92/06187, EP0451700, WO92/13069 and WO89/06689. Methods for producing genetically-modified cows using microinjection are described, for example, in Krimpenfort et al., Bio/Technol., 9: 844-847, 1991; Hyttinen et al., Bio/Technol., 12: 606-608, 1994 and/or Eyestone, Theriogeriology, 51: 509-517, 1998.

Alternatively, a genetically-modified non-human mammal is produced by introducing an expression construct or expression vector as described according to any embodiment hereof into a somatic cell, e.g., a fibroblast of a non-human mammal, resulting in a genetically-modified non-human mammalian cell. Methods for introducing a nucleic acid into a somatic cell are known in the art and include, for example, electroporation, microinjection, transfection mediated by DEAE-dextran, transfection mediated by calcium phosphate, transfection mediated by liposomes such as by using Lipofectamine (Invitrogen) and/or cellfectin (Invitrogen), transduction by Adenoviruses, Herpesviruses, Togaviruses or Retroviruses and microparticle bombardment such as by using DNA-coated tungsten or gold particles (Agacetus Inc., WI, USA). The genetically-modified non-human mammalian cell or the nucleus thereof is then injected into or fused with an enucleated mature oocyte from the same species of non-human mammal as the somatic cell to produce a monocell embryo. This monocell embryo is them maintained for a time and under conditions sufficient for a multi-cell embryo to form, e.g., a blastocyst to form, and the multi-cell embryo is then administered to or implanted into a uterus of a non-human mammal and maintained under conditions for a non-human mammal to develop and be born. Exemplary methods for producing a genetically-modified non-human mammal using these methods, e.g., nuclear transfer, will be apparent to the skilled artisan and/or are described, for example, in Schnieke et al. Science, 278: 2131-2133, 1997; and Baguisi et al., Nature Biotech., 17: 456-461, 1999.

In another example, an expression construct is included in a retrovirus particle, preferably containing the envelope protein from vesicular somatic protein. The retrovirus particle is injected between the *zona pellucida* and the membrane of an oocyte of a non-human mammal at a period when the nuclear membrane is absent, e.g., during metaphase II (MII) of the second meiosis. The infected oocyte is then fertilized with a sperm cell, and incubated for a time and under conditions sufficient for an embryo, e.g., a blastocyst to form. The embryo is then administered to, e.g., implanted in a uterus of a female non-human mammal and a genetically-modified non-human mammal permitted to develop and be born. A suitable retrovirus-mediated method for producing a genetically-modified non-human mammal, e.g., a cow or bull, is described in Chan et al., Proc Natl Acad Sci U S A. 95: 14028-33, 1998.

It is to be understood that the genetically modified non-human mammals described herein can be produced by methods other than the specific methods taught herein, e.g., sperm-mediated transgenesis or embryonic stem cell-mediated transgenesis

### Production of a genetically-modified non-human mammal

A fertilized oocyte or a zygote or an embryo into which an expression construct of the invention has been introduced is preferably transferred to a uterus of a pregnant or pseudopregant female non-human mammal and allowed to develop into an entire mammal. Following birth, animals are screened to identify those carrying the expression construct using a method known in the art and/or described herein.

Generally, such a method results in production of a non-human mammal heterozygous for the expression construct of the invention or in which some cells comprise the expression construct and some do not, i.e., a chimeric non-human mammal. By breeding either the non-human mammal heterozyogous for the expression construct or the chimeric non-human mammal with a wild-type non-human mammal offspring that are heterozygous for the expression construct are produced.. Breeding two non-human mammals heterozygous for the expression construct or two non-human mammals homozygous for the expression construct or a non-human mammal heterozygous for the expression construct and a non-human mammal homozygous for the expression construct produces at least some offspring that are homozygous for the expression construct.

The present invention clearly contemplates either a genetically-modified non-human mammal homozygous for a genetic construct as described according to any embodiment hereof or a genetically-modified non-human mammal heterozygous for a genetic construct as described according to any embodiment hereof.

The present invention additionally contemplates a cell, a cell line, a cell culture, a primary tissue, a cellular extract or a cell organelle isolated from a genetically-modified non-human mammal of the present invention. For example, a cell culture or cell line or cell is derived from any desired tissue or cell-type from the genetically-modified non-human mammal, e.g., a mammary gland or cell or tissue thereof, or a stem cell or a reproductive cell, e.g., an oocyte or a sperm.

*Isolation of an antimicrobial peptide and*/*or analog and*/*or derivative from milk* Suitable methods for isolating an antimicrobial peptide and/or analog and/or derivative have been described *supra* and are to be taken to apply *mutatis mutandis* to isolation from milk.

In on example, an antimicrobial peptide and/or analog and/or derivative as described herein is isolated from milk by chromatography and concentration. Different types of chromatography can be employed and include ion exchange chromatography, reverse phase chromatography, molecular exclusion chromatography or affinity chromatography. The ion exchange chromatography can be anion exchange chromatography. The affinity chromatography can be immunoaffinity chromatography. Furthermore, multiple chromatography steps may be performed.

For example, an antimicrobial peptide and/or analog and/or derivative is isolated form milk by clarifying the milk of a non-human genetically-modified mammal as described according to any embodiment hereof, resulting in a clarified milk, and subjecting the clarified milk to chromatography, thereby isolating the peptide and/or analog and/or derivative.

In another example, an antimicrobial peptide and/or analog and/or derivative is isolated from milk by clarifying the milk of a non-human genetically-modified mammal as described according to any embodiment hereof, resulting in a clarified milk, subjecting the clarified milk to expanded-bed anion exchange chromatography, resulting in an anion exchange chromatographed material, subjecting the anion exchange chromatographed material to reverse phase chromatography, resulting in a reverse phase chromatographed material, subjecting the reverse phase chromatographed material to anion exchange chromatography, resulting in an anion exchange chromatographed material, subjecting the anionic exchange chromatographed material to molecular exclusion chromatography, resulting in a molecular exclusion chromatographed material, concentrating the molecular exclusion chromatographed material, resulting in a concentrated material, and subjecting the concentrated material to molecular exclusion chromatography, thereby isolating the antimicrobial peptide and/or analog and/or derivative.

### Determining the antimicrobial activity of a peptide

Methods for determining the antimicrobial activity of a peptide will be apparent to the skilled artisan, for example, based on the description herein. For example, as exemplified herein, the present inventors have used a radial diffusion assay.

Other suitable methods include, for example, a broth dilution method. Essentially, this method involves growing a microorganism in liquid media until log phase is reached. The peptide, analog or derivative to be tested is serially diluted in media in which the microorganism is grown are grown and a sample of the microorganism added to the peptide containing sample. The sample is then maintained for a time and under conditions sufficient for growth of the microorganism, and the amount of growth of the microorganism determined relative to a negative control by detecting the absorbance at A₆₀₀.

Another method disclosed herein comprises contacting a microorganism previously contacted with a peptide to be tested with an agent that has affinity for a compound located within the microorganism, but is not able to cross an intact or undamaged membrane. The presence of the agent within the microorganism indicates that the agent crossed the membrane indicating that the membrane of the microorganism was damaged by the peptide. An example of such an agent is Sytox green dye (Molecular Probes, Eugene, Oreg.). This dye has a strong affinity for nucleic acids, but can only penetrate cells that have a damaged membrane.

Yet another method for determining whether a peptide being assayed for antimicrobial activity has damaged the membrane of the microorganism involves contacting the microorganism with a test peptide and an agent capable of crossing the membrane of the microorganism. The agent is capable of being processed within the microorganism to form a product that is unable to cross an undamaged membrane. The medium surrounding the microorganism is then assayed for the presence of said product. The presence of said product in the medium in which the microorganism is grown is indicative of damage to the membrane of the microorganism caused by the peptide, and is indicative of the antimicrobial activity of the peptide. An example of a suitable agent is calcein AM. Calcein AM is converted into free calcein within the microorganism. Normally, free calcein is unable to cross the cell membrane of the microorganism and enter the surrounding culture. Thus, detection of free calcein in the medium surrounding the microorganism is indicative of damage to the cell membrane of the microorganism, and thus the antimicrobial activity of the peptide.

### Identifying peptides/analogs/derivatives for treating mastitis

Methods for testing a genetically-modified non-human mammal as described according to any embodiment hereof for protection against mastitis will be apparent to the skilled artisan.

In one example, a genetic construct is tested for its ability to protect against mastitis prior to use to produce a genetically-modified non-human mammal. For example, an *in vitro* assay is performed essentially as described in Almeida et al., J. Vet. Pharmacol. Ther., 30: 151-156, 2007, however, modified to test a genetic construct as described according to any embodiment hereof. For example, a mammary epithelial cell line, e.g., a bovine mammary epithelial cell line such as MAC-T is transfected with an expression construct or expression vector as described according to any embodiment hereof and/or contacted with a peptide and/or analog and/or derivative as described herein. Following sufficient time for a peptide or analog or derivative to be expressed and/or secreted the transfected cell is contacted with a bacterium that causes mastitis, e.g., *S. uberis* (e.g., a strain designated UT888) or *S. dysgalactiae* subsp. *dysgalactiae* (e.g., a strain designated UT19) or *S. aureus* (e.g., a strain designated UT23). The bacteria are fluorescently labelled, e.g., with a LIVE/DEAD *Bac*Light viability kit from Molecular Probes, Eugene, OR, USA. Following washing to remove unbound or non-internalized bacteria, cells are viewed under a fluorescent microscope to determine the number of cells internalized and, of those cells internalized the number of cells that are viable. A peptide and/or analog and/or derivative or expression construct or expression vector that reduces the number of internalized and/or viable internalized bacterial cells is considered useful for preventing mastitis.

In another example, a mammary epithelial cell is contacted with bacteria prior to treatment, e.g., transfection with an expression construct or expression vector as described according to any embodiment hereof and/or contact with a peptide and/or analog and/or derivative as described herein. The level of internalized and/or viable internalized bacterial cells is then determined and a peptide and/or analog and/or derivative or expression construct or expression vector that reduces the number of internalized and/or viable internalized bacterial cells is considered useful for preventing mastitis.

In a further example, a genetically-modified non-human mammal is produced by performing a method as described according to any embodiment hereof. At an appropriate time, e.g., during pregnancy and/or during lactation bacteria (e.g., *S. uberis*) that cause mastitis are infused through a teat canal into a teat cistern using, for example, a syringe. Following a sufficient time for an infection to occur and mastitis to develop the non-human mammal is assessed for mastitis development and infection, e.g., by determining the level of somatic cells in milk, and/or culturing bacteria growing in milk. Examples of suitable methods for infusing bacteria into a mammary gland and/or assessing infection/mastitis status will be apparent to the skilled artisan and/or described, for example, in Nickerson et al., J. Dairy Sci., 73: 2774-2784, 1990.

In another example, a non-human mammal is infected with bacteria that cause mastitis and then a peptide and/or analog and/or derivative and/or expression construct is administered to the mammal. Following sufficient time, e.g., for mastitis to develop and/or for a peptide and/or analog and/or derivative to kill or prevent growth of the bacteria and/or for a peptide to be expressed from said expression construct and to kill or prevent growth of the bacteria, the level of infection and/or mastitis is assessed e.g., as described *supra.*

In another example, milk is isolated from a genetically-modified non-human mammal as described according to any embodiment hereof, and the milk is contacted with a sample of bacteria that cause mastitis, e.g., in a radial diffusion method and/or a broth dilution assay as described herein. An exemplary method is described in Wall et al., Nature Biotech., 23: 445-451, 2005.

### Compositions comprising an antimicrobial peptide, analog or derivative

For a composition to be administered to a mammary gland or a cell or tissue thereof, a preferred composition comprises a carrier or excipient is suitable for intra-mammary administration. For example, the carrier or excipient does not inhibit the activity of the peptide and/or analog and/or derivative even following administration to a mammary gland. Preferably the carrier or excipient does not cause inflammation in a mammary gland or a tissue thereof. Preferably the carrier does not change the constitution of milk produced from the mammary gland and/or a milk product produced there from. In one example, the carrier or excipient is a non- phosphate containing isotonic buffer at the physiological pH of milk i.e. pH 6.7.

In another example, the carrier or excipient is a liquid, for example, a physiologic salt solution containing non-phosphate buffer at pH 6.7 to 7.6.

For intra-mammary administration by injection into a mammary gland, a preferred carrier or excipient is oil- based, preferably using mineral oil.

An antimicrobial peptide and/or analog and/or derivative as described herein and/or produced by a method as described according to any embodiment hereof is preferably provided in a composition, e.g., a pharmaceutical composition, a disinfecting composition, a preservative composition, a cosmetic composition or a phytoprotective composition. Such a composition additionally comprises, for example, a suitable carrier, e.g., pharmaceutically acceptable carrier. The term "carrier" as used herein, refers to a carrier that is conventionally used in the art to facilitate the storage, administration, and/or the biological activity of a regulatory agent. A carrier may also reduce any undesirable side effects of the regulatory agent. A suitable carrier is stable, i.e., incapable of reacting with other ingredients in the formulation. The carrier does not produce significant local or systemic adverse effect in recipients at the dosages and concentrations employed for treatment. Such carriers are generally known in the art. Suitable carriers for this invention include those conventionally used. Water, saline, aqueous dextrose, and glycols are preferred liquid carriers, particularly (when isotonic) for solutions. Alternatively, the carrier is selected from various oils, including those of petroleum, animal, vegetable or synthetic origin, for example, peanut oil, soybean oil, mineral oil, sesame oil, and the like. Suitable pharmaceutical carriers include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like.

A composition comprising an antimicrobial peptide of the invention or a derivative or analog thereof can be subjected to conventional pharmaceutical expedients, such as sterilization, and can contain conventional pharmaceutical additives, such as preservatives, stabilizing agents, wetting, or emulsifying agents, salts for adjusting osmotic pressure, buffers, and the like. Other acceptable components in the composition of the invention include, but are not limited to, isotonicity-modifying agents such as water, saline, and buffers including phosphate, citrate, succinate, acetic acid, and other organic acids or their salts. However, because an antimicrobial peptide of the present invention is a soluble hydrophilic molecule, sprays, solutions, lotions and topical ointments for administration are readily formulated without the need for chemical solvent-based solubilizing agents, which may be detrimental to a subject to which the peptide is to be administered.

Preferably a composition of the invention also includes one or more stabilizers, reducing agents, anti-oxidants and/or anti-oxidant chelating agents. The use of buffers, stabilizers, reducing agents, anti-oxidants and chelating agents in the preparation of protein-based compositions, is known in the art and described, for example, in Wang et al. J. Parent. Drug Assn. 34:452-462, 1980; Wang et al. J. Parent. Sci. Tech. 42:S4-S26 (Supplement), 1988. Suitable buffers include acetate, adipate, benzoate, citrate, lactate, maleate, phosphate, tartarate, borate, tri(hydroxymethyl aminomethane), succinate, glycine, histidine, the salts of various amino acids, or the like, or combinations thereof. Suitable salts and isotonicifiers include sodium chloride, dextrose, mannitol, sucrose, trehalose, or the like. Where the carrier is a liquid, it is preferred that the carrier is hypotonic or isotonic with oral, conjunctival, or dermal fluids and has a pH within the range of 4.5-8.5. Where the carrier is in powdered form, it is preferred that the carrier is also within an acceptable non-toxic pH range.

An antimicrobial peptide of the invention or analog or derivative thereof may be incorporated within a composition for administration to a mucus membrane, e.g., by nasal administration. Such a composition generally includes a biocompatible polymer functioning as a carrier or base. Such polymer carriers include polymeric powders, matrices or microparticulate delivery vehicles, among other polymer forms. The polymer can be of plant, animal, or synthetic origin. Often the polymer is crosslinked. Additionally, in these delivery systems the biologically active agent, can be functionalized in a manner where it can be covalently bound to the polymer and rendered inseparable from the polymer by simple washing. Polymers useful in this respect are desirably water interactive and/or hydrophilic in nature to absorb significant quantities of water, and they often form hydrogels when placed in contact with water or aqueous media for a period of time sufficient to reach equilibrium with water.

Drug delivery systems based on biodegradable polymers are preferred in many biomedical applications because such systems are broken down either by hydrolysis or by enzymatic reaction into non-toxic molecules. The rate of degradation is controlled by manipulating the composition of the biodegradable polymer matrix. These types of systems can therefore be employed in certain settings for long-term release of biologically active agents. Examples of suitable biodegradable polymers include, for example, poly(glycolic acid) (PGA), poly-(lactic acid) (PLA), and poly(D,L-lactic-coglycolic acid) (PLGA).

Alternatively, a peptide or analog or derivative thereof of the invention can be administered via *in vivo* expression of the recombinant protein. *In vivo* expression can be accomplished via somatic cell expression according to suitable methods (see, e.g. U.S. Pat. No. 5,399,346). In this embodiment, nucleic acid encoding the protein can be incorporated into a retroviral, adenoviral or other suitable vector (preferably a replication deficient infectious vector) for delivery, or can be introduced into a transfected or transformed host cell capable of expressing the protein for delivery. In the latter embodiment, the cells can be implanted (alone or in a barrier device), injected or otherwise introduced in an amount effective to express the protein in a therapeutically effective amount.

The antimicrobial peptides of the invention may be used in combination with or to enhance the activity of other antimicrobial agents or antibiotics. Combinations of the peptides with other agents may be useful to allow antibiotics to be used at lower doses due to toxicity concerns, to enhance the activity of antibiotics whose efficacy has been reduced or to effectuate a synergism between the components such that the combination is more effective than the sum of the efficacy of either component independently. Antibiotics that may be combined with an antimicrobial peptide in combination therapy include but are not limited to penicillin, ampicillin, amoxycillin, vancomycin, cycloserine, bacitracin, cephalolsporin, methicillin, streptomycin, kanamycin, tobramycin, gentamicin, tetracycline, chlortetracycline, doxycycline, chloramphenicol, lincomycin, clindamycin, erythromycin, oleandomycin, polymyxin nalidixic acid, rifamycin, rifampicin, gantrisin, trimethoprim, isoniazid, paraminosalicylic acid, and ethambutol.

As exemplified herein, a peptide and/or analog and/or derivative of the present invention has a synergistic effect on the antimicrobial activity of chloramphenicol and/or tetracycline. Accordingly, a composition may comprise a peptide and/or analog and/or derivative of the present invention and tetracycline or a structurally and functionally-related antibiotic and/or chloramphenicol and/or a structurally and functionally-related antibiotic.

The composition may be a disinfecting or preservative composition, e.g., for cleaning a surface and/or for preserving food or pharmaceuticals. Such a composition comprises a suitable carrier, such as, for example, as described *supra.* Such a composition also preferably comprises one or more protease inhibitors to reduce or prevent degradation of the antimicrobial peptide of the invention.

The composition may be a phytoprotective composition. Such a composition is, for example, sprayed onto or applied to a plant or soil in which a plant is grown or is to be grown to prevent a microbial infection or to treat a microbial infection.

As will be apparent to the skilled artisan based on the foregoing, a preferred composition is suitable for spray application. For example, the composition is suitable for spraying onto a food product or onto a food preparation surface or onto a plant. Such spray compositions are useful for the treatment of food, e.g., to prevent food spoilage without actually handling the food. The skilled artisan will be aware of suitable components of a composition suitable for spray application. For example the composition comprises an antimicrobial peptide or analog or derivative as described herein and a suitable carrier, e.g., water or saline. Such a composition may also comprise, for example, a surfactant, e.g., Tween 20, preferably a surfactant does not inhibit or reduce the antimicrobial activity of said peptide, analog or derivative.

A peptide described herein may be applied to a surface of a device to prevent microbial proliferation on that surface of the device. The device is, for example, a medical device, which includes any material or device that is used on, in, or through a patient's body in the course of medical treatment (e.g., for a disease or injury). Medical devices include but are not limited to such items as medical implants, wound care devices, drug delivery devices, and body cavity and personal protection devices. The medical implants include but are not limited to urinary catheters, intravascular catheters, dialysis shunts, wound drain tubes, skin sutures, vascular grafts, implantable meshes, intraocular devices, heart valves, prosthetic devices (e.g., hip prosthetics) and the like. Wound care devices include but are not limited to general wound dressings, biologic graft materials, tape closures and dressings, and surgical incise drapes. Drug delivery devices include but are not limited to needles, drug delivery skin patches, drug delivery mucosal patches and medical sponges.

### Routes of administration

In one example, a peptide and/or analog and/or derivative or composition comprising same is administered to a mammary gland to thereby treat or prevent mastitis. The peptide and/or analog and/or derivative or composition may be infused into a canal of a mammary gland, e.g., by dipping a teat or mammary gland into a composition peptide and/or analog and/or derivative or by infusion by injection into a canal of a mammary gland. Alternatively, the peptide and/or analog and/or derivative or composition is injected directly into a mammary gland or a region thereof.

In one example, an expression construct or expression vector of the present invention is administered to a mammary gland or a cell or tissue thereof by high-pressure jet-injection, e.g., as described in Kerr et al., Anim. Biotechnol., 7: 33-45, 1996 or Zheng et al., Gene, 353: 107-117, 2005. For example, using high-pressure jet-injection Kerr *et al.,* delivered naked DNA into parenchyma of lactating sheep parenchyma.

In another example, an expression construct or expression vector of the present invention is administered to a mammary gland or cell or tissue thereof in a virus, e.g., an adenovirus. For example, a replication defective adenovirus, e.g., human adenovirus comprising an expression construct of the invention is produced and infused or injected into a canal of a mammary gland. For example, US Pat. No. 6,875,903 describes suitable methods for administering an adenovirus comprising an expression construct to a mammary gland or a cell or tissue thereof of a ruminant animal.

Other viruses will be apparent to the skilled artisan and include, for example, a retrovirus, e.g., a lentivirus, or an adeno-associated virus.

For example, a retroviral vector generally comprises cis-acting long terminal repeats (LTRs) with packaging capacity for up to 6-10 kb of foreign sequence. The minimum cis-acting LTRs are sufficient for replication and packaging of a vector, which is then used to integrate the expression construct into the target cell to provide expression. Widely used retroviral vectors include those based upon murine leukemia virus (MuLV), gibbon ape leukemia virus (GaLV), simian immunodeficiency virus (SIV), human immunodeficiency virus (HIV), and combinations thereof (see, e.g., Buchscher et al., J. Virol. 66:2731-2739 (1992); Johann et al., J. Virol. 66:1635-1640 (1992); Sommerfelt et al., Virol. 176:58-59 (1990); Wilson et al, J. Virol. 63:274-2378 (1989); Miller et al., J. Virol. 65:2220-2224 (1991); PCT/US94/05700; Miller and Rosman BioTechniques 7:980-990, 1989; Miller, A. D. Human Gene Therapy 1:5-14, 1990; Scarpa et al) Virology 180:849-852, 1991; Burns et al. Proc. Natl. Acad. Sci. USA 90:8033-8037, 1993.).

Various adeno-associated virus (AAV) vector systems have also been developed for nucleic acid delivery. AAV is a helper-dependent DNA parvovirus which belongs to the genus Dependovirus. AAV has no known pathologies and is incapable of replication without additional helper functions provided by another virus, such as an adenovirus, vaccinia or a herpes virus, for efficient replication and a productive life cycle. In the absence of the helper virus, AAV establishes a latent state by insertion of its genome into a host cell chromosome. Subsequent infection by a helper virus rescues the integrated copy which can then replicate to produce infectious viral progeny. The combination of the wild type AAV virus and the helper functions from either adenovirus or herpes virus generates a recombinant AVV (rAVV) that is capable of replication. One advantage of this system is its relative safety (For a review, see Xiao et al., (1997) Exp. Neurol. 144: 113-124). Vectors containing as little as 300 base pairs of AAV can be packaged and can integrate. Space for exogenous DNA is about 4.7 kb, which is sufficient to incorporate a nucleic acid encoding a peptide or analog or derivative of the present invention. An AAV vector such as that described in Tratschin et al. , (1985) Mol. Cell. Biol. 5: 3251-3260 can be used to introduce DNA into cells. A variety of nucleic acids have been introduced into different cell types using AAV vectors (see for example Hermonat et al. , (1984) PNAS USA 81: 6466-6470; Tratschin et al. , (1985) Mol. Cell. Biol. 4: 2072-2081 ; Wondisford et al., (1988) Mol. Endocrinol. 2: 32-39; Tratschin et al. , (1984) J. Virol. 51: 611-619; and Flotte et al. , (1993) J. Biol. Chem. 268: 3781-3790). An AAV-based expression vector typically includes the 145 nucleotide AAV inverted terminal repeats (ITRs) flanking a restriction site that can be used for subcloning of a desired nucleic acid, either directly using the restriction site available, or by excision of the desired nucleotide sequence with restriction enzymes followed by blunting of the ends, ligation of appropriate DNA linkers, restriction digestion, and ligation into the site between the ITRs. For additional detailed guidance on AAV technology which may be useful in the practice of the subject invention, including methods and materials for the incorporation of a nucleotide sequence, the propagation and purification of the recombinant AAV vector containing the nucleotide sequence, and its use in transfecting cells and mammals, see e. g. Carter et al, US Patent No. 4,797, 368 (10 Jan 1989) ; Muzyczka et al, US Patent No. 5,139, 941 (18 Aug 1992); Lebkowski et al, US Patent No. 5,173, 414 (22 Dec 1992); Srivastava, US Patent No. 5,252, 479 (12 Oct 1993); Lebkowski et al, US Patent No. 5,354, 678 (11 Oct 1994); Shenk et al, US Patent No. 5,436, 146 (25 July 1995) ; Chatterjee et al, US Patent No. 5,454, 935 (12 Dec 1995), Carter et al WO 93/24641 (published 9 Dec 1993), and Natsoulis, U. S. Patent No. 5,622, 856 (April 22,1997).

### Additional uses of an antimicrobial peptide and/or analog and/or derivative

The antimicrobial activity of the peptides of the present invention also make them useful for treating or preventing an infection in a subject. Accordingly, the present invention also provides an expression construct or expression vector or composition of the invention or composition comprising same for use in a method of therapeutic or prophylactic treatment of a subject comprising administering the expression construct or expression vector or composition of the invention or composition comprising same to a subject in need thereof. In this respect, a subject in need of treatment is, for example, a subject suffering from an infection or suspected of suffering from an infection or at risk of developing an infection.

As used herein, the term "subject" shall be taken to mean any animal, including a human, plant or insect that may be infected by a microorganism. Preferably the subject is any animal, including a human that may be infected by a microorganism against which a peptide, analog, derivative, fusion protein, complex or composition of the invention has antimicrobial activity.

Preferably the peptide is administered under conditions sufficient for the peptide, analog and/or derivative to reduce or prevent microbial growth and/or to kill a microorganism, e.g., in a pharmaceutical composition or a cosmetic composition.

As used herein, the term "infection" shall be taken to mean the invasion, development and/or multiplication of a microorganism within or on another organism. An infection may be localized to a specific region of an organism or systemic. Infections for which a peptide, analog and/or derivative of the invention are useful for treating include any infection caused by a bacteria or a fungus and will be apparent to the skilled artisan from the disclosure herein.

In this respect, the present disclosure is not limited to the treatment of an infection in an animal subject. Rather, a peptide, analog, derivative, fusion protein, complex or composition of the present disclosure is also useful for, for example, treatment of a plant to thereby reduce or prevent a microbial infection therein or thereon. Accordingly, the peptide of the invention or analog or derivative thereof is a phytoprotective agent.

In a preferred embodiment, the subject is an animal, and more preferably a mammal. Accordingly, the peptide of the invention or analog or derivative thereof is a pharmaceutical agent or a cosmetic agent.

The peptide, analog and/or derivative of the invention can be administered to a subject by any of a variety of means, such as, for example, topical administration, nasal administration, oral administration, vaginal administration, rectal administration, intravenous administration, intraperitoneal administration, or subcutaneous administration. For example, as infectious microorganisms generally enter a mammal by way of a membrane, e.g., a mucus membrane, a peptide, analog or derivative of the invention is preferably administered in a manner suitable to contact a membrane.

In a preferred embodiment, use in a method of treating a subject of the invention additionally comprises providing or obtaining the peptide, analog, derivative, fusion protein, complex or composition of the invention or information concerning same.

As will be apparent to the skilled artisan based on the foregoing, the present invention also provides for the use of an expression construct or expression vector or composition of the invention in medicine. For example, the present invention provides for the use of the expression construct or expression vector of the present invention in the manufacture of a medicament for the treatment or prophylaxis of an infection.

The present inventors have demonstrated that the peptides of the present invention are active against a variety of microorganisms. Accordingly, also disclosed herein is a method for reducing or preventing microbial growth, said method comprising contacting a microorganism or a surface or composition of matter suspected of comprising a microorganism with a peptide, analog, derivative, fusion protein, complex or composition of the invention for a time and under conditions sufficient to reduce microbial growth and/or kill a microorganism, thereby reducing or preventing microbial growth. Such a method is suitable for, for example, disinfecting a surface and/or preserving a food product and/or reducing or preventing water contamination.

Alternatively, or in addition, the method comprises applying a peptide, analog, derivative, fusion protein, complex or composition of the invention to a surface or composition of matter suspected of comprising a microorganism for a time and under conditions sufficient to reduce microbial growth and/or kill a microorganism, thereby reducing or preventing microbial growth. For example, the peptide, analog, derivative, fusion protein, complex or composition of the invention is sprayed onto the surface or composition of matter. Such spray application is useful for, for example, applying a peptide, analog or derivative of the invention to a food product or a fluid to be consumed, e.g., by a human. This is because spraying the peptide, analog, derivative, fusion protein, complex or composition reduces the handling of said food product or fluid, thereby further reducing the risk of microorganism contamination.

The method may additionally comprise performing a method to detect the presence of a microorganism. Such a detection method may be performed prior to and/or following contacting with a peptide, analog, derivative, fusion protein, complex or composition of the invention.

As will be apparent to the skilled artisan based on the foregoing, also disclosed herein is the use of a peptide, analog, derivative, fusion protein or complex of the invention in the manufacture of a composition for reducing or preventing microbial growth.

As a peptide of the present invention is useful for reducing microbial growth in a food product, additionally disclosed herein is a method for prolonging the storage life of a perishable product, said method comprising:
(i) contacting a perishable product with the peptide, analog, derivative, fusion protein, complex or composition of the present invention for a time and under conditions sufficient to reduce or prevent growth of a microorganism and/or to kill a microorganism; and
(ii) storing the perishable product.

In this respect, the perishable product is capable of being stored for a longer period of time than the same product that has not been contacted with the peptide, analog, derivative, fusion protein, complex or composition of the invention.

Moreover, disclosed herein is a method for preserving milk or a milk product, said method comprising obtaining milk from a genetically-modified non-human mammal as described according to any embodiment hereof or a milk product produced there from, wherein said milk or milk product comprises an antimicrobial peptide and/or analog and/or derivative of the invention that is active therein, thereby preserving the milk or milk product.

In one example, the method comprises obtaining milk from a genetically-modified non-human mammal as described according to any embodiment hereof and producing a milk product there from.

Also disclosed herein is milk or a milk product comprising a peptide and/or analog and/or derivative of the present invention.

The present invention is described further in the following non-limiting examples.

### EXAMPLE 1

### Peptides having antimicrobial activity against a variety of microorganisms including mastitis causing microorganisms

This example demonstrates the antimicrobial profile of synthetic peptides designated AGG01 (SEQ ID NO: 7) and AGG02 (SEQ ID NO: 8) against agents of mastitis.

### Synthetic peptides

Two amidated peptides were commercially synthesized by Auspep. The sequences of the peptides are as follows:
KRGFGKKLRKRLKKFRNSIKKRLKNFNVVIPIPLP-NH₂ (SEQ ID NO: 7); and
KRGLWESLKRKATKLGDDIRNTLRNFKIKFPVPRQ-NH₂ (SEQ ID NO: 8).

### Antimicrobial assays

Peptides were tested for antimicrobial activity against *Streptococcus uberis, Escherichia coli* DH5α, *Escherichia coli* DH5α comprising an ampicillin resistant gene, *Pseudomonas spp., Pseudomonas vulgaris, Proteus vulgaris, Pseudomonas aeruginosa* (ATCC 27853), *Salmonella choleraesuis* (ATCC 14028), *Bacillacs subtilis, Staphylococcus aureus* (ATCC 25923), *Streptococcus pyogenes* (ATCC 19615), *Streptococcus Agalactiae* (ATCC 12927), *Streptococcus equi equi* (β-Haemolytic streptococcus) (ATCC 9527), and the yeast *Candida albicans* (ATCC753), by a two stage radial diffusion assay essentially as described in Steinberg and Lehrer, Methods Mol. Biol., 78: 169-88, 1997. Briefly, approximately 4 X 10⁶ of mid-logarithmic-phase organisms were grown on plates in 11 ml of warm 0.8% agarose containing 0.03% (w/v) Trytpicase soy broth (TSB) powder, with or without 100 mM NaCl, buffered with 10 mM sodium phosphate, pH 7.4. In the case *of S. uberis* bacteria were grown on plates comprising 5% horse serum. The test peptide was serially diluted in acidified water (0.01% acetic acid), and 5 µl of diluted peptide sample was loaded in a 2.5 diameter well in the agarose. A 10 ml overlay gel composed of 6% TSB, 0.8% agarose and 10 mM sodium phosphate buffer (pH 7.4) was poured into each well. Plates were then incubated overnight to allow the surviving organisms to form microcolonies. The clear zone were measured to the nearest 0.1 mm using a magnified transilluminator and expressed in units (1mm =10 U) after subtracting the well diameter. The minimum inhibitory concentration (MIC) is defined by the χ intercept of a regression line through zone diameters obtained from a series of serially diluted peptide samples.

### Results

Table 1 shows the minimum inhibitory concentration (MIC) of each of the antimicrobial peptides set forth in SEQ ID Nos: 7 and 8 required to inhibit a range of gram-negative bacteria, gram positive bacteria and a fungus. Data in Table 1 are presented as means ± standard error of the mean (SEM) from two experiments. Partial inhibition without obvious definition of a clear zone is indicated by asterisks (**). The MICs obtained for a peptide comprising an amino acid sequence set forth in SEQ ID Nos: 7 and 8 in low salt are also represented graphically in Figures 1a and 1b.

**TABLE 1**

| **Microorganism** | **MIC (µg/ml) in media containing 0 mM NaCl or 100 mM NaCl** | | | |
|---|---|---|---|---|
| | **SEQ ID NO: 7** | | **SEQ ID NO: 8** | |
| | **0 mM** | **100 mM** | **0 mM** | **100 mM** |
| **Gram-negative bacteria** | | | | |
| *S. uberis* | 4.91 ± 0.11 | 6.60 ± 0.18 | 2.34 ± 0.18 | 17.67 ± 0.13 |
| *E. coli* DH5α | 1.75 ± 0.22 | 1.32 ± 0.35 | 19.97 ± 0.41 | 26.10 ± 0.41 |
| *Pseudomonas spp* | 1.80 ± 0.30 | 1.83 ± 0.23 | 15.94 ± 0.29 | 22.12 ± 10.43 |
| *P. aeruginosa* (ATCC 28753) | 2.28 ± 0.53 | 1.51 ± 0.51 | 9.19 ± 0.41 | 10.45 ± 0.24 |
| *Salmonella choleraesuis* (ATCC 14028) | 3.46 ± 0.66 | 2.05 ± 0.62 | 9.32 ± 0.38 | ** |
| *Proteus vulgaris* | 1.64 ± 0.32 | 1.73 ± 0.23 | 9.82 ± 0.28 | 67.45 ± 0.20 |

| **Gram**-**positive bacteria** | | | | |
|---|---|---|---|---|
| *Bacillus subtilis* | 1.99 ± 0.38 | 13.83 ± 0.45 | 2.74 ± 0.48 | 8.67 ± 0.39 |
| *Staphylococcus aureus* (ATCC 25923) | 5.72 ± 0.37 | ** | 5.44 ± 0.49 | ** |
| *Streptococcus pyogenes* (ATCC 19615) | 2.42 ± 0.25 | 3.57 ± 0.41 | 1.19 ± 0.37 | 8.24 ± 0.37 |
| *Streptococcus equi equi* (ATCC 9527) | 2.39 ± 0.35 | 4.05 ± 0.39 | 4.85 ± 0.35 | ** |
| *Streptococcus agalactiae* (ATCC 12927*)* | 3.81 | | 1.2 | |

| **Fungus** | | | | |
|---|---|---|---|---|
| *Candida albicans* (ATCC 753) | 5.48 ± 0.16 | ** | 10.01 ± 0.47 | |

The data presented in Table 1 and Figures 1a and 1b indicate a broad spectrum of activity for the identified antimicrobial peptides, in low and high salt concentrations. The maintenance of antimicrobial activity in high salt suggests efficacy in body fluids, such as, for example, blood.

SEQ ID NO: 7 appears active against all microorganisms tested at less than 10 µg/ml, these low MIC values suggesting that the base peptide and analogs and derivatives thereof having enhanced activity and/or half-life, are particularly strong candidates for development into therapeutic formulations. SEQ ID NO: 7 was capable of inhibiting growth of and/or killing *S. uberis* in both high and low salt concentrations. Moreover, SEQ ID NO: 7 also exhibited antimicrobial activity against other pathogens that cause mastitis, e.g., *E. coli, S. aureus* and *S. agalactiae.*

SEQ ID NO: 8 also has antimicrobial activity against *S*. *uberis* and other organisms causative of mastitis e.g., *E. coli, S. aureus* and *S*. *agalactiae.*

In separate experiments, the peptide AGG01 (SEQ ID NO: 7) or AG002 (SEQ ID NO: 8) is shown to be suitable for use in a dairy starter culture comprising lactobacilli, especially one or more *Lactis spp.,* in particular one or more organisms selected individually or collectively from the group consisting of: *L. helveticus, L. acidophilus, L. lactis, L. bugaricus* and *L. citrovorum,* and especially *L. acidophilus.* This low activity against lactobacilli suggests utility of the peptides in dairy starter cultures.

### EXAMPLE 2

### Production of additional antimicrobial peptides by mutagenesis

This example demonstrates the production of new synthetic antimicrobial peptides by evolution of antimicrobial peptides of the invention and C- termini of cathelicidin proteins.

### Peptide synthesis

Several mutagenesis approaches were employed to generate peptides having antimicrobial activity based on the sequences of peptides comprising SEQ ID NO: 7 and/or 8.

In a first process, the nucleotide sequences of nucleic acids encoding SEQ ID Nos: 7 and 8 were aligned, and codons encoding variable amino acids identified. A nucleotide sequence was then determined that was capable of encoding a sequence comprising an amino acid at any position that occurs in either SEQ ID NO: 7 or SEQ ID NO: 8. This consensus nucleotide sequence is set forth in SEQ ID NO: 88. Synthetic nucleic acids comprising possible sequences conforming to the sequence set forth in SEQ ID NO: 88 were then synthesized by PCR using degenerate olignonucleotides. Exemplary sequences conforming to the consensus sequence set forth in SEQ ID NO: 88 are set forth in SEQ ID NO: 90 and SEQ ID NO: 91.

In a second process, the nucleotide sequences of nucleic acids encoding antimicrobial peptide domains of cathelicidins from a number of different species were aligned and codons encoding variable amino acids were identified. Nucleotide sequences were determined that are capable of encoding the aligned sequences. Synthetic nucleic acids comprising possible sequences conforming to the aligned sequences were then synthesized by PCR using degenerate oligonucleotides. Exemplary encoded sequences derived by this approach are set forth in SEQ ID Nos: 23-32.

In a third process, a pool of overlapping degenerate oligonucleotides were produced that span the aligned lengths of SEQ ID NOs: 7 and 8, wherein the degenerate oligonucleotides comprise the sequences set forth in SEQ ID Nos: 113-120 (Figure 2a). These oligonucleotides were then used in a splice overlap extension protocol to produce a single nucleic acid. Briefly, the reaction was performed using a 50 µl reaction containing 200 µM of each dNTP, 0.1 µM of each oligonucleotide, 0.5 U of Platinum Taq DNA Polymerase (Invitrogen) in a buffer containing 1.5 mM MgCl₂. The reaction was then performed with the following conditions: denaturation at 94°C for 5 min, followed by 30 cycles at 94°C for 30 s 20/35°C for 30 s and 72°C for 30s; or denaturation at 94°C for 5 min followed by 10 cycles at 94°C for 30 s, 30°C for 30 s, 72°C for 30s and then followed by anther 30 cycles at 94°C for 30 s, 50°C for 30 s and 72°C for 30 s, and terminated by an incubation at 72°C for 7 min. Following overlap extension, 1-2 µl of the amplification reaction was used as a template for a standard PCR in a 50 µl solution using the same reaction solution as described *supra,* under the same conditions as before except with primers comprising sequences set forth in SEQ ID NOs: 121 and 122. PCR cycling was conducted as follows: denaturation at 94°C for 5 min followed by 30 cycles at 95°C for 30 s, 55°C for 30 s and 72°C for 30 s, and terminated by incubation at 72°C for 7 min.

In a third process, a pool of overlapping degenerate oligonucleotides were produced that span the aligned lengths of SEQ ID NOs: 7 and 8, wherein the degenerate oligonucleotides comprise the sequences set forth in SEQ ID Nos: 123-126 (Figure 2b). These oligonucleotides were then used in a splice overlap extension protocol to produce a single nucleic acid. Briefly, the reaction was performed using a 50 µl reaction containing 200 µM of each dNTP, 0.1 µM of each oligonucleotide, 0.5 U of Platinum Taq DNA Polymerase (Invitrogen) in a buffer containing 1.5 mM MgCl₂. The reaction was then performed with the following conditions: denaturation at 94°C for 5 min, followed by 30 cycles at 94°C for 30 s 20/35°C for 30 s and 72°C for 30s; or denaturation at 94°C for 5 min followed by 10 cycles at 94°C for 30 s, 30°C for 30 s, 72°C for 30s and then followed by anther 30 cycles at 94°C for 30 s, 50°C for 30 s and 72°C for 30 s, and terminated by an incubation at 72°C for 7 min. Following overlap extension, 1-2 µl of the amplification reaction was used as a template for a standard PCR in a 50 µl solution using the same reaction solution as described *supra,* under the same conditions as before except with primers comprising sequences set forth in SEQ ID NOs: 127 and 128. PCR cycling was conducted as follows: denaturation at 94°C for 5 min followed by 30 cycles at 95°C for 30 s, 55°C for 30 s and 72°C for 30 s, and terminated by incubation at 72°C for 7 min.

Amplicons produced using each of the approaches *supra* were directly cloned into pGEM-T easy vector (Promega), essentially according to manufacturer's instructions for sequencing to confirm their sequences. Alternatively, the amplicons were cloned into pBAD/gIIIA vector (Invitrogen) and transformed into an *E. coli* stain TOP10 host for expression and screening purposes.

### Sequence analyses

To confirm that the processes described *supra* produced variant sequences, sequence analysis of the recombinant mutants was performed using nucleic acid from the various clones produced using fully synthesized oligonucleotides or by splice overlap extension. The sequences of peptides identified by these approaches and having antimicrobial activity are shown in SEQ ID Nos: 10-32.

### Screening of peptides for antimicrobial activity

Screens were performed based on the principle that over expressing an antimicrobial peptide in bacteria will kill the host bacteria or inhibit the growth of the host bacteria. Screens were performed with recombinant nucleic acids cloned into the pBAD/gIIIA vector (Invitrogen), which contains secretion signal gene IIIA which targets proteins to the periplasmic space e.g., of *E. coli.* The nucleic acids were clones so as to produce an in-frame fusion between the encoded peptide and an upstream translation start site and downstream hexahistidine-encoding sequence and translation termination signal provided by the vector. As a positive control nucleic acids encoding the parental peptides were cloned into the pBAD/gIIIA vector.

Growth inhibition assays on solid LB medium were also performed essentially as described in Example 1. Briefly, primary expression was carried in several bacterial strains, i.e., *E. coli* strains DH5α, TOP10 and LMG194 in LB medium. Growth inhibition obviously appears in TOP10 and LMG194 stains two hours after 0.02% or 0.002% L-arabinose induction.

More particularly, recombinant nucleic acids were digested with *Nco*I and *Xho*I and cloned into the same sites of pBAD/gIIIA vector (Figure 3) and then transformed into *E. coli* TOP10 cells. Colonies were randomly picked and sub-cultured into a 48-well plate with LB medium containing 100 µg/ml ampicillin per well at 37°C overnight. Colonies expressing parental peptides or calmodulin were used as controls. Overnight culture was then subjected to the solid medium screen. At the same time, a 1:100 dilution of the overnight culture was also used in a liquid screen. An OD600 value was determined before induction of expression of peptides using L-arabinose and 4 hours after induction. A group of peptides capable of inhibiting bacterial growth and/or killing bacteria were then identified, in particular peptides designated A12 (SEQ ID NO: 10), 5 (SEQ ID NO: 11), 6 (SEQ ID NO: 12), 13 (SEQ ID NO: 13), A4 (SEQ ID NO: 14), A5 (SEQ ID NO: 15), A6 (SEQ ID NO: 16), A10 (SEQ ID NO: 17), A11 (SEQ ID NO: 18), 10 (SEQ ID NO: 19), 20 (SEQ ID NO: 20), 27 (SEQ ID NO: 21), 28 (SEQ ID NO: 22), B1 (SEQ ID NO: 23), B6 (SEQ ID NO: 24), E3 (SEQ ID NO: 25), F4 (SEQ ID NO: 26), F6 (SEQ ID NO: 27), F7 (SEQ ID NO: 28), F12 (SEQ ID NO: 29), G9 (SEQ ID NO: 30), G10 (SEQ ID NO: 31) and H6 (SEQ ID NO: 32) were identified as having antimicrobial activity.

The growth inhibition curves of expression of the parental peptides with control of vector expressing calmodulin is shown in Figures 4a and 4b. Peptides designated 5, 6, 13, A12, B6, E3, F7 and G9 *inter alia* inhibited bacterial growth and/or killed bacteria to a greater extent than the parental peptide comprising a sequence set forth in SEQ ID NO: 7 or SEQ ID NO: 8 (Figure 3a, Figure 3b).

### EXAMPLE 3

### Stability of antimicrobial peptides in milk

This example demonstrates the resistance of the bioactive synthetic antimicrobial peptide designated AGG01 (SEQ ID NO: 7) to proteolysis by milk proteases, thereby showing utility of the peptide in mammary glands or secretions thereof before or during or after lactation, or as a milk additive.

To determine whether or not antimicrobial peptides are bioactive in milk peptides comprising a sequence set forth in SEQ ID NO: 7 or SEQ ID NO: 8 were diluted in either 10mM phosphate buffer or fresh or pasteurized milk to a final concentration of 200 µg/ml. Treatment groups are shown in Table 2.

**TABLE 2**

| Peptide | 1 | 2 | 3 | 4 | 5 | 6 |
|---|---|---|---|---|---|---|
| | | Fresh milk | | Pasteurized milk | | |
| SEQ ID NO: 7 | In sodium phosphate buffer, 4°C, 1 hour | 37°C, 30 min | 37°C, 60 min | 37°C, 30 min | 37°C, 60 min | Milk only (fresh) |
| SEQ ID NO: 8 | In sodium phosphate buffer, 4°C, 1 hour | 37°C, 30 min | 37°C, 60 min | 37°C, 30 min | 37°C, 60 min | Milk only (pasteurized) |

Peptides having or comprising sequences set forth in any one of SEQ ID Nos: 9-83 hereof are tested similarly to demonstrate their stability in milk.

In this example, peptide AGG01 (SEQ ID NO: 7) showed antimicrobial activity in both fresh and pasteurized milk (data not shown). The antimicrobial peptide was retained in fresh milk, however was slightly reduced in pasteurized milk. Accordingly, SEQ ID NO: 7 retains bioactivity in milk, making this peptide useful for expression in a mammary gland or cell or tissue thereof and/or secretion into milk, e.g., to treat mastitis and/or to produce the peptide, e.g., as a bioreactor. Bioactivity of a peptide comprising the sequence set forth in SEQ ID NO: 8 was reduced or inhibited in the presence of fresh or pasteurized milk. Accordingly, SEQ ID NO: 8 is useful for expressing in a mammary gland or cell or tissue thereof before lactation, e.g., during pregnancy, to thereby prevent mastitis or infection by a microorganism that causes mastitis. Without being bound by theory or mode of action, sequence differences between SEQ ID NO: 7 and 8 may explain these different stabilities in milk e.g., by virtue of the presence of one or more protease recognition sequences in SEQ ID NO: 8 that are missing from SEQ ID NO: 7.

### EXAMPLE 4

### Heat resistance of antimicrobial peptides

This example demonstrates the heat resistance of the bioactive synthetic antimicrobial peptides designated AGG01 (SEQ ID NO: 7) and AGG02 (SEQ ID NO: 8) to heat treatment similar to that employed during pasteurisation processes for milk products, thereby showing utility of the AGG01 peptide at least as a milk additive before, during or after pasteurization occurs, and the utility of both peptides in non-dairy environments requiring heat treatments.

To determine whether or not antimicrobial peptides are bioactive following heating, e.g., following pasteurization peptides comprising a sequence set forth in SEQ ID NO: 7 or SEQ ID NO: 8 were diluted in either 10mM phosphate buffer or fresh or pasteurized milk to a final concentration of 200 µg/ml and incubated at any of a variety of temperatures. Treatment groups are shown in Tables 3 and 4.

**TABLE 3**

| Peptide | 1 | 2 | 3 | 4 | 5 | 6 |
|---|---|---|---|---|---|---|
| SEQ ID NO: 7 | In sodium phosphate buffer, 4°C, 1 hour | In pasteurised milk, 4°C, 1 hour | In pasteurised milk, 37°C, 30 min | In pasteurised milk, 68°C, 30 min. | In pasteurised milk, 71.7°C, 20 sec., then 4°C | Milk only |
| SEQ ID NO: 8 | In sodium phosphate buffer, 4°C, 1 hour | In pasteurised milk, 4°C, 1 hour | In pasteurised milk, 37°C, 30 min | In pasteurised milk, 68°C, 30 min. | In pasteurised milk, 71.7°C, 20 sec., then 4°C | Milk only |

**TABLE 4**

| Peptide | 1 | 2 | 3 | 4 | 5 |
|---|---|---|---|---|---|
| SEQ ID NO: 7 | 4°C, 1 hour | 37°C, 30 min. | 56°C, 30 min | 70°C, 15 min. | Milk only |
| SEQ ID NO: 8 | 4°C, 1 hour | 37°C, 30 min. | 56°C, 30 min | 70°C, 15 min. | Milk only |

Peptides having or comprising sequences set forth in any one of SEQ ID Nos: 9-83 hereof are tested similarly to demonstrate their heat stability in phosphate buffer, milk and milk products.

A peptide comprising a sequence set forth in SEQ ID NO: 7 is active in pasteurized milk, even following heat treatment (data not shown). However, the bioactivity of a peptide comprising a sequence set forth in SEQ ID NO: 8 is reduced or inhibited in pasteurized milk, as expected from the results described in Example 3. However, both peptides AGG01 and AGG02 retain their antimicrobial bioactivity in phosphate buffer following heat treatment, indicating that these peptides are heat resistant.

### EXAMPLE 5

### Expression of antimicrobial peptides in mammalian cells

This example demonstrates expression and correct processing of antimicrobial peptide expressed with a prepro leader sequence in mammalian cells, and resistance of the processed peptides to proteolysis by milk proteases.

### Expression vectors

To confirm that the antimicrobial peptides of the invention can be expressed in mammalian cells, nucleic acids encoding the antimicrobial peptides AGG01 (SEQ ID NO: 7) and AGG02 (SEQ ID NO: 8) peptides were cloned into the vector pCMV-SPORT6 (Invitrogen) in operable connection with the CMV promoter for expression in COS cells and CHO cells.

In one example, vectors designated pCMV-SPORT6-AGG01 and pCMV-SPORT6-AGG02 were produced by cloning nucleic acids encoding the antimicrobial peptides in-frame to upstream sequences encoding *Macropus eugenii* cathelicidin prepro sequences. The peptides expressed from pCMV-SPORT6-AGG01 and pCMV-SPORT6-AGG02 comprised the amino acid sequences set forth in SEQ ID Nos: 104 and 105.

In another example, vectors designated pCMV-SPORT6-mAGG01 and pCMV-SPORT6-mAGG02 were produced by cloning nucleic acids encoding the antimicrobial peptides in-frame to upstream sequences encoding a translation start site and a downstream translation termination signal. Peptides expressed from these vectors comprised SEQ ID No: 7 or 8.

In another example, hexahistidine-encoding sequence was placed in-frame downstream of the antimicrobial peptide and upstream of the translation termination signals in vectors designated pCMV-SPORT6-mAGG01 and pCMV-SPORT6-mAGG02, to produce the vectors designated pCMV-SPORT6-mAGG01-6xHis and pCMV-SPORT6-mAGG02-6xHis. Peptides expressed from these vectors were fusion proteins comprising SEQ ID No: 7 or 8 with C-terminal hexahistidine tags.

Vectors were transfected into COS cells and CHO cells according to standard procedures.

### Western blotting

Western blotting of cell lysates was performed according to standard procedures to confirm expression of peptides in mammalian cells. Briefly, cell extracts and culture supernatants were obtained from CHO cells transfected with the vectors pCMV-SPORT6-AGG01 and pCMV-SPORT6-AGG02, resolved in 18% (w/v) SDS/polyacrylamide electrophoresis gels, transferred by electroblotting onto nitrocellulose membrane and either stained in Ponceau S or probed with polyclonal antisera. In a further example, cell extracts were incubated in bovine milk for 2hrs prior to their preparation for electrophoresis, to determine stability in milk. Control samples comprises non-transfected CHO cell extracts and supernatants. Membranes were developed using polyclonal rabbit anti-mAGG01 serum that had been produced according to standard procedures, by immunizing rabbits with synthetic peptide (i.e., SEQ ID NO: 7). The antiserum against AGG01 (SEQ ID NO: 7) was used at a dilution of 1:4000 (v/v).

Membranes are also developed using polyclonal rabbit anti-AGG02 serum that had been produced according to standard procedures, by immunizing rabbits with synthetic peptide (i.e., SEQ ID NO: 8). The antiserum against AGG02 (SEQ ID NO: 8) is used at a dilution of 1:500 (v/v). The procedures herein are also applied to demonstrate expression of other antimicrobial peptides of the invention e.g., exemplified by SEQ ID Nos: 9-58.

In one example, expression of correctly-processed antimicrobial peptide AGG01 (SEQ ID NO: 7) was confirmed in CHO cells, by the presence of a peptide of the same size as the synthetic peptide in lysates of cells transfected with the vector pCMV-SPORT6-AGG01 and to a lesser extent in culture media, as determined in Ponceau S-stained gels and following development with anti-AGG01 serum. The peptide was also present following incubation with bovine milk for 2 hr, extending other data herein showing that the synthetic AGG01 peptide retained activity in milk (Example 3). These data suggest that the AGG01 peptide (SEQ ID NO: 7) is not processed by milk proteases and would be suitable for expressing in mammary glands during lactation or as an additive to milk and milk products. The prepro sequence and AGG01 precursor polypeptide expected to be expressed from the vector pCMV-SPORT6-AGG01were not detectable using Ponceau S staining or anti-AGG01 serum, suggesting that the fusion polypeptide is process rapidly to a mature form by CHO cells and that the prepro sequence may be rapidly degraded.

In a further example, the CHO cell lysates and/or supernatants are also tested for their ability to inhibit growth of bacteria e.g., *E. coli* according to procedures described herein, to demonstrate that they retain antimicrobial activity following synthesis and processing by mammalian cells. Further purification of the peptides may be employed. For example, the peptides may be expressed as fusions with an affinity tag e.g., FLAG and isolated e.g., by nickel-NTA purification. Testing of peptides for their antimicrobial activities may be by means of performing a radial diffusion assay, e.g., as described in Example 1 for antimicrobial activity. For example, peptides are tested in a radial diffusion assay to identify those having antimicrobial activity against one or more bacteria that cause(s) mastitis, e.g., *S. uberis.* Alternatively, or in addition, heat stability of expressed and processed peptides is determined essentially as described in Example 4.

### EXAMPLE 6

### Antibacterial activity of peptides against agents of mastitis in vivo

This example discloses expression vectors and methods for demonstrating efficacy of one or more peptides of the invention against one or more agents of mastitis in mammary epithelial cells in which they are expressed and, where applicable, correctly processed.

The procedures herein are applied to demonstrate expression of any antimicrobial peptide of the invention e.g., exemplified by SEQ ID Nos: 7-58 without undue experimentation.

### Expression vectors

To confirm that the antimicrobial peptides of the invention can be expressed in mammary epithelial cells, and correctly processed by such cells to their bioactive antimicrobial form, nucleic acids encoding the antimicrobial peptides AGG01 (SEQ ID NO: 7) and AGG02 (SEQ ID NO: 8) were cloned into an expression vector e.g., pVEX, in operable connection with a bovine beta-casein promoter e.g., comprising a sequence set forth in SEQ ID NO: 92 or functional fragment thereof.

In one example, the antimicrobial peptide-encoding nucleic acids were produced by cloning nucleic acids encoding the antimicrobial peptides in-frame to upstream sequences encoding *Macropus eugenii* cathelicidin prepro sequences, such that the expressed peptides comprised the amino acid sequences set forth in SEQ ID NO: 104 (AGG01) and SEQ ID NO: 105 (AGG02). In this example, the AGG01 and AGG02 peptides are expressed under control of the beta-casein promoter and processed in mammary epithelial cells by virtue of cleavage of the prepro sequence from the cathelicidin proteins by endogenous mammary epithelial cell proteases.

In another example, the expression constructs of the preceding paragraph are modified to comprise nucleic acid encoding an alpha-lactalbumin signal peptide (e.g., SEQ ID NO: 101). The sequence encoding the signal peptide was placed upstream and in-frame with sequence encoding SEQ ID No: 104 or SEQ ID NO: 105. In this example, the AGG01 and AGG02 peptides are expected to be expressed as secretory proteins under control of the beta-casein promoter and signal peptide activities, and then processed in by virtue of cleavage of the prepro sequence from the cathelicidin proteins by endogenous mammary epithelial cell proteases.

In another example, the expression construct of the preceding paragraphs are further modified to comprise nucleic acid encoding a recognition sequence for enterokinase (e.g., SEQ ID NO: 112). In one example, the sequence encoding the enterokinase cleavage site is positioned in-frame between the sequence encoding the prepro sequence of *M. eugenii* cathelicidin and the sequence encoding AGG01 peptide (SEQ ID NO: 7) or AGG02 (SEQ ID NO: 8), to ensure correct processing in mammary epithelial cells. In another example, the sequence encoding the enterokinase cleavage site is positioned in-frame between the sequence encoding the alpha-lactalbumin signal sequence and the sequence encoding the prepro sequence of *M. eugenii* cathelicidin, to ensure removal of the signal peptide. In this example, the AGG01 and AGG02 peptides are in mammary epithelial cells in secretable or non-secretable form, optionally isolated, and then processed by enterokinase. Protease recognition sites that are known to be endogenous to mammary epithelial cells may promote correct processing *in vivo.* Protease recognition sites that are non-endogenous to mammary epithelial cells require processing *in vitro.*

For construction of pVEX-based expression constructs, the 554bp early SV40 promoter of pVEX is removed by digestion using the enzymes *Stu*I and *Nde*I*,* blunt-ended using Klenow fragment of DNA *Pol*I*,* and re-circularized by self-ligation. A fragment of the β-casein promoter (SEQ ID NO: 92) is obtained by amplification of a 1.3 kb fragment there from using polymerase chain reaction (PCR) with the following primers:
TCTACTCGAGGATCATCTATCTGTCCCAAAG (SEQ ID NO: 129); and
CTAGGATCCAATGATCTGATTTTGTGG (SEQ ID NO: 130).

The amplified fragment comprises 1230 bp of the canonical promoter plus 49 bp of the first non coding exon of the β-casein gene. The amplified β-casein promoter fragment is blunt-ended using Klenow enzyme and inserted into pVEX that has been digested using *Bam*HI and end-filled sing Klenow enzyme. Recombinant clones are selected and nucleic acid encoding the antimicrobial peptide, optionally with in-frame upstream sequences as described in the preceding example (e.g., sequence encoding alpha-lactalbumin signal sequence and/or sequence encoding *M. eugenii* cathelicidin prepro sequence and/or sequence encoding a protease recognition sequence (SEQ ID NO: 112 or any one of a plasmin, MT-MSP1, furin or urokinase recognition sequence as appropriate) is inserted into a unique HindIII site located downstream of the β-casein promoter fragment.

### Bacterial strains and culture conditions

*Streptococcus uberis* (UT888), *Streptococcus dysgalactiae* subsp. dysgalactiae (UT19), and *Staphylococcus aureus* (UT23) isolated from dairy cows with mastitis are used. A non-pathogenic strain of *Escherichia coli* (DH5α; Gibco, Grand Island, NY, USA) is used as a negative control. Gram-positive bacteria are cultivated in Todd-Hewitt broth (THB; Becton Dickinson and Co., Franklin Lakes, NJ, USA) and *E. coli* is cultivated in Luria broth (LB). All bacteria are subcultured and grown on blood agar. For internalization assays, bacterial lawns are harvested, washed and resuspended at approximately 10⁷ colony-forming units per millilitre (cfu/mL) in Dulbecco's Modified Eagle's medium (DMEM, Gibco). The concentration of bacteria and strain purity are determined by standard plate count techniques.

### Fluorescent labeling of bacteria

Procedures for immunofluorescence staining and confocal laser microscopy (CLM) are performed essentially as described in Barker et al., Infection and Immunity 65: 1497-1504, 1997. Briefly, mastitis pathogens and *E. coli* DH5α kept are thawed at room temperature, plated onto blood agar, and incubated overnight at 37 °C in 5% CO₂:95% air (v/v). After incubation, the bacterial lawn is harvested with 0.5 mL of THB or LB, seeded into 9.5 mL of the same media (THB or LB), and incubated for 1 h at 37 °C with orbital shaking at 150 r.p.m. After incubation, bacterial suspensions are washed three times by centrifugation (20 800 g for 3 min at 4 °C) with phosphate-buffered saline (PBS) (pH 7.4) and fluorescently labeled (LIVE/DEAD BacLight Bacterial Viability Kits L-7007; Molecular Probes, Eugene, OR, USA) following manufacturer's instructions.

### Mammary epithelial cell culture

A bovine mammary epithelial cell line (MAC-T) (Huynh et al., Exp. Cell. Res., 197: 191-199, 1991) is used for infection studies. Cells are transfected with a nucleic acid encoding an antibacterial peptide of the invention e.g., any one of SEQ ID Nos: 7-58. Transfected MAC-T cells are grown in 24-well cell culture plates (Corning Inc., Corning, NY, USA) or 8-well slides (Lab-Tek II; Nalge Nunc International Corp., Rochester, NY, USA) at 37 °C in 5% CO2:95% air (v/v) using cell growth media described previously (Almeida et al., J. Vet. Med., 45: 385-392, 1996). Mammary epithelial cell viability is monitored by trypan blue dye exclusion.

### Internalization and intracellular bactericidal assay

Fluorescent-labeled or untreated mastitis pathogens or *E. coli* DH5α are co-cultured with MAC-T cells in DMEM. Following incubation [2 h at 37 °C in 5% CO₂:95% air (v/v)], monolayers are washed three times with PBS (pH 7.4).

As a control, co-cultures are incubated with non-transfected cells or non-transfected cells in DMEM containing gentamicin (100 µg/mL; Sigma Chemical Co., St Louis, MO, USA) and penicillin (100 IU/mL, Sigma). Penicillin and gentamicin do not penetrate into mammary epithelial cells and are used in the standard internalization protocol to eliminate bacteria that are outside of mammary epithelial cells and allow discrimination between intracellular and extracellular micro-organisms. After removing media containing antibiotics, MAC-T cell monolayers are washed and lysed. MAC-T cell lysates are 10-fold serially diluted, plated in triplicate on blood agar, and incubated overnight. Intracellular survival is evaluated by determining the number of cfu/mL in MAC-T cell lysates. For fluorescent assays, after incubation bacteria, slides are washed with PBS (pH 7.4) and mounted. Cover slips are then sealed onto slides with nail polish and kept at 4 °C until visualization by CLM (Leica TCS SP2; Leica Microsystems, Heidelberg, Germany). Internalization and intracellular survival assays are performed in triplicate three times.

### Image analysis

Red/green images are collected and overlaid using Leica Lite software (Leica Microsystems, Heidelberg, Germany).

### Results

Peptides capable of killing bacteria or preventing growth of bacteria within a mammary cell, e.g., as determined by reducing c.f.u from lysed MAC-T cells compared to non-transfected cells and/or that reduce the number of live bacteria and/or increase the number of dead bacteria within MAC-T cells are selected as peptides suitable for treatment of mastitis.

### EXAMPLE 7

### Production of transgenic cows

This example discloses expression vectors and methods for producing transgenic cattle, especially cows, expressing the antimicrobial peptide(s) of the invention, and the efficacy of the expressed peptides in protecting animals against mastitis.

It is to be understood that the procedures herein are applied to demonstrate expression and efficacy of any antimicrobial peptide of the invention e.g., exemplified by SEQ ID Nos: 7-58 without undue experimentation.

### Expression vectors

Any of the expression vectors described in the preceding example are employed herein, especially those in a pVEX backbone or other at-recognized vector suitable for transfection of somatic cells and their subsequent fusion with enucleated oocytes to produce transgenic cattle.

### a) Preparation of pVEβcashAMP

An expression construct pVEβcashAMP is produced essentially in accordance with the description in Example 6, employing a pVEX backbone. The vector is modified to express gene constructs encoding the antimicrobial fusion proteins as described in Example 6.

### Transfection of Somatic Cells

Expression vectors based on pVEβcashAMP are used for transfecting a primary culture of somatic cells, using calcium phosphate or liposome method. Fetal calf fibroblasts are generally transfected. Transfected cells are then selected using geneticin, and following a period of 2 to 8 weeks, the cells that are resistant to geneticin are isolated for use as donor cells to obtain transgenic clones. Transfected selected cells are analyzed by PCR to confirm presence of the expression construct to ensure the appropriate nuclei are transferred to generate transgenic embryos.

### Oocyte Enucleation and Metaphase Nuclear Transfer in Mature Enucleated Oocytes

Bovine oocytes are aspirated from slaughterhouse ovaries and matured in TCM-199+5% FCS at 39°C for 24 hours. The maturation medium is equilibrated with CO₂ for at least 2 hours prior to use. Mature oocytes are denuded by vortexing for 2 minutes in warm TL-HEPES with 1 mg/ml bovine testis hyaluronidase.

### Nuclear Transfer

Oocytes are treated with roscovitine to suspend meiosis. Oocytes are mechanically enucleated using a Narishige hydraulic micromanipulators and Nikon Diaphot microscopy. Enucleation is performed with a 20 µm beveled and sharpened pipette. Oocytes are stained with 5 µg/ml bisbenzimidine (Hoechst 33342) dye for 20 minutes. Metaphases are enucleated by visualization of the stained chromosomes under ultraviolet light. Metaphase chromosomes are assessed after aspiration inside the pipette. A transgenic somatic cell produced as described *supra* is then transferred into the perivitelline space and tightly opposed to the enucleated oocyte.

### Fusion

A transgenic somatic cell and an enucleated oocyte are manually aligned in the fusion chamber so that the membranes to be fused are parallel to the electrodes. Fusion is performed using one electrical pulse of 180 volts/cm for 15 µs (BTX Electro Cell Manipulator 200) and monitored with a BTX Optimizer-Graphic Pulse Analyzer. The chamber for pulsing embryos consists of two 0.5 mm stainless steel wire electrodes mounted 0.5 mm apart on glass microscope slide. Presumptive zygotes are monitored for fusion, lysis, and fragmentation.

### Transfer to recipient cows

Zygotes are evaluated at 48 hours after fertilization for cleavage and after 7 to 9 days for development to morulae or blastocysts. Generally, two blastocysts are transferred non-surgically per recipient cow, and pregnancies determined at 30-35 days by ultrasonography.

The implanted cows are allowed to normally pass the pregnancy up to a natural delivery. Newborn calves are fed with Ig rich colostrum during the first 48 hours, and then synthetic, later natural foods are used.

Following sufficient time for transgenic animals to reach maturity, animals are bred to produce female transgenic cows.

### EXAMPLE 8

### Production of transgenic goats

This example discloses expression vectors and methods for producing transgenic goats expressing the antimicrobial peptide(s) of the invention, and the efficacy of the expressed peptides in protecting animals against mastitis.

It is to be understood that the procedures herein are applied to demonstrate expression and efficacy of any antimicrobial peptide of the invention e.g., exemplified by SEQ ID Nos: 7-58 without undue experimentation.

### Expression constructs

A 2.0-kb promoter fragment from bovine alpha-lactalbumin (α-LA) gene is generated by PCR amplification using a genomic DNA from high milk-producing Holstein cow as the template. This PCR product containing entire α-LA promoter and 19-aa leader sequence are then subsequently inserted into the pCR3 vector (Invitrogen, San Diego, Calif.). Nucleic acid encoding an antimicrobial peptide (any one of SEQ ID Nos: 7-58 or a fusion construct as described in Example 6 or 7 lacking the introduced alpha-lactalbumin signal peptide-encoding sequence) is cloned into the pCR3 vector in-frame with the leader sequence to produce the vector pCR3-α-LA-AMP.

### Transgenic Animal Production

For transgenic goat production, pronuclear stage embryos are flushed from a donor goat's oviduct at the one and half day after insemination. The collected embryos are then rinsed with sterile phosphate buffered saline and placed under a phase contrast microscope for microinjection of the expression construct. An expression construct is then microinjected into the male pronucleus of the embryo. After a transient *in vitro* culture, healthy microinjected embryos are then transferred into recipient oviducts for development into a transgenic goat.

Following birth, ear punctures are taken from newborn goats and genomic DNA isolated there from. PCR is then performed suing the genomic DNA as a template to determine whether or not each newborn goat comprises the expression construct. Goats comprising the expression construct are then bred with wild-type goats to produce female goats, which are also screened to select those comprising the expression construct.

### EXAMPLE 9

### Preparation of a monoclonal antibody that recognizes an antimicrobial peptide

This example discloses a procedure for preparing monoclonal antibodies that specifically recognize antimicrobial peptides of the invention for diagnostic purposes. The procedure is applied to any peptide disclosed herein as SEQ ID Nos: 7-83.

A monoclonal antibody that specifically binds to an antimicrobial peptide comprising a sequence set forth in any one of SEQ ID Nos: 7-83 is produced using methods known in the art. Briefly, the peptide antigen is synthesized essentially using the methods described in Bodanszky, M. (1984) Principles of Peptide Synthesis, Springer-Verlag, Heidelberg and Bodanszky, M. & Bodanszky, A. (1984) The Practice of Peptide Synthesis, Springer-Verlag, Heidelberg. Peptides are purified using HPLC and purity assessed by amino acid analysis.

Female BalB/c mice are immunized with a purified form of the peptide. Initially mice are sensitized by intraperitoneal injection of Hunter's Titermax adjuvant (CytRx Corp., Norcross, GA,). Three boosts of the peptide are administered at 2, 5.5 and 6.5 months post initial sensitization. The first of these boosts is a subcutaneous injection while the remaining are administered by intraperitoneal injection. The final boost is administered 3 days prior to fusion.

The splenocytes of one of the immunized BALB/c mice is fused to X63-Ag8.653 mouse myeloma cells using PEG 1500. Following exposure to the PEG 1500 cells are incubated at 37°C for 1 hour in heat inactivated fetal bovine serum. Fused cells are then transferred to RPMI 1640 medium and incubated overnight at 37°C with 10% CO₂. The following day, cells are plated using RPMI 1640 media that has been supplemented with macrophage culture supernatants.

Two weeks after fusion, hybridoma cells are screened for antibody production by solid phase ELISA assay. Standard microtitre plates are coated with the peptide antigen in a carbonate based buffer. Plates are then blocked with BSA, washed and then the test samples (i.e. supernatant from the fused cells) is added, in addition to control samples, (i.e. supernatant from an unfused cell). Antigen-antibody binding is detected by incubating the plates with goat-anti-mouse HRP conjugate (Jackson ImmunoResearch Laboratories) and ABTS peroxidase substrate system (Vector Laboratories, Burlingame, Ca 94010, USA). Absorbance is read on an automatic plate reader at a wavelength of 405 nm.

Any colonies that are identified as positive by these screens continue to be grown and screened for several further weeks. Stable colonies are then isolated and stored at -80°C.

Positive stable hybridomas are then cloned by growing in culture for a short period of time and diluting the cells to a final concentration of 0.1 cells/well of a 96 well tissue culture plate. These clones are then screened using the previously described assay. This procedure is then repeated in order to ensure the purity of the clone.

Four different dilutions, 5 cells/well, 2 cells/well, 1 cell/well, 0.5 cells/well of the primary clone are prepared in 96-well microtiter plates to start the secondary cloning. Cells are diluted in IMDM tissue culture media containing the following additives: 20% fetal bovine serum (FBS), 2 mM L-glutamine, 100 units/ml of penicillin, 100 µg/ml of streptomycin, 1% GMS-S, 0.075% NaHCO3. To determine clones that secrete anti-antimicrobial peptide antibody, supernatants from individual wells of the 0.2 cells/well microtiter plate are withdrawn after two weeks of growth and tested for the presence of antibody by ELISA assay as described above.

All positive clones are then adapted and expanded in RPMI media containing the following additives: 10% FBS, 2 mM L-glutamine, 100 units/ml of penicillin, 100 µg/ml of streptomycin, 1% GMS-S, 0.075% NaHCO3, and 0.013 mg/ml of oxaloacetic acid. A specific antibody is purified by Protein A affinity chromatography from the supernatant of cell culture.

The titers of the antibodies produced using this method are determined using the Easy Titer kit available from Pierce (Rockford, Il, USA). This kit utilizes beads that specifically bind mouse antibodies, and following binding of such an antibody these beads aggregate and no longer absorb light to the same degree as non-associated beads. Accordingly, the amount of an antibody in the supernatant of a hybridoma is assessed by comparing the OD measurement obtained from this sample to the amount detected in a standard, such as for example mouse IgG.

The specificity of the monoclonal antibody is then determined by Western blotting according to standard procedures.

### EXAMPLE 10

### Immunoblot analysis of milk from transgenic cows and goats

This example discloses diagnostic procedures for determining expression of antimicrobial peptides in transgenic animals e.g., cattle and goats such as for the selection of breeding stocks expressing the antimicrobial peptides at particular levels.

Milk is collected from lactating females produced in Examples 7 and 8 essentially as described in Simons et al., Nature 328: 530-532, 1987) and resolved using SDS-polyacrylamide gel electrophoresis (SDS-PAGE) essentially as described in Cheng et al., Human Gene Therapy 9: 1995-2003, 1998). Proteins are electro-transferred from the gel to a PVDF membrane. The blots are then probed with the monoclonal antibody described in Example 7 and washed with phosphate-buffered saline containing 0.1% Tween-20 (PBS-T). Blots are then incubated with an anti-mouse secondary antibody conjugate to horseradish peroxidase (HRP). Blots are then developed with the chemiluminescent ECL™ detection system (Amersham, UK) and exposure to x-ray film. Band intensities were compared by densitometry.

Transgenic goats and cows secreting an antimicrobial peptide into their milk are then selected.

### EXAMPLE 11

### Antimicrobial activity of milk from transgenic animals

This example discloses diagnostic procedures for determining activity of antimicrobial peptides expressed in milk of in transgenic animals e.g., cattle and goats such as for the selection of breeding stocks.

### Milk collection

Milk is either collected from goats or cows described in Example 7 or 8 using an automated sampling device during the normal process of milking or collected by hand in an aseptic manner. All samples are centrifuged at 800g for 15 min at 4 °C and the supernatant collected and tested immediately or frozen at -20 °C until use.

### Antimicrobial assays

### 1. Radial diffusion assay

Spot-on-lawn assays are then performed to compare the antimicrobial activity of milk from transgenic cows or goats with that of synthetic antimicrobial peptides. Samples are assayed in triplicate and a dilution series of synthetic peptide is included in the assay. *S. uberis* or *S*. *aureus* or *E. coli* in log-phase growth containing are added to a Petri dish containing appropriate growth medium. Dishes are air dried for 30-60 min before 10µl of a dilutions series (from undiluted to 1:32) of test milk samples or synthetic peptide standards, both diluted in skim milk (Difco Laboratories), are added to the dishes. After an additional 30-min drying time, dishes are inverted and incubated at 37 °C in air overnight.

Optical density of cleared zones is determined with a ChemiDoc XRS with Quantity One software (Bio-Rad Laboratories). The antimicrobial activity of milk from transgenic cows or goats is quantified by comparing optical densities of cleared zones with optical densities produced by synthetic peptides in a dilution series. In this manner, a level of bioactivity of peptide expressed in cow's milk relative to synthetic peptide is determined.

### 2. Mastitis challenge assays

Forty-eight hours before initiating bacterial challenges, health of the animals as described in Example 7 or 8 is assessed by differential leukocyte and milk somatic cell counts to verify that they are within normal ranges. Animals that appear not to be suffering from mastitis are then infected with a mastitis causing bacterium by infusing 2 ml *S*. *uberis* and/or *S*. *aureus* and/or *E. coli* via the streak canal after morning milking. One animal is infused with 2 ml of sterile PBS. The cows and goats are closely monitored, initially at 6-h intervals, and then every 12 h for 48 h. Body temperature, blood and milk samples are taken at 12-h intervals or more frequently throughout the study. Milk samples (20 1) are plated on suitable growth medium depending on the bacteria infused into the goat or cow and incubated at 37 °C for 18 to 24 h. Once an infection was confirmed by the presence of viable *S*. *uberis* and/or *S*. *aureus* and/or *E. coli* in two consecutive milk samples, the animal is treated with antibiotics for five consecutive milkings. Milk somatic cells and bacteria are monitored weekly thereafter to assure that infections are eliminated.

For enumeration of somatic cells, milk samples were heated to 60 °C for 15 min, cooled to 40 °C and cells counted on a Fossomatic 90 (Foss Electric). The device is calibrated quarterly with bovine milk somatic cell standards (Dairy Quality Control Institute Services). Samples are counted in duplicate. Cows and goats that do not show significant increases in somatic cell counts following infusion of an infectious bacteria are considered resistant to that bacteria and mastitis.

To determine whether or not transgenic animals are resistant to mastitis, each animal is infused a plurality of times with an infectious bacteria and the number of infections resulting determined. Animals that do not become infected or rarely become infected are considered resistant to mastitis causing bacteria.

### EXAMPLE 12

### Synergistic effect of antimicrobial peptides and antibiotics

This example discloses synergism between synthetic antimicrobial peptides of the invention and known antibiotics. The procedure is applied to any peptide disclosed herein as SEQ ID Nos: 7-83.

### Methods

To determine whether or not antimicrobial peptides as described herein act in a synergistic manner with antibiotic compounds, a microbroth dilution method was performed against *E. coli DH5α* performed according to the guidelines of the National Committee for Clinical Laboratory Standards.

### Results

As shown in Table 5, a peptide comprising a sequence set forth in SEQ ID NO: 7 or 8 reduces the MIC of tetracycline or chloramphenicol indicating that these peptides act synergistically with tetracycline or chloramphenicol.

**TABLE 5**

| **MIC (µg/ml)** | | | |
|---|---|---|---|
| **Combination** | **-** | **SEQ ID NO: 7** | **SEQ ID NO: 8** |
| Ampicillin | 2 | 2 | 4 |
| Chloramphenicol | 4 | 1 | 2 |
| Tetracycline | 2 | 0.25 | 0.5 |

### EXAMPLE 13

### Efficacy of antimicrobial peptides in the treatment of Bovine Respiratory Disease and/or Swine Respiratory Disease

This example discloses the efficacy of antimicrobial peptide of the invention against known agents of BRD and/or SRD, as determined by the MIC value. The procedure is applied to any peptide disclosed herein as SEQ ID Nos: 7-83.

**TABLE 6**

| Peptide activity against BRD/SRD isolates | | |
|---|---|---|
| **Microorganism [No. strains tested]** | **MIC (µg/ml)** | |
| | **SEQ ID NO: 7** | **SEQ ID NO: 8** |
| *E. coli* [9] | 1-8 | 8-32 |
| *P. haemolytica* [4] | 1-16 | 4-64 |
| *P. multicoda* [4] | 16 | 8-32 |
| *B. bronchiseptica* [2] | 1-2 | 32 |
| *H. somnus* [3] | 16 | 32 |
| *A. pleuropneumoniae* [3] | 8 | 16 |
| *S. suis* [3] | 16-32 | 4-8 |
| *S. choleraesuis* [3] | 4-8 | >64 |

The data presented in Table 6 indicate utility of peptide AGG01 in particular against one or more agents of BRD/SRD, with especially strong activity against isolates of *E. coli, B. bronchiseptica* and *S. choleraesuis* and possibly also against *P. haemolytica. ,* Intermediate activity was observed against *A. pleuropneumoniae* and *S*. *choleraesuis,* and weaker activity against *S. suis* and *H. somnus.*

### EXAMPLE 14

### Efficacy of antimicrobial peptides in the treatment of otitis externa

This example discloses the efficacy of antimicrobial peptide of the invention against known agents of otitis externa e.g., in canines, as determined by the MIC value. The procedure is applied to any peptide disclosed herein as SEQ ID Nos: 7-83.

**TABLE 7**

| Peptide activity against otitis externa isolates | | |
|---|---|---|
| **Microorganism [No. strains tested]** | **MIC (µg/ml)** | |
| | **SEQ ID NO: 7** | **SEQ ID NO: 8** |
| *S. aureus* [1] | **2** | **4** |
| *S. schleiferi* [5] | 2-4 | 2-4 |
| *S. epidermis* [1] | 1 | 2 |
| *S. pseudointermedin* [3] | 1-2 | 1 |
| *Pseudomonas aeruginosa* [5] | 2-4 | 8-32 |

The data presented in Table 7 indicate utility of peptides AGG01 (SEQ ID NO: 7) and AGG02 (SEQ ID NO: 8) against one or more agents of otitis externa, with especially strong activity against isolates of *S*. *aureus, S. schleiferi, S. epidermis* and *S*. *pseudointermedin.* Strong antimicrobial activity was observed using SEQ ID NO: 7 against *Pseudomonas aeruginosa,* with slightly weaker activity against this pathogen using SEQ ID NO: 8.

### SEQUENCE LISTING

<110> Agriculture Victoria Services Pty Limited
<120> Method of treatment using antimicrobial composition
<130> 141882/MRO
<150> AU 2008901249
   <151> 2008-03-13
<160> 128
<170> Patent In version 3.5
<210> 1
   <211> 15
   <212> PRT
   <213> artificial sequence
<220>
   <223> Consensus sequence of antimicrobial peptide
<220>
   <221> VARIANT
   <222> (8)..(8)
   <223> Xaa is a basic amino acid, preferably K or R
<220>
   <221> VARIANT
   <222> (9) .. (9)
   <223> Xaa is N or K
<400> 1
   Thr Lys Phe Arg Asn Ser Ile Xaa Xaa Arg Leu Lys Asn Phe Asn
   1               5                   10                  15
<210> 2
   <211> 5
   <212> PRT
   <213> ARTIFICIAL
<220>
   <223> Consensus sequence of antimicrobial peptide
<220>
   <221> misc_feature
   <222> (4) .. (4)
   <223> Xaa can be any naturally occurring amino acid
<400> 2
   Lys Arg Gly Xaa Gly
   1               5
<210> 3
   <211> 8
   <212> PRT
   <213> artificial
<220>
   <223> Consensue sequence of antimicrobial peptide
<220>
   <221> VARIANT
   <222> (6) .. (6)
   <223> Xaa is a non-polar amino acid, preferably L or F
<400> 3
   Met Val Lys Arg Gly Xaa Gly Glu
   1               5
<210> 4
   <211> 10
   <212> PRT
   <213> artificial
<220>
   <223> Consensus sequence for antimicrobial peptide
<220>
   <221> VARIANT
   <222> (2) .. (2)
   <223> Xaa is a basic amino acid, preferably K or R
<220>
   <221> VARIANT
   <222> (3)..(3)
   <223> Xaa is K or N
<220>
   <221> VARIANT
   <222> (6)..(6)
   <223> Xaa is a basic amino acid, preferably K or R
<220>
   <221> VARIANT
   <222> (9) .. (9)
   <223> Xaa is K or N
<220>
   <221> VARIANT
   <222> (10)..(10)
   <223> Xaa is a non-polar amino acid, preferably F or L
<400> 4
   Ile Xaa Xaa Thr Leu Xaa Asn Phe Xaa Xaa
   1               5                   10
<210> 5
   <211> 15
   <212> PRT
   <213> artificial
<220>
   <223> Consensus sequence of antimicrobial peptide
<400> 5
   Ser Ser Arg Ser Ala Ala Gly Thr Ile Trp Glu Phe Glu Ala Phe
   1               5                   10                  15
<210> 6
   <211> 20
   <212> PRT
   <213> artificial
<220>
   <223> Consensus sequence of antimicrobial peptide
<220>
   <221> misc_feature
   <222> (2)..(2)
   <223> Xaa can be any naturally occurring amino acid
<400> 6
   Leu Xaa Leu Arg Gly Ser Ser Arg Ser Ala Ala Gly Thr Ile Trp Glu
   1               5                   10                  15
   Phe Glu Ala Phe
               20
<210> 7
   <211> 35
   <212> PRT
   <213> artificial
<220>
   <223> antimicrobial peptide AGG01
<400> 7
   Lys Arg Gly Phe Gly Lys Lys Leu Arg Lys Arg Leu Lys Lys Phe Arg
   1               5                   10                  15
   Asn Ser Ile Lys Lys Arg Leu Lys Asn Phe Asn Val Val Ile Pro Ile
               20                  25                  30
   Pro Leu Pro
           35
<210> 8
   <211> 35
   <212> PRT
   <213> artificial
<220>
   <223> antimicrobial peptide AGG02
<400> 8
   Lys Arg Gly Leu Trp Glu Ser Leu Lys Arg Lys Ala Thr Lys Leu Gly
   1               5                   10                  15
   Asp Asp Ile Arg Asn Thr Leu Arg Asn Phe Lys Ile Lys Phe Pro Val
               20                  25                  30
   Pro Arg Gln
           35
<210> 9
   <211> 32
   <212> PRT
   <213> artificial
<220>
   <223> antimicrobial peptide AGG03
<400> 9
   Arg Lys Lys Gly Ser Lys Arg His Lys Pro Gly Ser Tyr Ser Val Ile
   1               5                   10                  15
   Ala Leu Gly Lys Pro Gly Val Lys Lys Ser Pro Tyr Met Glu Ala Leu
               20                  25                  30
<210> 10
   <211> 38
   <212> PRT
   <213> artificial
<220>
   <223> antimicrobial peptide A12
<400> 10
   Met Val Lys Arg Gly Phe Gly Lys Arg Leu Arg Lys Arg Pro Lys Lys
   1               5                   10                  15
   Phe Arg Asn Ser Ile Lys Lys Arg Leu Lys Asn Phe Asn Val Val Phe
               20                  25                  30
   Pro Val Pro Arg Pro Gly
           35
<210> 11
   <211> 38
   <212> PRT
   <213> artificial
<220>
   <223> Antimicrobial peptide 5
<400> 11
   Met Val Lys Arg Gly Leu Gly Lys Asn Leu Lys Arg Arg Ala Thr Lys
   1 5 10 15
   Ile Gly Asn Gly Ile Lys Asn Thr Leu Arg Asn Phe Asn Val Val Phe
   20 25 30
   Pro Ile Pro Arg Pro Gly
   35
<210> 12
   <211> 38
   <212> PRT
   <213> artificial
<220>
   <223> Antimicrobial peptide 6
<400> 12
   Met Val Lys Arg Gly Leu Gly Glu Ser Leu Lys Lys Arg Val Thr Lys
   1 5 10 15
   Phe Gly Asp Ser Ile Arg Lys Thr Leu Arg Asn Phe Asn Ile Ile Ile
   20 25 30
   Pro Ile Pro Leu Pro Gly
   35
<210> 13
   <211> 24
   <212> PRT
   <213> artificial
<220>
   <223> Antimicrobial peptide 13
<400> 13
   Met Val Lys Arg Gly Phe Trp Lys Ser Leu Lys Arg Lys Leu Lys Lys
   1 5 10 15
   Phe Gly Met Ile Leu Gly Ile Asp
   20
<210> 14
   <211> 38
   <212> PRT
   <213> artificial
<220>
   <223> Antimicrobial peptide A4
<400> 14
   Met Val Lys Arg Gly Phe Gly Lys Asn Leu Arg Arg Arg Pro Thr Lys
   1               5                   10                  15
   Phe Arg Asn Ser Ile Arg Asn Arg Leu Lys Asn Phe Asn Ile Glu Phe
               20                  25                  30
   Pro Val Pro Arg Gln Gly
           35
<210> 15
   <211> 38
   <212> PRT
   <213> artificial
<220>
   <223> Antimicrobial peptide A5
<400> 15
   Met Val Lys Arg Gly Phe Trp Lys Lys Leu Lys Arg Lys Ala Lys Lys
   1               5                   10                  15
   Phe Arg Asn Ser Ile Arg Lys Thr Leu Lys Asn Phe Asn Ile Ile Ile
               20                  25                  30
   Pro Val Pro Leu Pro Gly
           35
<210> 16
   <211> 38
   <212> PRT
   <213> artificial
<220>
   <223> Antimicrobial peptide A6
<400> 16
   Met Val Lys Arg Gly Leu Gly Lys Lys Leu Lys Lys Lys Pro Lys Lys
   1               5                   10                  15
   Phe Arg Asn Ser Ile Lys Lys Arg Leu Lys Asn Phe Asn Ile Glu Ile
               20                  25                  30
   Pro Ile Pro Arg Gln Gly
           35
<210> 17
   <211> 38
   <212> PRT
   <213> artificial
<220>
   <223> Antimicrobial peptide A10
<400> 17
   Met Val Lys Arg Gly Phe Gly Lys Lys Leu Lys Lys Lys Ala Lys Lys
   1               5                   10                  15
   Phe Arg Asn Ser Ile Arg Lys Arg Leu Lys Asn Phe Asn Val Lys Ile
               20                  25                  30
   Pro Val Pro Leu Pro Gly
           35
<210> 18
   <211> 38
   <212> PRT
   <213> artificial
<220>
   <223> Antimicrobial peptide A11
<400> 18
   Met Val Lys Arg Gly Phe Gly Lys Lys Leu Arg Lys Arg Ala Thr Lys
   1               5                   10                  15
   Phe Arg Asn Ser Ile Arg Lys Arg Leu Lys Asn Phe Asn Ile Ile Phe
               20                  25                  30
   Pro Val Pro Arg Gln Gly
           35
<210> 19
   <211> 24
   <212> PRT
   <213> artificial
<220>
   <223> Antimicrobial peptide 10
<400> 19
   Met Val Lys Arg Gly Leu Gly Glu Arg Leu Arg Arg Lys Leu Thr Asn
   1               5                   10                  15
   Leu Gly Ile Val Leu Arg Arg His
               20
<210> 20
   <211> 24
   <212> PRT
   <213> artificial
<220>
   <223> Antimicrobial peptide 20
<400> 20
   Met Val Lys Arg Gly Phe Gly Glu Ser Leu Arg Arg Arg Val Thr Lys
   1               5                   10                  15
   Leu Arg Asp Asp Ile Lys Lys His
               20
<210> 21
   <211> 24
   <212> PRT
   <213> artificial
<220>
   <223> Antimicrobial peptide 27
<400> 21
   Met Val Lys Arg Gly Phe Gly Lys Arg Leu Arg Arg Lys Ala Thr Lys
   1               5                   10                  15
   Leu Gly Met Ile Leu Arg Arg His
               20
<210> 22
   <211> 24
   <212> PRT
   <213> artificial
<220>
   <223> Antimicrobial peptide 28
<400> 22
   Met Val Lys Arg Gly Leu Gly Glu Lys Leu Arg Arg Arg Pro Lys Lys
   1               5                   10                  15
   Phe Gly Ile Val Leu Gly Ile His
               20
<210> 23
   <211> 53
   <212> PRT
   <213> artificial
<220>
   <223> Antimicrobial peptide B1
<400> 23
   Met Val Leu Gly Asp Leu Leu Arg Arg Val Gly Gly Lys Phe Glu Glu
   1               5                   10                  15
   Phe Phe Gly Lys Ile Arg Glu Thr Ile Arg Thr Ile Arg Ile Cys Ser
               20                  25                  30
   His Leu Leu Leu Arg Gly Ser Ser Arg Ser Ala Ala Gly Thr Ile Trp
           35                  40                  45
   Glu Phe Glu Ala Phe
       50
<210> 24
   <211> 64
   <212> PRT
   <213> artificial
<220>
   <223> Antimicrobial peptide B6
<400> 24
   Met Val Gly Ile Val Ser Gly Glu Phe Arg Gly Ser Phe Glu Lys Cys
   1               5                   10                  15
   Val Gly Gly Ile Asp Lys Ser Leu Gly Ile Leu Arg Leu Ser Trp Asp
               20                  25                  30
   Leu Val Leu Arg Gly Ile Leu Arg Leu Ser Trp Asp Leu Val Leu Arg
           35                  40                  45
   Gly Ser Ser Arg Ser Ala Ala Gly Thr Ile Trp Glu Phe Glu Ala Phe
       50                  55                  60
<210> 25
   <211> 38
   <212> PRT
   <213> artificial
<220>
   <223> Antimicrobial peptide E3
<400> 25
   Met Val Leu Gly Gly Cys Leu Arg Lys Gly Gly Gly Arg Leu Val Gly
   1               5                   10                  15
   Met Phe Gly Arg Phe Arg Ala Thr Leu Lys Asn Leu Leu Asp Met Leu
               20                  25                  30
   Gly Arg Arg Ile Glu Gly
           35
<210> 26
   <211> 38
   <212> PRT
   <213> artificial
<220>
   <223> Antimicrobial peptide F4
<400> 26
   Met Val Leu Gly Gly Arg Ser Gly Lys Val Arg Glu Arg Phe Arg Arg
   1               5                   10                  15
   Ile Phe Glu Arg Tyr Glu Ala Lys Ile Arg Thr Phe Met Glu Ile Leu
               20                  25                  30
   Ala Ala Pro Ser Gln Asp
   35
<210> 27
   <211> 38
   <212> PRT
   <213> artificial
<220>
   <223> Antimicrobial peptide F6
<400> 27
   Met Val Leu Arg Gly Phe Leu Lys Arg Phe Gly Gly Lys Ile Leu Arg
   1               5                   10                  15
   Asn Arg Gly Arg Asp Gly Ala Thr Leu Lys Asp Phe Leu Lys Met Ile
               20                  25                  30
   Ala Thr Arg Ile Pro His
           35
<210> 28
   <211> 29
   <212> PRT
   <213> artificial
<220>
   <223> Antimicrobial peptide F7
<400> 28
   Met Val Leu Gly Gly Phe Ser Arg Cys Arg Trp Leu Glu Glu Lys Leu
   1               5                   10                  15
   Arg Asp Phe Met Lys Asn Leu Val Val Arg Ile Ala Gly
               20                  25
<210> 29
   <211> 38
   <212> PRT
   <213> artificial
<220>
   <223> Antimicrobial peptide F12
<400> 29
   Met Val Ile Gly Asp Arg Phe Lys Lys Phe Arg Glu Arg Leu Val Glu
   1 5 10 15
   Leu Arg Gly Arg Asn Gly Ala Arg Leu Arg Pro Leu Met Asn Ile Met
   20 25 30
   Gly Gly Arg Ser Ala Gly
   35
<210> 30
   <211> 47
   <212> PRT
   <213> artificial
<220>
   <223> Antimicrobial peptide G9
<400> 30
   Met Val Leu Gly Gly Phe Leu Arg Asn Val Arg Trp Glu Ile Trp Lys
   1 5 10 15
   Lys Gly Leu Ser Leu Phe Cys Gln Ile Ser Tyr Phe Val Val Arg Ser
   20 25 30
   Ser Ser Arg Ser Ala Ala Gly Thr Ile Trp Glu Phe Glu Ala Phe
   35 40 45
<210> 31
   <211> 38
   <212> PRT
   <213> artificial
<220>
   <223> Antimicrobial peptide G10
<400> 31
   Met Val Ile Arg Gly Leu Leu Lys Arg Gly Lys Gly Arg Ile Leu Gly
   1 5 10 15
   Leu Leu Gly Arg Asn Gly Lys Arg Leu Arg Ala Ile Met Asp Met Phe
   20 25 30
   Arg Arg Arg Ser Gln Gln
   35
<210> 32
   <211> 32
   <212> PRT
   <213> artificial
<220>
   <223> Antimicrobial peptide H6
<400> 32
   Met Val Ile Gly Asp Cys Leu Arg Arg Val Arg Gly Ser Leu Glu Asn
   1               5                   10                  15
   Thr Val Arg Asp Leu Asn Gln Gly Leu Gly Ile Leu Arg Leu Arg Trp
               20                  25                  30
<210> 33
   <211> 35
   <212> PRT
   <213> artificial
<220>
   <223> Antimicrobial peptide- Retro form of antimicrobial peptide AGG01
<400> 33
   Pro Leu Pro Ile Pro Ile Val Val Asn Phe Asn Lys Leu Arg Lys Lys
   1               5                   10                  15
   Ile Ser Asn Arg Phe Lys Lys Leu Arg Lys Arg Leu Lys Lys Gly Phe
               20                  25                  30
   Gly Arg Lys
           35
<210> 34
   <211> 35
   <212> PRT
   <213> artificial
<220>
   <223> Antimicrobial peptide-retro form of antimicrobial peptide AGG02
<400> 34
   Gln Arg Pro Val Pro Phe Lys Ile Lys Phe Asn Arg Leu Thr Asn Arg
   1               5                   10                  15
   Ile Asp Asp Gly Leu Lys Thr Ala Lys Arg Lys Leu Ser Glu Trp Leu
               20                  25                  30
   Gly Arg Lys
           35
<210> 35
   <211> 32
   <212> PRT
   <213> artificial
<220>
   <223> Antimicrobial peptide retro form of antimicrobial peptide AGG03
<400> 35
   Leu Ala Glu Met Tyr Pro Ser Lys Lys Val Gly Pro Lys Gly Leu Ala
   1               5                   10                  15
   Ile Val Ser Tyr Ser Gly Pro Lys His Arg Lys Ser Gly Lys Lys Arg
               20                  25                  30
<210> 36
   <211> 38
   <212> PRT
   <213> artificial
<220>
   <223> Antimicrobial peptide retro form of antimicrobial peptide A12
<400> 36
   Gly Pro Arg Pro Val Pro Phe Val Val Asn Phe Asn Lys Leu Arg Lys
   1               5                   10                  15
   Lys Ile Ser Asn Arg Phe Lys Lys Pro Arg Lys Arg Leu Arg Lys Gly
               20                  25                  30
   Phe Gly Arg Lys Val Met
           35
<210> 37
   <211> 38
   <212> PRT
   <213> artificial
<220>
   <223> Antimicrobial peptide retro form of antimicrobial peptide 5
<400> 37
   Gly Pro Arg Pro Ile Pro Phe Val Val Asn Phe Asn Arg Leu Thr Asn
   1               5                   10                  15
   Lys Ile Gly Asn Gly Ile Lys Thr Ala Arg Arg Lys Leu Asn Lys Gly
               20                  25                  30
   Leu Gly Arg Lys Val Met
           35
<210> 38
   <211> 38
   <212> PRT
   <213> artificial
<220>
   <223> Antimicrobial peptide retro form of antimicrobial peptide 6
<400> 38
   Gly Pro Leu Pro Ile Pro Ile Ile Ile Asn Phe Asn Arg Leu Thr Lys
   1               5                   10                  15
   Arg Ile Ser Asp Gly Phe Lys Thr Val Arg Lys Lys Leu Ser Glu Gly
               20                  25                  30
   Leu Gly Arg Lys Val Met
           35
<210> 39
   <211> 24
   <212> PRT
   <213> artificial
<220>
   <223> Antimicrobial peptide retro form of antimicrobial peptide 13
<400> 39
   Asp Ile Gly Leu Ile Met Gly Phe Lys Lys Leu Lys Arg Lys Leu Ser
   1               5                   10                  15
   Lys Trp Phe Gly Arg Lys Val Met
               20
<210> 40
   <211> 38
   <212> PRT
   <213> artificial
<220>
   <223> Antimicrobial peptide retro form of antimicrobial peptide A4
<400> 40
   Gly Gln Arg Pro Val Pro Phe Glu Ile Asn Phe Asn Lys Leu Arg Asn
   1               5                   10                  15
   Arg Ile Ser Asn Arg Phe Lys Thr Pro Arg Arg Arg Leu Asn Lys Gly
               20                  25                  30
   Phe Gly Arg Lys Val Met
           35
<210> 41
   <211> 38
   <212> PRT
   <213> artificial
<220>
   <223> Antimicrobial peptide retro form of antimicrobial peptide A5
<400> 41
   Gly Pro Leu Pro Val Pro Ile Ile Ile Asn Phe Asn Lys Leu Thr Lys
   1               5                   10                  15
   Arg Ile Ser Asn Arg Phe Lys Lys Ala Lys Arg Lys Leu Lys Lys Trp
               20                  25                  30
   Phe Gly Arg Lys Val Met
           35
<210> 42
   <211> 38
   <212> PRT
   <213> artificial
<220>
   <223> Antimicrobial peptide retro form of antimicrobial peptide A6
<400> 42
   Gly Gln Arg Pro Ile Pro Ile Glu Ile Asn Phe Asn Lys Leu Arg Lys
   1               5                   10                  15
   Lys Ile Ser Asn Arg Phe Lys Lys Pro Lys Lys Lys Leu Lys Lys Gly
               20                  25                  30
   Leu Gly Arg Lys Val Met
           35
<210> 43
   <211> 38
   <212> PRT
   <213> artificial
<220>
   <223> Antimicrobial peptide retro form of antimicrobial peptide A10
<400> 43
   Gly Pro Leu Pro Val Pro Ile Lys Val Asn Phe Asn Lys Leu Arg Lys
   1               5                   10                  15
   Arg Ile Ser Asn Arg Phe Lys Lys Ala Lys Lys Lys Leu Lys Lys Gly
               20                  25                  30
   Phe Gly Arg Lys Val Met
           35
<210> 44
   <211> 38
   <212> PRT
   <213> artificial
<220>
   <223> Antimicrobial peptide retro form of antimicrobial peptide A11
<400> 44
   Gly Gln Arg Pro Val Pro Phe Ile Ile Asn Phe Asn Lys Leu Arg Lys
   1               5                   10                  15
   Arg Ile Ser Asn Arg Phe Lys Thr Ala Arg Lys Arg Leu Lys Lys Gly
               20                  25                  30
   Phe Gly Arg Lys Val Met
           35
<210> 45
   <211> 24
   <212> PRT
   <213> artificial
<220>
   <223> Antimicrobial peptide retro form of antimicrobial peptide 10
<400> 45
   His Arg Arg Leu Val Ile Gly Leu Asn Thr Leu Lys Arg Arg Leu Arg
   1               5                   10                  15
   Glu Gly Leu Gly Arg Lys Val Met
               20
<210> 46
   <211> 24
   <212> PRT
   <213> artificial
<220>
   <223> Antimicrobial peptide retro form of antimicrobial peptide 20
<400> 46
   His Lys Lys Ile Asp Asp Arg Leu Lys Thr Val Arg Arg Arg Leu Ser
   1               5                   10                  15
   Glu Gly Phe Gly Arg Lys Val Met
               20
<210> 47
   <211> 24
   <212> PRT
   <213> artificial
<220>
   <223> Antimicrobial peptide retro form of antimicrobial peptide 27
<400> 47
   His Arg Arg Leu Ile Met Gly Leu Lys Thr Ala Lys Arg Arg Leu Arg
   1               5                   10                  15
   Lys Gly Phe Gly Arg Lys Val Met
               20
<210> 48
   <211> 24
   <212> PRT
   <213> artificial
<220>
   <223> Antimicrobial peptide retro form of antimicrobial peptide 28
<400> 48
   His Ile Gly Leu Val Ile Gly Phe Lys Lys Pro Arg Arg Arg Leu Lys
   1               5                   10                  15
   Glu Gly Leu Gly Arg Lys Val Met
               20
<210> 49
   <211> 53
   <212> PRT
   <213> artificial
<220>
   <223> Antimicrobial peptide retro form of antimicrobial peptide B1
<400> 49
   Phe Ala Glu Phe Glu Trp Ile Thr Gly Ala Ala Ser Arg Ser Ser Gly
   1               5                   10                  15
   Arg Leu Leu Leu His Ser Cys Ile Arg Ile Thr Arg Ile Thr Glu Arg
               20                  25                  30
   Ile Lys Gly Phe Phe Glu Glu Phe Lys Gly Gly Val Arg Arg Leu Leu
           35                  40                  45
   Asp Gly Leu Val Met
       50
<210> 50
   <211> 64
   <212> PRT
   <213> artificial
<220>
   <223> Antimicrobial peptide retro form of antimicrobial peptide B6
<400> 50
   Phe Ala Glu Phe Glu Trp Ile Thr Gly Ala Ala Ser Arg Ser Ser Gly
   1               5                   10                  15
   Arg Leu Val Leu Asp Trp Ser Leu Arg Leu Ile Gly Arg Leu Val Leu
               20                  25                  30
   Asp Trp Ser Leu Arg Leu Ile Gly Leu Ser Lys Asp Ile Gly Gly Val
           35                  40                  45
   Cys Lys Glu Phe Ser Gly Arg Phe Glu Gly Ser Val Ile Gly Val Met
       50                  55                  60
<210> 51
   <211> 38
   <212> PRT
   <213> artificial
<220>
   <223> Antimicrobial peptide retro form of antimicrobial peptide E3
<400> 51
   Gly Glu Ile Arg Arg Gly Leu Met Asp Leu Leu Asn Lys Leu Thr Ala
   1               5                   10                  15
   Arg Phe Arg Gly Phe Met Gly Val Leu Arg Gly Gly Gly Lys Arg Leu
               20                  25                  30
   Cys Gly Gly Leu Val Met
           35
<210> 52
   <211> 38
   <212> PRT
   <213> artificial
<220>
   <223> Antimicrobial peptide retro form of antimicrobial peptide F4
<400> 52
   Asp Gln Ser Pro Ala Ala Leu Ile Glu Met Phe Thr Arg Ile Lys Ala
   1               5                   10                  15
   Glu Tyr Arg Glu Phe Ile Arg Arg Phe Arg Glu Arg Val Lys Gly Ser
               20                  25                  30
   Arg Gly Gly Leu Val Met
           35
<210> 53
   <211> 38
   <212> PRT
   <213> artificial
<220>
   <223> Antimicrobial peptide retro form of antimicrobial peptide F6
<400> 53
   His Pro Ile Arg Thr Ala Ile Met Lys Leu Phe Asp Lys Leu Thr Ala
   1               5                   10                  15
   Gly Asp Arg Gly Arg Asn Arg Leu Ile Lys Gly Gly Phe Arg Lys Leu
               20                  25                  30
   Phe Gly Arg Leu Val Met
           35
<210> 54
   <211> 29
   <212> PRT
   <213> artificial
<220>
   <223> Antimicrobial peptide retro form of antimicrobial peptide F7
<400> 54
   Gly Ala Ile Arg Val Val Leu Asn Lys Met Phe Asp Arg Leu Lys Glu
   1               5                   10                  15
   Glu Leu Trp Arg Cys Arg Ser Phe Gly Gly Leu Val Met
               20                  25
<210> 55
   <211> 38
   <212> PRT
   <213> artificial
<220>
   <223> Antimicrobial peptide retro form of antimicrobial peptide F12
<400> 55
   Gly Ala Ser Arg Gly Gly Met Ile Asn Met Leu Pro Arg Leu Arg Ala
   1               5                   10                  15
   Gly Asn Arg Gly Arg Leu Glu Val Leu Arg Glu Arg Phe Lys Lys Phe
               20                  25                  30
   Arg Asp Gly Ile Val Met
           35
<210> 56
   <211> 47
   <212> PRT
   <213> artificial
<220>
   <223> Antimicrobial peptide retro form of antimicrobial peptide G9
<400> 56
   Phe Ala Glu Phe Glu Trp Ile Thr Gly Ala Ala Ser Arg Ser Ser Ser
   1               5                   10                  15
   Arg Val Val Phe Tyr Ser Ile Gln Cys Phe Leu Ser Leu Gly Lys Lys
               20                  25                  30
   Trp Ile Glu Trp Arg Val Asn Arg Leu Phe Gly Gly Leu Val Met
           35                  40                  45
<210> 57
   <211> 38
   <212> PRT
   <213> artificial
<220>
   <223> Antimicrobial peptide retro form of antimicrobial peptide G10
<400> 57
   Gln Gln Ser Arg Arg Arg Phe Met Asp Met Ile Ala Arg Leu Arg Lys
   1               5                   10                  15
   Gly Asn Arg Gly Leu Leu Gly Leu Ile Arg Gly Lys Gly Arg Lys Leu
               20                  25                  30
   Leu Gly Arg Ile Val Met
           35
<210> 58
   <211> 32
   <212> PRT
   <213> artificial
<220>
   <223> Antimicrobial peptide retro form of antimicrobial peptide H6
<400> 58
   Trp Arg Leu Arg Leu Ile Gly Leu Gly Gln Asn Leu Asp Arg Val Thr
   1 5 10 15
   Asn Glu Leu Ser Gly Arg Val Arg Arg Leu Cys Asp Gly Ile Val Met
   20 25 30
<210> 59
   <211> 35
   <212> PRT
   <213> artificial
<220>
   <223> Retro-inverted peptide analog of peptide AGG01 wherein each amino acid other than glycine is a D-amino acid
<400> 59
   Pro Leu Pro Ile Pro Ile Val Val Asn Phe Asn Lys Leu Arg Lys Lys
   1 5 10 15
   Ile Ser Asn Arg Phe Lys Lys Leu Arg Lys Arg Leu Lys Lys Gly Phe
   20 25 30
   Gly Arg Lys
   35
<210> 60
   <211> 35
   <212> PRT
   <213> artificial
<220>
   <223> Retro-inverted peptide analog of peptide AGG02 wherein each amino acid other than glycine is a D-amino acid
<400> 60
   Gln Arg Pro Val Pro Phe Lys Ile Lys Phe Asn Arg Leu Thr Asn Arg
   1 5 10 15
   Ile Asp Asp Gly Leu Lys Thr Ala Lys Arg Lys Leu Ser Glu Trp Leu
   20 25 30
   Gly Arg Lys
   35
<210> 61
   <211> 38
   <212> PRT
   <213> artificial
<220>
   <223> retro-inverso form of antimicrobial peptide A12 in which all amino acids other than glycine are D amino acids
<400> 61
   Gly Pro Arg Pro Val Pro Phe Val Val Asn Phe Asn Lys Leu Arg Lys
   1 5 10 15
   Lys Ile Ser Asn Arg Phe Lys Lys Pro Arg Lys Arg Leu Arg Lys Gly
               20                  25                  30
   Phe Gly Arg Lys Val Met
           35
<210> 62
   <211> 38
   <212> PRT
   <213> artificial
<220>
   <223> retro-inverso form of antimicrobial peptide 5 in which all amino acids other than glycine are D amino acids
<400> 62
   Gly Pro Arg Pro Ile Pro Phe Val Val Asn Phe Asn Arg Leu Thr Asn
   1               5                   10                  15
   Lys Ile Gly Asn Gly Ile Lys Thr Ala Arg Arg Lys Leu Asn Lys Gly
               20                  25                  30
   Leu Gly Arg Lys Val Met
           35
<210> 63
   <211> 38
   <212> PRT
   <213> artificial
<220>
   <223> retro-inverso form of antimicrobial peptide 6 in which all amino acids other than glycine are D amino acids
<400> 63
   Gly Pro Leu Pro Ile Pro Ile Ile Ile Asn Phe Asn Arg Leu Thr Lys
   1               5                   10                  15
   Arg Ile Ser Asp Gly Phe Lys Thr Val Arg Lys Lys Leu Ser Glu Gly
               20                  25                  30
   Leu Gly Arg Lys Val Met
           35
<210> 64
   <211> 24
   <212> PRT
   <213> artificial
<220>
   <223> retro-inverso form of antimicrobial peptide 13 in which all amino acids other than glycine are D amino acids
<400> 64
   Asp Ile Gly Leu Ile Met Gly Phe Lys Lys Leu Lys Arg Lys Leu Ser
   1               5                   10                  15
   Lys Trp Phe Gly Arg Lys Val Met
               20
<210> 65
   <211> 38
   <212> PRT
   <213> artificial
<220>
   <223> retro-inverso form of antimicrobial peptide A4 in which all amino acids other than glycine are D amino acids
<400> 65
   Gly Gln Arg Pro Val Pro Phe Glu Ile Asn Phe Asn Lys Leu Arg Asn
   1               5                   10                  15
   Arg Ile Ser Asn Arg Phe Lys Thr Pro Arg Arg Arg Leu Asn Lys Gly
               20                  25                  30
   Phe Gly Arg Lys Val Met
           35
<210> 66
   <211> 38
   <212> PRT
   <213> artificial
<220>
   <223> retro-inverso form of antimicrobial peptide A5 in which all amino acids other than glycine are D amino acids
<400> 66
   Gly Pro Leu Pro Val Pro Ile Ile Ile Asn Phe Asn Lys Leu Thr Lys
   1               5                   10                  15
   Arg Ile Ser Asn Arg Phe Lys Lys Ala Lys Arg Lys Leu Lys Lys Trp
               20                  25                  30
   Phe Gly Arg Lys Val Met
           35
<210> 67
   <211> 38
   <212> PRT
   <213> artificial
<220>
   <223> retro-inverso form of antimicrobial peptide A6 in which all amino acids other than glycine are D amino acids
<400> 67
   Gly Gln Arg Pro Ile Pro Ile Glu Ile Asn Phe Asn Lys Leu Arg Lys
   1               5                   10                  15
   Lys Ile Ser Asn Arg Phe Lys Lys Pro Lys Lys Lys Leu Lys Lys Gly
               20                  25                  30
   Leu Gly Arg Lys Val Met
           35
<210> 68
   <211> 38
   <212> PRT
   <213> artificial
<220>
   <223> retro-inverso form of antimicrobial peptide A10 in which all amino acids other than glycine are D amino acids
<400> 68
   Gly Pro Leu Pro Val Pro Ile Lys Val Asn Phe Asn Lys Leu Arg Lys
   1               5                   10                  15
   Arg Ile Ser Asn Arg Phe Lys Lys Ala Lys Lys Lys Leu Lys Lys Gly
               20                  25                  30
   Phe Gly Arg Lys Val Met
           35
<210> 69
   <211> 38
   <212> PRT
   <213> artificial
<220>
   <223> retro-inverso form of antimicrobial peptide A11 in which all amino acids other than glycine are D amino acids
<400> 69
   Gly Gln Arg Pro Val Pro Phe Ile Ile Asn Phe Asn Lys Leu Arg Lys
   1               5                   10                  15
   Arg Ile Ser Asn Arg Phe Lys Thr Ala Arg Lys Arg Leu Lys Lys Gly
               20                  25                  30
   Phe Gly Arg Lys Val Met
           35
<210> 70
   <211> 24
   <212> PRT
   <213> artificial
<220>
   <223> retro-inverso form of antimicrobial peptide 10 in which all amino acids other than glycine are D amino acids
<400> 70
   His Arg Arg Leu Val Ile Gly Leu Asn Thr Leu Lys Arg Arg Leu Arg
   1               5                   10                  15
   Glu Gly Leu Gly Arg Lys Val Met
               20
<210> 71
   <211> 24
   <212> PRT
   <213> artificial
<220>
   <223> retro-inverso form of antimicrobial peptide 20 in which all amino acids other than glycine are D amino acids
<400> 71
   His Lys Lys Ile Asp Asp Arg Leu Lys Thr Val Arg Arg Arg Leu Ser
   1               5                   10                  15
   Glu Gly Phe Gly Arg Lys Val Met
               20
<210> 72
   <211> 24
   <212> PRT
   <213> artificial
<220>
   <223> retro-inverso form of antimicrobial peptide 27 in which all amino acids other than glycine are D amino acids
<400> 72
   His Arg Arg Leu Ile Met Gly Leu Lys Thr Ala Lys Arg Arg Leu Arg
   1               5                   10                  15
   Lys Gly Phe Gly Arg Lys Val Met
               20
<210> 73
   <211> 24
   <212> PRT
   <213> artificial
<220>
   <223> retro-inverso form of antimicrobial peptide 28 in which all amino acids other than glycine are D amino acids
<400> 73
   His Ile Gly Leu Val Ile Gly Phe Lys Lys Pro Arg Arg Arg Leu Lys
   1               5                   10                  15
   Glu Gly Leu Gly Arg Lys Val Met
               20
<210> 74
   <211> 53
   <212> PRT
   <213> artificial
<220>
   <223> retro-inverso form of antimicrobial peptide B1 in which all amino acids other than glycine are D amino acids
<400> 74
   Phe Ala Glu Phe Glu Trp Ile Thr Gly Ala Ala Ser Arg Ser Ser Gly
   1               5                   10                  15
   Arg Leu Leu Leu His Ser Cys Ile Arg Ile Thr Arg Ile Thr Glu Arg
               20                  25                  30
   Ile Lys Gly Phe Phe Glu Glu Phe Lys Gly Gly Val Arg Arg Leu Leu
           35                  40                  45
   Asp Gly Leu Val Met
       50
<210> 75
   <211> 64
   <212> PRT
   <213> artificial
<220>
   <223> retro-inverso form of antimicrobial peptide B6 in which all amino acids other than glycine are D amino acids
<400> 75
   Phe Ala Glu Phe Glu Trp Ile Thr Gly Ala Ala Ser Arg Ser Ser Gly
   1               5                   10                  15
   Arg Leu Val Leu Asp Trp Ser Leu Arg Leu Ile Gly Arg Leu Val Leu
               20                  25                  30
   Asp Trp Ser Leu Arg Leu Ile Gly Leu Ser Lys Asp Ile Gly Gly Val
           35                  40                  45
   Cys Lys Glu Phe Ser Gly Arg Phe Glu Gly Ser Val Ile Gly Val Met
       50                  55                  60
<210> 76
   <211> 38
   <212> PRT
   <213> artificial
<220>
   <223> retro-inverso form of antimicrobial peptide E3 in which all amino acids other than glycine are D amino acids
<400> 76
   Gly Glu Ile Arg Arg Gly Leu Met Asp Leu Leu Asn Lys Leu Thr Ala
   1               5                   10                  15
   Arg Phe Arg Gly Phe Met Gly Val Leu Arg Gly Gly Gly Lys Arg Leu
               20                  25                  30
   Cys Gly Gly Leu Val Met
           35
<210> 77
   <211> 38
   <212> PRT
   <213> artificial
<220>
   <223> retro-inverso form of antimicrobial peptide F4 in which all amino acids other than glycine are D amino acids
<400> 77
   Asp Gln Ser Pro Ala Ala Leu Ile Glu Met Phe Thr Arg Ile Lys Ala
   1               5                   10                  15
   Glu Tyr Arg Glu Phe Ile Arg Arg Phe Arg Glu Arg Val Lys Gly Ser
               20                  25                  30
   Arg Gly Gly Leu Val Met
           35
<210> 78
   <211> 38
   <212> PRT
   <213> artificial
<220>
   <223> retro-inverso form of antimicrobial peptide F6 in which all amino acids other than glycine are D amino acids
<400> 78
   His Pro Ile Arg Thr Ala Ile Met Lys Leu Phe Asp Lys Leu Thr Ala
   1               5                   10                  15
   Gly Asp Arg Gly Arg Asn Arg Leu Ile Lys Gly Gly Phe Arg Lys Leu
               20                  25                  30
   Phe Gly Arg Leu Val Met
           35
<210> 79
   <211> 29
   <212> PRT
   <213> artificial
<220>
   <223> retro-inverso form of antimicrobial peptide F7 in which all amino acids other than glycine are D amino acids
<400> 79
   Gly Ala Ile Arg Val Val Leu Asn Lys Met Phe Asp Arg Leu Lys Glu
   1               5                   10                  15
   Glu Leu Trp Arg Cys Arg Ser Phe Gly Gly Leu Val Met
               20                  25
<210> 80
   <211> 38
   <212> PRT
   <213> artificial
<220>
   <223> retro-inverso form of antimicrobial peptide F12 in which all amino acids other than glycine are D amino acids
<400> 80
   Gly Ala Ser Arg Gly Gly Met Ile Asn Met Leu Pro Arg Leu Arg Ala
   1               5                   10                  15
   Gly Asn Arg Gly Arg Leu Glu Val Leu Arg Glu Arg Phe Lys Lys Phe
               20                  25                  30
   Arg Asp Gly Ile Val Met
           35
<210> 81
   <211> 47
   <212> PRT
   <213> artificial
<220>
   <223> retro-inverso form of antimicrobial peptide G9 in which all amino acids other than glycine are D amino acids
<400> 81
   Phe Ala Glu Phe Glu Trp Ile Thr Gly Ala Ala Ser Arg Ser Ser Ser
   1               5                   10                  15
   Arg Val Val Phe Tyr Ser Ile Gln Cys Phe Leu Ser Leu Gly Lys Lys
               20                  25                  30
   Trp Ile Glu Trp Arg Val Asn Arg Leu Phe Gly Gly Leu Val Met
           35                  40                  45
<210> 82
   <211> 38
   <212> PRT
   <213> artificial
<220>
   <223> retro-inverso form of antimicrobial peptide G10 in which all amino acids other than glycine are D amino acids
<400> 82
   Gln Gln Ser Arg Arg Arg Phe Met Asp Met Ile Ala Arg Leu Arg Lys
   1               5                   10                  15
   Gly Asn Arg Gly Leu Leu Gly Leu Ile Arg Gly Lys Gly Arg Lys Leu
               20                  25                  30
   Leu Gly Arg Ile Val Met
           35
<210> 83
   <211> 32
   <212> PRT
   <213> artificial
<220>
   <223> retro-inverso form of antimicrobial peptide H6 in which all amino acids other than glycine are D amino acids
<400> 83
   Trp Arg Leu Arg Leu Ile Gly Leu Gly Gln Asn Leu Asp Arg Val Thr
   1               5                   10                  15
   Asn Glu Leu Ser Gly Arg Val Arg Arg Leu Cys Asp Gly Ile Val Met
               20                  25                  30
<210> 84
   <211> 108
   <212> DNA
   <213> artificial
<220>
   <223> Nucleic acid encoding antimicrobial peptide
<400> 84
   aagagaggat ttgggaagaa gttgaggaag agactgaaga aatttcggaa cagcattaag      60
   aaaagattaa agaactttaa cgttgtaatt cctatcccac tgccaggg                  108
<210> 85
   <211> 108
   <212> DNA
   <213> artificial
<220>
   <223> Nucleic acid encoding antimicrobial peptide
<400> 85
   aagagaggac tttgggagag tttgaagagg aaagcgacga aactcgggga tgatattagg      60
   aatacattaa ggaactttaa aattaaattt cctgtccccc gccagggg                  108
<210> 86
   <211> 108
   <212> DNA
   <213> artificial
<220>
   <223> Nucleic acid encoding antimicrobial peptide
<400> 86
   aagcgaggat ttgggaagaa gctcaggaag agactgaaga aatttaggaa tagtattaag      60
   aagagattaa agaactttaa cgttgtaatt ccaatcccac tgccggga                  108
<210> 87
   <211> 108
   <212> DNA
   <213> artificial
<220>
   <223> Nucleic acid encoding antimicrobial peptide
<400> 87
   aagcgaggac tttgggagag tctcaagagg aaagcgacga aacttgggga tgatattagg       60
   aatacattaa ggaactttaa aattaaattt ccagtcccac ggcaggga                   108
<210> 88
   <211> 128
   <212> DNA
   <213> artificial
<220>
   <223> consensus sequence
<220>
   <221> CDS
   <222> (9) .. (119)
<400> 88
   ccatggtg aag aga gga ytt kgg rag ark ctc arg arg ara syg amg aaa        50
            Lys Arg Gly Xaa Xaa Xaa Xaa Leu Xaa Xaa Xaa Xaa Xaa Lys
            1               5                   10
   ytt rgg rat rrt att arg aak asa tta arg aac ttt aam rtt rwa wtt         98
   Xaa Xaa Xaa Xaa Ile Xaa Xaa Xaa Leu Xaa Asn Phe Xaa Xaa Xaa Xaa
   15                  20                  25                  30
   cca rtc cca ckg cmg gga tag ctcgagtta                                  128
   Pro Xaa Pro Xaa Xaa Gly
                   35
<210> 89
   <211> 36
   <212> PRT
   <213> artificial
<220>
   <221> misc_feature
   <222> (4) .. (4)
   <223> The 'Xaa' at location 4 stands for Leu, or Phe.
<220>
   <221> misc_feature
   <222> (5) .. (5)
   <223> The 'Xaa' at location 5 stands for Gly, or Trp.
<220>
   <221> misc_feature
   <222> (6)..(6)
   <223> The 'Xaa' at location 6 stands for Glu, or Lys.
<220>
   <221> misc_feature
   <222> (7) .. (7)
   <223> The 'Xaa' at location 7 stands for Arg, Ser, Lys, or Asn.
<220>
   <221> misc_feature
   <222> (9) .. (9)
   <223> The 'Xaa' at location 9 stands for Arg, or Lys.
<220>
   <221> misc_feature
   <222> (10) .. (10)
   <223> The 'Xaa' at location 10 stands for Arg, or Lys.
<220>
   <221> misc_feature
   <222> (11) .. (11)
   <223> The 'Xaa' at location 11 stands for Arg, or Lys.
<220>
   <221> misc_feature
   <222> (12) .. (12)
   <223> The 'Xaa' at location 12 stands for Ala, Val, Pro, or Leu.
<220>
   <221> misc_feature
   <222> (13) .. (13)
   <223> The 'Xaa' at location 13 stands for Lys, or Thr.
<220>
   <221> misc_feature
   <222> (15) .. (15)
   <223> The 'Xaa' at location 15 stands for Leu, or Phe.
<220>
   <221> misc_feature
   <222> (16) .. (16)
   <223> The 'Xaa' at location 16 stands for Gly, or Arg.
<220>
   <221> misc_feature
   <222> (17) .. (17)
   <223> The 'Xaa' at location 17 stands for Asp, or Asn.
<220>
   <221> misc_feature
   <222> (18) .. (18)
   <223> The 'Xaa' at location 18 stands for Gly, Asp, Ser, or Asn.
<220>
   <221> misc_feature
   <222> (20) .. (20)
   <223> The 'Xaa' at location 20 stands for Arg, or Lys.
<220>
   <221> misc_feature
   <222> (21) .. (21)
   <223> The 'Xaa' at location 21 stands for Lys, or Asn.
<220>
   <221> misc_feature
   <222> (22) .. (22)
   <223> The 'Xaa' at location 22 stands for Arg, or Thr.
<220>
   <221> misc_feature
   <222> (24) .. (24)
   <223> The 'Xaa' at location 24 stands for Arg, or Lys.
<220>
   <221> misc_feature
   <222> (27) .. (27)
   <223> The 'Xaa' at location 27 stands for Lys, or Asn.
<220>
   <221> misc_feature
   <222> (28) .. (28)
   <223> The 'Xaa' at location 28 stands for Val, or Ile.
<220>
   <221> misc_feature
   <222> (29) .. (29)
   <223> The 'Xaa' at location 29 stands for Glu, Val, Lys, or Ile.
<220>
   <221> misc_feature
   <222> (30) ..(30)
   <223> The 'Xaa' at location 30 stands for Ile, or Phe.
<220>
   <221> misc_feature
   <222> (32) .. (32)
   <223> The 'Xaa' at location 32 stands for Val, or Ile.
<220>
   <221> misc_feature
   <222> (34) .. (34)
   <223> The 'Xaa' at location 34 stands for Arg, or Leu.
<220>
   <221> misc_feature
   <222> (35) .. (35)
   <223> The 'Xaa' at location 35 stands for Gln, or Pro.
<220>
   <223> Synthetic Construct
<400> 89
   Lys Arg Gly Xaa Xaa Xaa Xaa Leu Xaa Xaa Xaa Xaa Xaa Lys Xaa Xaa
   1               5                   10                  15
   Xaa Xaa Ile Xaa Xaa Xaa Leu Xaa Asn Phe Xaa Xaa Xaa Xaa Pro Xaa
               20                  25                  30
   Pro Xaa Xaa Gly
           35
<210> 90
   <211> 108
   <212> DNA
   <213> artificial
<220>
   <223> sequence encoding antimicrobial peptide
<400> 90
   aagcgaggat ttgggaagaa gctcaggaag agactgaaga aatttaggaa tagtattaag 60
   aagagattaa agaactttaa cgttgtaatt ccaatcccac tgccggga 108
<210> 91
   <211> 108
   <212> DNA
   <213> artificial
<220>
   <223> sequence encoding antimicrobial peptide
<400> 91
   aagcgaggac tttgggagag tctcaagagg aaagcgacga aacttgggga tgatattagg 60
   aatacattaa ggaactttaa aattaaattt ccagtcccac ggcaggga 108
<210> 92
   <211> 1723
   <212> DNA
   <213> artificial
<220>
   <223> bovine beta-casein promoter
<400> 92
   tcgaatccat ctctatcaat taatgtaatt caaaattggt gagagacagt cattaggaaa 60
   ttctctgttt attgcacaat atgtaaagca tcttcctgag aaaagggaaa tgttgaatgg 120
   gaaggacatg ctttcttttg tattcctttt ctcagaaatc acactttttt gcctgtggcc 180
   ttggcaacca aaagctaaca cataaagaaa ggcatatgaa gtagccaagg ccttttctag 240
   ttatatctat gacactgagt tcatttcatc atttattttc ctgacttcct cctgggccat 300
   atgagcagtc ttagaatgaa tattagctga ataatccaaa tgcatagtag atgttgattt 360
   gggttttcta agcaatacaa gacttctatg acagtgagat gtattaccat ccaacacaca 420
   tctcagcatg atataaatgt aaggtatatt gtgaagaaaa attatcaatt atgtcaaagt 480
   gcttacttta gaagatcatc tatctgtccc aaagctgtga atatatatat tgaacataat 540
   taatagacga aacaaacctt gtaaaaatga gtagtgtaaa atacaactac atttatgaac 600
   atctatcact aaagaggcaa agaaagttga ggactgcttt tgtaaatggg ctcttattaa 660
   tgaaaagtac ttttgaggtc tggcttagac tctattgtag tacttatggt aagaccctcc      720
   tcttgtctgg gctttcattt tctttcttcc ttccctcatt tgcccttcca tgaatactag      780
   ctgataaaca ttgactcact ataaaagata tgaggccaaa cttgagctgt ccattttaat      840
   aaatctgtat aaataatatt tgttctacag aagtatctct aaataaatgt actttctctc      900
   ttaaaatccc tcaacaaatc cccactatct agagaataag attgacattc cctggagtca      960
   cagcatgctt tgtctgccat tatctgaccc ctttctcttt ctctcttctc acctccatct     1020
   actccttttt ccttgcaata catgacccag attcactgtt tgatttggct tgcatgtgtg     1080
   tgtgctgagt tgtgtctcac tcttgtcaac cccatgaatg acagtccacc aggctccact     1140
   atttccagtt aagaatactg gagtggattg tgtttcctac ttcatttgat taatttagtg     1200
   actttttaaa tttttttcca tattcaggag gctattcttt ccttttagtc tatactgtct     1260
   tcgctcttca ggtctaagct atcatcatgt gcttgttagc ttgtttcttt ctccattata     1320
   gcataaacac taacaactat tcaggttagc atgagattgt gttctttgtg tggcctgtgt     1380
   atttctggtg tgtattagaa tttaccccaa gatctcaaag acccaccgaa tactaaagag     1440
   acctcattgt agttacaata atttggggac tgggccaaaa cttccgtgtg tcccagccaa     1500
   ggtctgtagc tactggacaa tttaatttcc tttatcagat tgtgaattat tccctttaaa     1560
   atgctcccca gaatttttgg ggacagaaaa ataggaagaa ttcattttct aatcatgcag     1620
   atttctagga attcaaatcc actattggtt ttatttcaaa ccacaaaatt agcatgccat     1680
   taaatactat atataaacaa ccacaaaatc agatcattat cca                       1723
<210> 93
   <211> 595
   <212> DNA
   <213> artificial
<220>
   <223> murine prolactin-inducible mammary gland promoter
<400> 93
   ggatccaagt agtagttgag tctcatgcta aatgccacca tgttccatcc cttttcccaa 60
   ggctctcagt tatgagtctc catatcaagg ggctttcctg gactttgtcc tatggctagg 120
   ttggacagac aaatatcacc tttgatccta ggatgtgata catccccttt ccacgttctg 180
   tatgtgttta ggggtaagca tggagttggc tgtagccaac tgtgttttcc agtcacctcc 240
   cttgtattgt ctctgaagcc tcctttgttc caaaagtagg ttaaggaaat cctgcttcct 300
   ggaagcagcc ctaaaagaaa tgaaggttta ccagagccaa gtgagaagct gggtcatgtg 360
   tggaattatg tgggaagaaa acaatacttg gtattgactg gatcgaggag atggggggag 420
   ggtggcagga tggagggagg ctggcaggct cagggtttct attttggcat aagcatctct 480
   tcatcattgt cttcctagag agaaggcccg gtgccaggag gccagaggcc ttcttcatac 540
   ataaaagcag atgaagtgag cggtgtctgc attacaaggt ccaggagcag tcaaa 595
<210> 94
   <211> 706
   <212> DNA
   <213> artificial
<220>
   <223> Bubalis bubalis alpha-lactalbumin promoter
<400> 94
   tatttagtgg tattggtggt tggggatggg gaagctgata acatctcaga gggtagctag 60
   atactgtcat acacactttt caagttctcc atttttgtga aatagaaagt ctctggatcc 120
   aagttatatg tgattctcag tctctgtggt catattctat ttctattcct gaccactcaa 180
   caaggaacca agatatcaag ggacacttct tttgtttcat gcctgggttg agtgggccag 240
   tgtcagctct gatcctggga ccatgacata cgatgatgta cagtcctttc ccatattctg 300
   tatgtctcta aggggaagga ggagttggcc atggaccctt tgtgcatttt ctcattttct 360
   gattgcttca cttgtattac ccctgaggcc ccctttgttc ctgaagtatg ttgggcacat 420
   cttgcttcct agaaccaaca ctaccagaaa caacataaag ccaaatggga aacaggatca 480
   tgtttgtaac actgcttggg caggtaacaa tacctagtat ggactagaga ttctggggag 540
   gaaaggaaaa gtggggtgaa attactgaag gaagctagca ggctcaatgt ttctttgttg 600
   gttttactgg cctctctcgt catcctcttc ctggatgtaa ggcttgatgc cagggcccct 660
   gaggcttttt ccacaaataa aaggaggtga gcagtgtggt gacccc 706
<210> 95
   <211> 2410
   <212> DNA
   <213> artificial
<220>
   <223> murine whey acidic gene promoter
<220>
   <221> misc_feature
   <222> (1679)..(1679)
   <223> n is a, c, g, or t
<400> 95
   gaattctttc actgctaaaa cagggcggga ggagtccaga gccctgccac tgggtgcaga      60
   acatgaagac cccttgagtg gaaaggggtt atacagctgg acagtggtgg cgcacacctt     120
   taatcccagc actcgggagg cagaggcaga cggatctctg agttcgaggc cagcctggtc     180
   tacagaatga gttccgggac agccagggcc acacagagaa actcttgtct cgaaaaacca     240
   aaaaaaaaca aaaaaggaaa ggggttacac aacagagact caggtcacag ctacccatca     300
   cacacaggat acacatacaa aggtgttcac aggcagatga ggaacgagga gaaggggctc     360
   aagcaagggc ctaaagtttc tttttttttt tttcttcttt ttttttttcc ctgtggccta     420
   gagtttcaag aggctgagga cctaggcatg aaccaagagg ggccaaacca cttcaagaag     480
   cagggggtag cagcagaatc tcactatcag ccttgagcac agctgggaag gagatccatg     540
   gaaacaacca agaaagagct gaaaggggct ggagagatgg ctcagcagtt aagagcactg     600
   agtgctcttc cgaagtccta gattcaaatc ccagcaacca tatggtgggt cacaaccatc     660
   tgtaatgaga tctgatgccc tcttctggtg tgtctgaaga cagctacagt gtacttatgt     720
   ataataaata aataaatctt tagaaaggga gggggggaga gagagagaga gagagagaga     780
   gagagagaga gagagagaga gagagagaga gagctggaag agggagatct gggaagtctg     840
   ctggctttat atgctgacca tatatagtca cctgtgttta cacactgtgc tcatcacttt     900
   gaaatctcag tggtttcttc tttgagcctg tgtctgtaag ttcaccagga gagtggtaca     960
   taggcaagaa taacagccag tgggcatagg acacagagtg catgggcccc agcaagactg    1020
   tagagagaac agagctctgg ctcctaagac atagggcctt ctgggaaact caagcagcca    1080
   agcaacccta gccagccctt tcctggtggc cctccttctg ttccagcaaa ggcggaaatg    1140
   ggaacagggg tggaagcaga gcattggcag agcataggta tgacttagtc ttgactaaca    1200
   caagcatggc agtagcctga cagtggccta aatgtgggga tgactgcctt agatggggat    1260
   gactgcctta gatgggcatg actgccttag atggggatga ctgccttaga tggggatgac    1320
   tgccttagat ggaacaacaa acatctatgg gcatgctgtg gaacactggc ccacacacgg    1380
   aactgaaggc actggcaatt tccatagggc agttaaacct aaaagcatgc tcacactcaa    1440
   caggctgccg gaatctcatg agacacctgg aatagacgaa tgtagaaaca gagcagagag    1500
   ttggttgcca aggtctgggg gctcagagga caagcaagag gcgcggcttt cctttggggc    1560
   tggcatgaaa ggaaatatcg aggttacagc ctgagagggc ttcccctgac acttcgtatt    1620
   caaagaggcc atgggcacca gtgaagacaa aggagtatgg cctgcaccac aggctggcnc 1680
   tgacagtcag taagcacaca gtcactctgg gtcatcccat ccccttcctt gcaagagaaa 1740
   tcaaggaaat gtcccgagaa caatggggca cagtgccagc aggacatctc ttcctgccca    1800
   tggcaccctt tggcacggta tgggcccttc tgggaaggtg gccttccaaa ttgctctgca    1860
   caggcagctc cttttcaatg tatgcccgac actctctaca tggagcaagc gcctccacac    1920
   tcttagaaga attttagaaa actccagaaa agcaccagga gaagtcaccc tcagatgtag    1980
   cccggactcg agccttgctc aaaacctcct gtcttgtttt ctatgtgacc tgtacaaatt    2040
   tggagctcag aattgccttt gtctgtgatg ggttccaacc caaccactca aagtgacact    2100
   tgtcacattt gtcactgatc ctatttcttc tttttctgct ccttcatttt ctccgctttc    2160
   ataataaaca agtattactt tttaagtggg ggaaaaaatg accaccctta caaaggactt    2220
   tttaaaaatg gcctccattg tggcccttgt tcctggcagc ctgggcctgc tctctctgtg    2280
   tggccaagaa ggaagtgttg tagcccatct agagctgtgc cagcctcttc ccccacccca   2340
   cccccaaagt cttcctcctg tgggtccttt aaatgcatcc cagacactca gacagccatc    2400
   agtcacttgc                                                           2410
<210> 96
   <211> 1332
   <212> DNA
   <213> artificial
<220>
   <223> camel whey acidic gene promoter
<400> 96
   tctgaagagg ggacattttg tgacctgcca acatgcaaag ttaccaaaac atagcaagtc      60
   gccatcggcc aggacctcta gaccccagtc gctaaagctc agtgctggct acccagggag     120
   gggcctggac tgaggtccta gaactctgct gaggccttgt agggactgag atggtgctac     180
   ctggggcctg gggcctgggg cctggggcct ggagcggggt gagccagggg gaccgtagca     240
   gcctgtcaaa gtggaagggt gttctgggca tctggaactg catgcagtcc aggctgaggg     300
   ccccagagaa gtactgaggg gctctgtgtc caaggccaag aagccacagg ccaggcagag     360
   gagtggggcc tggaccaggg gtgggcactg accaccagca cacgcagtca tcccgggcac     420
   accttccttg tccaagccct cagggcaaaa ggatcaagga aattccccgg agaaggaggg     480
   cacctagcct gagtgatcat cctgtcccca ccccggtccc tacacaggga cacaggcggg     540
   gcccttctgg gaataggctt tcccagtgtc tgccctgcgc agaaacagcc ccgaccctga     600
   acctgcctgc ccctcccttt ctaagacgcc cgacatcctc tgcacagagc atacggctcc     660
   taagtacaag acgactcgtt cttgccgtgg aaagttcggg aaaagacaaa aagatactgc     720
   aggaggaata aaatgccctc caaggtcccc ccacacccgg gctcctcctc cttctcctct     780
   cccggcggac ggagtgctga tttggacacg tcactccctg tccctgaagg gcctctccac     840
   gaccactgtg ttgtgtcatt gcggattcta tttcttcctt tgtctgccct taattttttg     900
   aatgttcaca ataaacatgc attactttca aagtggaaaa aaatggatcc actttatgag     960
   gaattctttt ttatttaaaa acgtggccca aggcagtggc cgcccagtct ggggtcggtc    1020
   caagctggaa gtcttttggt ccaactgggg cagggccagc cactcaccct cccccaccgt    1080
   gttcttcttc ccgcctcctc ctttaaaggt gccccagggc cacgagccac catctgtcac    1140
   ctgcctgcca cctgccacca tgcgctgtct tgccagctct ggcctctggc ctctgatctc    1200
   ctctggaggc tgctctttgc actggcccca gccatctcct tgccaggtaa gcccaggagg    1260
   ggcatcctgc catccctctg ctccaggtcc ccccaccccc cgaaatgctg cccaggcctc    1320
   acagtttggt ga                                                        1332
<210> 97
   <211> 1167
   <212> DNA
   <213> artificial
<220>
   <223> rat whey acidic gene promoter
<400> 97
   aggaaagcac actcgacact cgaacggact gcctactgtc agatcccatt tacatgagat 60
   gcccagaata gacagacgca gaaaccgagc agagaggtag ttgccaaggc ctgggggctc 120
   ggggaactag cgagaggctg ctggcaggca caggttttcc tttggggctg gcctgaaacg 180
   aaacatcaag gttacagcct gaaagagctt cccctgggac tttgtcttca aagaggagag 240
   gccatgggcc acagtgaaga cctccggcca gtcaaaggag tatgggctgc accataggct 300
   ggcgcgacag ccagtaaaca cacagtcact cactctcgag tcattgcatc cccttccttg 360
   caagagaagt caaggaaatg tcccgagagc aatgggcaca gtgcccaaca ggacatccca 420
   tccgggccca tgacaccgtt ggcacagcat ggggcccttc tgagaagtgg gctttcaagg 480
   ttccctgcac aggcaatcct tttttgatgt gtaccctgta ctctctacaa ggagcaagtg 540
   cctccacatt cttataaaac tttttagaaa actccagaaa agcaccaaga aaagaaacca 600
   tcctctgatg tgactgtaca catttggagc tcggaatttc cttttttttt tttttttaaa 660
   gatttttatt tatttcatgt atgggagcac actgtcgcta tcttcagaca caccagaaga 720
   gggcatcaga tcccactgga tcccagatgg ttgtgagcca ccatgtggtt gctgggacct 780
   gaactcagga cctctggaag agcagtcagt gctcccaacc actgagccat ctctccagcc 840
   ctcggaattt cctttgtccg agaaaggggt cccaacccaa ccattcaaag tgatatctgt 900
   cacatttgtt acagatccca tttcttcctt ctctgctcct taattttttt cgttttggcc 960
   ataaacaagt tttacctttt aagtgaaaaa ataacgacca cccttacaaa ggacttctta 1020
   aaaatggact ccgaattgtg aaccttgttc tggtagcctg ggcctgctct ctgcatgtgt 1080
   ccaagaggaa gtgttttagc ccatctacgc ctatgcaagc ctgcccccct ccttccccaa 1140
   agtcttcctc ctgtgggtcc tttaaat 1167
<210> 98
   <211> 195
   <212> DNA
   <213> artificial
<220>
   <223> Capra hircus beta-lactoglobulin promoter
<400> 98
   ggcccagagg gggacttcct gcttggcccc ggatggaaga aggcctccta ttgtcctcgt 60
   agaggaagcc accccggggc ccggggatga gccaagtagg attccgggaa cctcgtggct 120
   ggggcgcggc ccgggctggc tggctggcac gcctcctgta taaggccccg agcccgctgt 180
   ctcagccctc cactc 195
<210> 99
   <211> 4204
   <212> DNA
   <213> artificial
<220>
   <223> Ovis aries beta-lactoglobulin gene promoter
<400> 99
   gtcgacctgc aggtcaacgg atctctgtgt ctgttttcat gttagtacca cactgttttg      60
   gtggctgtag ctttcagcta cagtctgaag tcataaagcc tggtacctcc agctctgttc     120
   tctctcaaga ttgtgttctg ctgtttgggt ctttagtgtc tccacacaat ttttagaatt     180
   gtttgttcta gttctgtgaa aaatgatgct ggtattttga taaggattgc attgaatctg     240
   taaagctaca gatatagtca ttgggtagta cagtcacttt aacaatatta actcttcaca     300
   tctgtgagca tgatatattt tccccctcta tatcatcttc aattcctcct atcagtttct     360
   ttcattgcag ttttctgagt acaggtctta cacctccttg gttagagtca ttcctcagta     420
   ttttattcct ttgatacaat tgtgaatgag gtaattttct tagtttctct ttctgatagc     480
   tcattgttag tgtatatata gaaaagcaac agatttctat gtattaattt tgtatcctgc     540
   aacagatttc tatgtattaa ttttgtatcc tgctacttta cggaattcac ttattagctt     600
   tttggtgaca tcttgaggat tttctgaaga aaatggcatg gtatggtagg acaaggtgtc     660
   atgtcatctg caaacagtgg cagttttcct tcttcccttc caacctggat ttctttgatt     720
   tctttctgtc tgagtacgac taggattccc aatactatac cgaataaaag tggcaagagt     780
   ggacatcctt gtcttatttt tctgacctta gaggaaatgc tttcagtttt tcaccattaa     840
   ttataatgtt tactgtgggc ttgtcatatg tggccttcat tatatggagg tctattccct     900
   ctatacccac cttgttgaga gtttttatca taaaagtatg ttgaattttg tcaaaagttt     960
   ttcctgcatc tattgagatg atttttactc ttcaattcat taatgatttt tattcttcat    1020
   tttgttaatg atttccattc ttcaatttgt taacgtggta tatcacattg attgatttgt    1080
   ggataccttt gtatccctgg gataaacctc acttgatcat gagctttcaa tgtatttttg     1140
   aattcacttt gctaatattc tgttgggtat ttttgcatct ctattcatca atgatattgg    1200
   cctaagaaag gttttgtctg gttttagtat cagggtgatg ctggcctcat agagagagtt    1260
   tagaagcatt tcctcctctt tgatttttcg gaatagtttg agtaggatag gtattaactc    1320
   ttctttaaat gtttggggac ttccctggtg agccggtggt tgagaatccg cctcagggat    1380
   gtgggtttga tccctggtca gggaaccatt aataagatcc cacatgctgc aggcaacaag    1440
   cccccaagct gcaaccactg agctgcaacc gctgcagtgc ccacaggcca cgaccagaga    1500
   aagcccacat acagcaggga agacccagca caaccggaaa aaggagtttg gtggaataca    1560
   gctgtgaagc cgtctggtcc tggactcctg cttgagggaa ttttttaaaa attattgatt    1620
   caatttcatt actggtaact ggtctgttca tattttctat ttcttccggg ttcagtcttg    1680
   ggagattgta catgcctagg aatgtgtccg tttcttctag gttgtccatt ttattggaca    1740
   tgcatgggag cacacagcac cgaccagcga gactcatgct ggcttcctgg ggccaggctg    1800
   gggccccaag cagcatggca tcctagagtg tgtgaaagcc cactgaccct gcccagcccc 1860
   acaatttcat tctgagaagt gattccttgc ttctgcactt acaggcccag gatctgacct 1920
   gcttctgagg agcaggggtt ttggcaggac ggggagatgc tgagagccga cgggggtcca 1980
   ggtcccctcc caggcccccc tgtctggggc agcccttggg aaagattgcc ccagtctccc 2040
   tcctacagtg gtcagtccca gctgccccag gccagagctg ctttatttcc gtctctctct 2100
   ctggatggta ttctctggaa gctgaaggtt cctgaagtta tgaatagctt tcgggtgaag 2160
   ggcatggttt gtggtcacgg ttcacaggaa gcttgggaga ccctgcagct cagacgtccc 2220
   gagattggtg gcacccagat ttcctaagct cgctggggaa cagggcgctt gtttctccct 2280
   ggctgacctc cctcctccct gcatcaccca gttctgaaag cagagcggtg ctggggtcac 2340
   agcctctcgc atctaacgcc ggtgtccaaa ccacccgtgc tggtgttcgg ggggctacct 2400
   atggggaagg gcttctcact gcagtggtgc cccccgtccc ctctgagatc agaagtccca 2460
   gtccggacgt caaacaggcc gagctccctc cagaggctcc agggagggat ccttgccccc 2520
   ccgctgctgc ctccagctcc tggtgccgca cccttgagcc tgatcttgta gacgcctcag 2580
   tctagtctct gcctccgtgt tcacacgcct tctccccatg tcccctccgt gtccccgttt 2640
   tctctcacaa ggacaccgga cattagatta gcccctgttc cagcctcacc tgaacagctc 2700
   acatctgtaa agacctagat tccaaacaag attccaacct gaagttcccg gtggatgtga 2760
   gttctggggc gacatccttc aaccccatca cagcttgcag ttcatcgcaa aacatggaac 2820
   ctggggttta tcgtaaaacc caggttcttc atgaaacact gagcttcgag gcttgttgca 2880
   agaattaaag gtgctaatac agatcagggc aaggactgaa gctggctaag cctcctcttt 2940
   ccatcacagg aaaggggggc ctgggggcgg ctggaggtct gctcccgtga gtgagctctt 3000
   tcctgctaca gtcaccaaca gtctctctgg gaaggaaacc agaggccaga gagcaagccg 3060
   gagctagttt aggagacccc tgaacctcca cccaagatgc tgaccagcca gcgggccccc 3120
   tggaaagacc ctacagttca ggggggaaga ggggctgacc cgccaggtcc ctgctatcag 3180
   gagacatccc cgctatcagg agattccccc accttgctcc cgttccccta tcccaatacg 3240
   cccaccccac ccctgtgatg agcagtttag tcacttagaa tgtcaactga aggcttttgc 3300
   atcccctttg ccagaggcac aaggcaccca cagcctgctg ggtaccgacg cccatgtgga 3360
   ttcagccagg aggcctgtcc tgcaccctcc ctgctcgggc cccctctgtg ctcagcaaca 3420
   cacccagcac cagcattccc gctgctcctg aggtctgcag gcagctcgct gtagcctgag 3480
   cggtgtggag ggaagtgtcc tgggagattt aaaatgtgag aggcgggagg tgggaggttg 3540
   ggccctgtgg gcctgcccat cccacgtgcc tgcattagcc ccagtgctgc tcagccgtgc 3600
   ccccgccgca ggggtcaggt cactttcccg tcctggggtt attatgactc ttgtcattgc 3660
   cattgccatt tttgctaccc taactgggca gcaggtgctt gcagagccct cgataccgac 3720
   caggtcctcc ctcggagctc gacctgaacc ccatgtcacc cttgccccag cctgcagagg 3780
   gtgggtgact gcagagatcc cttcacccaa ggccacggtc acatggtttg gaggagctgg 3840
   tgcccaaggc agaggccacc ctccaggaca cacctgtccc cagtgctggc tctgacctgt 3900
   ccttgtctaa gaggctgacc ccggaagtgt tcctggcact ggcagccagc ctggacccag 3960
   agtccagaca cccacctgtg cccccgcttc tggggtctac caggaaccgt ctaggcccag 4020
   aggggacttc ctgcttggcc ttggatggaa gaaggcctcc tattgtcctc gtagaggaag 4080
   ccaccccggg gcctgaggat gagccaagtg ggattccggg aaccgcgtgg ctgggggccc 4140
   agcccgggct ggctggcctg catgcctcct gtataaggcc ccaagcctgc tgtctcagcc 4200
   ctcc 4204
<210> 100
   <211> 3834
   <212> DNA
   <213> artificial
<220>
   <223> Capra hircus beta-lactoglobulin gene promoter
<400> 100
   gtcaacggat ctctgtgtct gttttcatgt tagtaccata ctgttttggt ggctgtagct      60
   ttgagctata gtctgaagtc ataaagcccg atacctccag ctctgttctt ctttctcaag     120
   attgtgttct gctgtttggg tctttagtgt ctccacacaa tttttagaat tgtgtgttct     180
   agttctgtga aaaatgatgc tggcattttg ataaggattg cattgaatct gtaaagctac     240
   agatatagtc attgggtagt acaatcactt taacaatatt aactcttcaa atccgtgagc     300
   atgatgtatt ttccccctcc atatcatctt caattccttc tatcagtttc tttcattgca     360
   gttttctgag tataggtctt acacctcctt gattagagtc attcctcagt attttattcc     420
   tttgatacaa ttgtgaatga gatcattttc ttagtttctc tttctgatag cccattgtta     480
   gtgtatagaa aagcaacaga gttctatgta ttaattttgt atcctgcaac agatttctat     540
   gtattaattt tgtatcctgc tactttactg aatttactta ttagcttttt ggtgacatct     600
   taaggatttt cttaagaaaa tggcatggta tggtaggaca aggtgtcacg tcatctgcaa     660
   acagtggcag ttttacttct tcccttccag cctggatttc tttgatttct ttctgtctga     720
   gtactgtgac taggattccc aatactatac cgaacaaaag tggcaagagt ggacatcctt     780
   gtcttatttt tctgacctta gaggaaatgc tttcagtttt tcaccattaa ttataatgtt     840
   tactgtgggc ttgtcatatg tggccttcat tatatggagg tctattccct ctatacccac     900
   tttgttgaga gtttttatca tgaaagtatg ttgaattttg tcaacagttt ttcctgcatc     960
   tattgagatg atttttactc ttcaattcat taatgatttt tattcttcat tttgttaatg    1020
   atttccattc ttcaatgtgt taacgtggta tatcacattg attgatttgt ggatatcttt    1080
   gtatccctgg gataaacctc acctgatcat gagctttcaa tgtatttttg aattcacttt    1140
   gctaatattc tgctgggtat ttttacatct ctattcatca atgatattga cctaagattt 1200
   tctttctttt tttttttgta aagtttttgt gtggttttag tatcagggtg atgctggcct 1260
   catagagaga gtttagaagc atttcctcct ctttgatttt ttggaatagt ttgagtagga 1320
   taggtattaa ctcttcttta aatgtttggg gacttccctg gtgagccggt ggttgagaat 1380
   ccgcctcagg gatgtgggtt tgatccctgg tcagggaacc attaataagc tcccacatgc 1440
   tgcagggcaa caagccccca agctgcaacc actgagctgc aaccgctgca gtgcccacgg 1500
   gccacgacca gagaaagccc acatacagca gggaagaccc agcacaacct aaaaaaggag 1560
   tttggtggaa tacagctgtg aagccatctg gtcctggact cctgcttgag ggaatttttt 1620
   taaaattatt gattcaattt cattactgat tgccccagtc tccctcccac agtggtcagt 1680
   cccagctgcc ccaggccaga ggtgctttat ttccgtctct ctctctggat ggtattcttt 1740
   ggaagctgaa gattcctgga agttatgaat agcttcgccc tgaagggcat ggtttatggt 1800
   cacggttcac aggaacttgg gagaccctgc agctcagacg tcccgaggtt ggtggcaccc 1860
   agatttccta agctcgctgg ggaagggggc gcttgtttct ccctggctga cctccctccg 1920
   ccctgcatca cccagttctg agagcagagc ggtgctgggg ggcacagcct ctcgcatctg 1980
   acgccggtgt ccaaaccacc cgtgctggtg ttcggggggc tacctatggg gaagggctcc 2040
   tcactgcagg ggtgcccccc gtcccctctg agatcagaag tcccagtccg gacagcgaac 2100
   aggccaagct ccctccagag gctccaggaa gggatccttg ccccccgccg ccgcctccag 2160
   ctcctggtgc cgcacccttg agcctgatct tgtagacgcc tcagtctagt ctctgcctcc 2220
   gtgttcacat gccttctccc catgtcccct ccatgtcccc gttttctctc acaaggacac 2280
   cggacagtag attagcccct gttccagcct cacctgaaca gctcacatct gtaaagacct 2340
   agattccaaa caagattcca acctgaagtt cctggtggat gtgagttctg gggcaacatc 2400
   cttcaacccc atcacagctt gcagttcatc acaaaacatg gaacctgggg tttatcataa 2460
   aacctaggtt cttcatgaaa cactgagctt cgaggcttgt tgcaagaatt aaaggtgcta 2520
   atacagatca aggcaaggac tgaagctggc caagcctact ctttccatca caggaaaggg 2580
   gggtctgggg gcggctgggg gtctgctccc gtgagtgagc tcttttctgc tacagtcacc 2640
   aacagtctct ccgggaagga aaccagaggc cagagagcaa gccagagcta gtttaggaga 2700
   cccccgaacc tccaaccaag atgctgacca ggccagcggg ccccctggaa agaccctaca 2760
   gttcaggggg gaagaggggc tgacccgcca ggtccctgct atcaggagac atccccgcta 2820
   tcaggagatt cccccacctt gctcccgttc cgctacccca atacgcccac cccacccctg 2880
   tgatgagtgg tttagccact tagaatgtca actgaaggct tttgcaccct ctttgccaga 2940
   ggcacaaggc acccacagcc cgctgggtac caacgcccat gtggattcag ccaggaggcc 3000
   tgtcctgcac cctccctgct cgggcccctt ctgtactcag caacacaccc agcaccagca 3060
   ttcccactgc tcctgaggtc tgcaggcagc tcgctgtagc ctgagcggtg tggagggaag     3120
   tgtcctggga gacttaaaat gtgggaggtg ggaggggggg aggttgggcc ctgtgggcct     3180
   gcccaccccg tgtgcctgca tggagcccca gtgctgctca gccgtgcccc cgccgcaggg     3240
   gtcaggtcac tttcccgtcc tgggggttat tatgaccgtt gtcattttca ttgccatttt     3300
   tgctacccta actgggcagc aggtgcttgc agagccctcg ataccgacca ggtcccccct     3360
   cggagctcca cctgaacccc gtgtcaccct tgccccagcc tgcagaggat ggggtcactg     3420
   cagagatccc ttcacccaag gccacggtca catggtttgg aggagctggt gcccaaggca     3480
   gaggccaccc tctaggacac acctgtcccc agtgctggct ctgacctgcc cttgtctaag     3540
   aggctgaccc cggaagtgtt cctggcactg gcagccagcc tgacccagag tccagacacc     3600
   cacctgtgcc cccacttctg gggtctacca ggaaccgtct aggcccagag ggggacttcc     3660
   tgcttggccc cggatggaag aaggcctcct attgtcctcg tagaggaagc caccccgggg     3720
   cccggggatg agccaagtag gattccggga acctcgtggc tgggggcccg gcccgggctg     3780
   gctggctggc acgcctcctg tataaggccc cgagcccgct gtctcagccc tccg           3834
<210> 101
   <211> 21
   <212> PRT
   <213> artificial
<220>
   <223> alpha-1 lactalbumin secretory signal
<400> 101
   Met Met Ser Phe Val Ser Leu Leu Leu Val Gly Ile Leu Phe His Ala
   1               5                   10                  15
   Thr Gln Ala Val Asn
               20
<210> 102
   <211> 16
   <212> PRT
   <213> artificial
<220>
   <223> alpha S1-casein secretory signal
<400> 102
   Met Lys Leu Leu Ile Leu Thr Cys Ile Val Ala Val Ala Ala Arg Leu
   1               5                   10                  15
<210> 103
   <211> 16
   <212> PRT
   <213> artificial
<220>
   <223> beta lactoglobulin signal peptide
<400> 103
   Met Lys Cys Leu Leu Leu Ala Leu Ala Leu Thr Cys Gly Ala Gln Ala
   1               5                   10                  15
<210> 104
   <211> 162
   <212> PRT
   <213> Macropus eugenii
<400> 104
   Met Arg Gly Leu Thr Met Gln Val Leu Leu Leu Val Leu Gly Leu Leu
   1               5                   10                  15
   Ser Leu Met Thr Pro Leu Gly Tyr Ala Gln Asp Gln Pro Tyr Gln Asp
               20                  25                  30
   Val Leu Asn Arg Phe Ile Gln Glu Tyr Asn Thr Lys Ser Glu Ser Glu
           35                  40                  45
   Ser Leu Phe Arg Leu Ser Val Leu Asn Leu Pro Ser Gln Glu Ser Asn
       50                  55                  60
   Asp Pro Thr Ala Pro Gln Leu Leu Lys Phe Thr Ile Arg Glu Thr Val
   65                  70                  75                  80
   Cys Ser Lys Ser Glu His Arg Asn Pro Glu Glu Cys Asp Phe Lys Lys
                   85                  90                  95
   Asn Gly Leu Val Glu Glu Cys Ile Gly Thr Val Asp Leu Asp Ser Ser
               100                 105                 110
   Ser Pro Ser Val Asp Ile Ser Cys Asp Gly Pro Glu Lys Val Lys Arg
           115                 120                 125
   Gly Phe Gly Lys Lys Leu Arg Lys Arg Leu Lys Lys Phe Arg Asn Ser
       130                 135                 140
   Ile Lys Lys Arg Leu Lys Asn Phe Asn Val Val Ile Pro Ile Pro Leu
   145                 150                 155                 160
   Pro Gly
<210> 105
   <211> 161
   <212> PRT
   <213> Macropus eugenii
<400> 105
   Met Arg Asp Ser Thr Met Gln Val Leu Leu Leu Val Leu Gly Leu Leu
   1               5                   10                  15
   Ser Leu Met Thr Ser Leu Ala Cys Ala Gln Asp Gln Pro Tyr Gln Asp
               20                  25                  30
   Val Leu Asn Arg Phe Ile Gln Glu Tyr Asn Thr Lys Ser Glu Ser Glu
           35                  40                  45
   Ser Leu Phe Arg Leu Ser Val Leu Asn Leu Pro Pro Glu Glu Ser Asn
       50                  55                  60
   Asp Pro Ala Val Pro Leu Leu Lys Phe Thr Ile Arg Glu Thr Val Cys
   65                  70                  75                  80
   Pro Lys Thr Glu His Arg Asn Ala Asp Glu Cys Asp Phe Lys Lys Asn
                   85                  90                  95
   Gly Leu Val Lys Gln Cys Ile Gly Thr Ile Asp Leu Asp Ser Pro Asn
               100                 105                 110
   Pro Ser Val Asp Ile Ser Cys Asp Arg Pro Ala Lys Val Lys Arg Gly
           115                 120                 125
   Leu Trp Glu Ser Leu Lys Arg Lys Ala Thr Lys Leu Gly Asp Asp Ile
       130                 135                 140
   Arg Asn Thr Leu Arg Asn Phe Lys Ile Lys Phe Pro Val Pro Arg Gln
   145                 150                 155                 160
   Gly
<210> 106
   <211> 155
   <212> PRT
   <213> Bos taurus
<400> 106
   Met Glu Thr Pro Arg Ala Ser Leu Ser Leu Gly Arg Trp Ser Leu Trp
   1               5                   10                  15
   Leu Leu Leu Leu Gly Leu Ala Leu Pro Ser Ala Ser Ala Gln Ala Leu
               20                  25                  30
   Ser Tyr Arg Glu Ala Val Leu Arg Ala Val Asp Gln Leu Asn Glu Gln
           35                  40                  45
   Ser Ser Glu Pro Asn Ile Tyr Arg Leu Leu Glu Leu Asp Gln Pro Pro
       50                  55                  60
   Gln Asp Asp Glu Asp Pro Asp Ser Pro Lys Arg Val Ser Phe Arg Val
   65                  70                  75                  80
   Lys Glu Thr Val Cys Ser Arg Thr Thr Gln Gln Pro Pro Glu Gln Cys
                   85                  90                  95
   Asp Phe Lys Glu Asn Gly Leu Leu Lys Arg Cys Glu Gly Thr Val Thr
               100                 105                 110
   Leu Asp Gln Val Arg Gly Asn Phe Asp Ile Thr Cys Asn Asn His Gln
           115                 120                 125
   Ser Ile Arg Ile Thr Lys Gln Pro Trp Ala Pro Pro Gln Ala Ala Arg
       130                 135                 140
   Leu Cys Arg Ile Val Val Ile Arg Val Cys Arg
   145                 150                 155
<210> 107
   <211> 176
   <212> PRT
   <213> Bos taurus
<400> 107
   Met Glu Thr Gln Arg Ala Ser Leu Ser Leu Gly Arg Cys Ser Leu Trp
   1 5 10 15
   Leu Leu Leu Leu Gly Leu Val Leu Pro Ser Ala Ser Ala Gln Ala Leu
   20 25 30
   Ser Tyr Arg Glu Ala Val Leu Arg Ala Val Asp Gln Phe Asn Glu Arg
   35 40 45
   Ser Ser Glu Ala Asn Leu Tyr Arg Leu Leu Glu Leu Asp Pro Thr Pro
   50 55 60
   Asn Asp Asp Leu Asp Pro Gly Thr Arg Lys Pro Val Ser Phe Arg Val
   65 70 75 80
   Lys Glu Thr Asp Cys Pro Arg Thr Ser Gln Gln Pro Leu Glu Gln Cys
   85 90 95
   Asp Phe Lys Glu Asn Gly Leu Val Lys Gln Cys Val Gly Thr Val Thr
   100 105 110
   Leu Asp Pro Ser Asn Asp Gln Phe Asp Ile Asn Cys Asn Glu Leu Gln
   115 120 125
   Ser Val Arg Phe Arg Pro Pro Ile Arg Arg Pro Pro Ile Arg Pro Pro
   130 135 140
   Phe Tyr Pro Pro Phe Arg Pro Pro Ile Arg Pro Pro Ile Phe Pro Pro
   145 150 155 160
   Ile Arg Pro Pro Phe Arg Pro Pro Leu Gly Pro Phe Pro Gly Arg Arg
   165 170 175
<210> 108
   <211> 144
   <212> PRT
   <213> Bos taurus
<400> 108
   Met Gln Thr Gln Arg Ala Ser Leu Ser Leu Gly Arg Trp Ser Leu Trp
   1 5 10 15
   Leu Leu Leu Leu Gly Leu Val Val Pro Ser Ala Ser Ala Gln Ala Leu
   20 25 30
   Ser Tyr Arg Glu Ala Val Leu Arg Ala Val Asp Gln Leu Asn Glu Leu
   35 40 45
   Ser Ser Glu Ala Asn Leu Tyr Arg Leu Leu Glu Leu Asp Pro Pro Pro
   50 55 60
   Lys Asp Asn Glu Asp Leu Gly Thr Arg Lys Pro Val Ser Phe Thr Val
   65 70 75 80
   Lys Glu Thr Val Cys Pro Arg Thr Ile Gln Gln Pro Ala Glu Gln Cys
   85 90 95
   Asp Phe Lys Glu Lys Gly Arg Val Lys Gln Cys Val Gly Thr Val Thr
   100 105 110
   Leu Asp Pro Ser Asn Asp Gln Phe Asp Leu Asn Cys Asn Glu Leu Gln
   115 120 125
   Ser Val Ile Leu Pro Trp Lys Trp Pro Trp Trp Pro Trp Arg Arg Gly
   130 135 140
<210> 109
   <211> 159
   <212> PRT
   <213> Bos taurus
<400> 109
   Met Glu Thr Gln Arg Ala Ser Leu Ser Leu Gly Arg Trp Ser Leu Trp
   1 5 10 15
   Leu Leu Leu Leu Gly Leu Ala Leu Pro Ser Ala Ser Ala Gln Ala Leu
   20 25 30
   Ser Tyr Arg Glu Ala Val Leu Arg Ala Val Asp Gln Leu Asn Glu Lys
   35 40 45
   Ser Ser Glu Ala Asn Leu Tyr Arg Leu Leu Glu Leu Asp Pro Pro Pro
   50 55 60
   Lys Glu Asp Asp Glu Asn Pro Asn Ile Pro Lys Pro Val Ser Phe Arg
   65 70 75 80
   Val Lys Glu Thr Val Cys Pro Arg Thr Ser Gln Gln Ser Pro Glu Gln
   85 90 95
   Cys Asp Phe Lys Glu Asn Gly Leu Leu Lys Glu Cys Val Gly Thr Val
   100 105 110
   Thr Leu Asp Gln Val Gly Ser Asn Phe Asp Ile Thr Cys Ala Val Pro
   115 120 125
   Gln Ser Val Gly Gly Leu Arg Ser Leu Gly Arg Lys Ile Leu Arg Ala
   130 135 140
   Trp Lys Lys Tyr Gly Pro Ile Ile Val Pro Ile Ile Arg Ile Gly
   145 150 155
<210> 110
   <211> 158
   <212> PRT
   <213> Bos taurus
<400> 110
   Met Glu Thr Gln Arg Ala Ser Leu Ser Leu Gly Arg Trp Ser Leu Trp
   1 5 10 15
   Leu Leu Leu Leu Gly Leu Ala Leu Pro Ser Ala Ser Ala Gln Ala Leu
   20 25 30
   Ser Tyr Arg Glu Ala Val Leu Arg Ala Val Asp Gln Phe Asn Glu Arg
   35 40 45
   Ser Ser Glu Ala Asn Leu Tyr Arg Leu Leu Glu Leu Asp Pro Pro Pro
   50 55 60
   Lys Glu Asp Asp Glu Asn Pro Asn Ile Pro Lys Pro Val Ser Phe Arg
   65 70 75 80
   Val Lys Glu Thr Val Cys Pro Arg Thr Ser Gln Gln Pro Ala Glu Gln
                   85                  90                  95
   Cys Asp Phe Lys Glu Asn Gly Leu Val Lys Gln Cys Val Gly Thr Val
               100                 105                 110
   Thr Leu Asp Ala Val Lys Gly Lys Ile Asn Val Thr Cys Glu Glu Leu
           115                 120                 125
   Gln Ser Val Gly Arg Phe Lys Arg Phe Arg Lys Lys Phe Lys Lys Leu
       130                 135                 140
   Phe Lys Lys Leu Ser Pro Val Ile Pro Leu Leu His Leu Gly
   145                 150                 155
<210> 111
   <211> 165
   <212> PRT
   <213> Bos taurus
<400> 111
   Met Glu Thr Gln Arg Ala Ser Phe Ser Leu Gly Arg Ser Ser Leu Trp
   1               5                   10                  15
   Leu Leu Leu Leu Gly Leu Val Val Pro Ser Ala Ser Ala Gln Asp Leu
               20                  25                  30
   Ser Tyr Arg Glu Ala Val Leu Arg Ala Val Asp Gln Phe Asn Glu Arg
           35                  40                  45
   Ser Ser Glu Ala Asn Leu Tyr Arg Leu Leu Glu Leu Asp Pro Pro Pro
       50                  55                  60
   Glu Gln Asp Val Glu His Pro Gly Ala Arg Lys Pro Val Ser Phe Thr
   65                  70                  75                  80
   Val Lys Glu Thr Val Cys Pro Arg Thr Thr Pro Gln Pro Pro Glu Gln
                   85                  90                  95
   Cys Asp Phe Lys Glu Asn Gly Leu Val Lys Gln Cys Val Gly Thr Val
               100                 105                 110
   Thr Arg Tyr Trp Ile Arg Gly Asp Phe Asp Ile Thr Cys Asn Asn Ile
           115                 120                 125
   Gln Ser Ala Gly Leu Phe Arg Arg Leu Arg Asp Ser Ile Arg Arg Gly
       130                 135                 140
   Gln Gln Lys Ile Leu Glu Lys Ala Arg Arg Ile Gly Glu Arg Ile Lys
   145                 150                 155                 160
   Asp Ile Phe Arg Gly
                   165
<210> 112
   <211> 5
   <212> PRT
   <213> artificial
<220>
   <223> enterokinase cleavage signal
<400> 112
   Asp Asp Asp Asp Lys
   1 5
<210> 113
   <211> 15
   <212> DNA
   <213> artificial
<220>
   <223> Oligonucleotide for overlap extension designated MC1
<400> 113
   aagcgaggay ttkgg 15
<210> 114
   <211> 23
   <212> DNA
   <213> artificial
<220>
   <223> Oligonucleotide for overlap extension designated MC2
<400> 114
   ytcytgagac tctcccmaar tcc 23
<210> 115
   <211> 24
   <212> DNA
   <213> artificial
<220>
   <223> Oligonucleotide for overlap extension designated MC3
<400> 115
   tcargargag actgaagaaa yttr 24
<210> 116
   <211> 26
   <212> DNA
   <213> artificial
<220>
   <223> Oligonucleotide for overlap extension designated MC4
<400> 116
   tmttcytaat atcatcccya artttc 26
<210> 117
   <211> 22
   <212> DNA
   <213> artificial
<220>
   <223> Oligonucleotide for overlap extension designated MC5
<400> 117
   argaakgatt aaagaacttt aa 22
<210> 118
   <211> 24
   <212> DNA
   <213> artificial
<220>
   <223> Oligonucleotide for overlap extension designated MC6
<400> 118
   aytggaaatt taattttaaa gttc 24
<210> 119
   <211> 16
   <212> DNA
   <213> artificial
<220>
   <223> Oligonucleotide for overlap extension designated MC7
<400> 119
   ttccartccc ackgcm 16
<210> 120
   <211> 12
   <212> DNA
   <213> artificial
<220>
   <223> Oligonucleotide for overlap extension designated MC8
<400> 120
   ctatccckgc mg 12
<210> 121
   <211> 25
   <212> DNA
   <213> artificial
<220>
   <223> Oligonucleotide for overlap extension designated MC9
<400> 121
   catgccatgc aagcgaggay ttkgg 25
<210> 122
   <211> 26
   <212> DNA
   <213> artificial
<220>
   <223> Oligonucleotide for overlap extension designated MC10
<400> 122
   taagagctcc tatccckgcm gtggga 26
<210> 123
   <211> 19
   <212> DNA
   <213> artificial
<220>
   <223> Oligonucleotide for overlap extension designated MC17F
<400> 123
   aagagaggat ttgggaaga 19
<210> 124
   <211> 58
   <212> DNA
   <213> artificial
<220>
   <223> Oligonucleotide for overlap extension designated MC18R
<400> 124
   taatactatt cctaaatttc ktcrstytcy tcytgagmyt cttcccaaat cctctctt 58
<210> 125
   <211> 42
   <212> DNA
   <213> artificial
<220>
   <223> Oligonucleotide for overlap extension designated MC19F
<400> 125
   gaaatttagg aatagtatta rgaakasatt aaagaacttt aa 42
<210> 126
   <211> 46
   <212> DNA
   <213> artificial
<220>
   <223> Oligonucleotide for overlap extension designated MC20R
<400> 126
   ctatccckgc mgtgggaytg gaawtwyaay kttaaagttc tttaat 46
<210> 127
   <211> 27
   <212> DNA
   <213> artificial
<220>
   <223> Oligonucleotide for overlap extension designated MC9-1
<400> 127
   catgccatgg tgaagagagg ayttkgg 27
<210> 128
   <211> 26
   <212> DNA
   <213> artificial
<220>
   <223> Oligonucleotide for overlap extension designated MC10-1
<400> 128
   taactcgagc tatccckgcm gtggga 26

## Claims

1. An expression construct, or an expression vector or a virus particle comprising said expression construct, wherein said expression construct comprises a nucleic acid encoding a peptide, said peptide comprises an amino acid sequence selected from SEQ ID NOs: 7, 8, 10 to 32 or 36 to 58 and having antimicrobial activity at least against *Streptococcus uberis,* and wherein said nucleic acid is operably linked to a promoter that confers expression on said nucleic acid in a mammary gland or cell or tissue thereof, wherein the promoter is selected from the group consisting of a β-casein gene promoter, a prolactin-inducible mammary specific promoter, an α-lactalbumin gene promoter, a whey acidic protein (WAP) gene promoter and a nuclear factor-κB (NF-κB) responsive promoter.

2. The expression construct, vector or virus particle according to claim 1, wherein the promoter confers increased expression on the nucleic acid in a mammary gland or cell or tissue thereof during involution and/or mastitis and/or an infection in a mammary gland or cell or tissue thereof at least by *Streptococcus uberis,* and wherein the promoter is an NF-κB responsive promoter, and preferably wherein the promoter is derived from a lactoferrin gene such as a bovine lactoferrin gene.

3. The expression construct, vector or virus particle according to claim 1 or 2, wherein said construct further comprising one or more sequence(s) selected from:
(i) an intron sequence;
(ii) a sequence encoding a signal peptide for use in directing secretion of the antimicrobial peptide into milk; and
(iii) a sequence encoding a prepro sequence of a cathelicidin protein.

4. The expression construct, vector or virus particle according to claim 1 or 3, wherein said construct further comprising a sequence encoding a protease recognition sequence positioned between (i) a sequence encoding a prepro sequence of a cathelicidin protein and a sequence encoding the peptide, and/or (ii) a sequence encoding a signal peptide and a sequence encoding the peptide.

5. The expression construct, vector or virus particle according to claim 1 or 4, wherein said construct further comprising a sequence encoding a fusion protein between a plurality of the peptides.

6. The expression construct, vector or virus particle according to any one of claims 1 to 5, wherein the peptide has antimicrobial activity against *S*. *uberis* and a further organism selected from *S. suis, S. agalactiae, P. aeruginosa, E. coli, S. aureus*, *S. schleifen* subsp. coagulans, *S*. *schleiferi, S. epidermis*, *S. pseudointermedin, Mannheimia haemolytica (P. haemolytica), P. multocida, A. pleuropneumoniae* (APP), *H. somnus, Salmonella choleraesuis* and *B. bronchiseptica* and any combinations thereof.

7. The expression construct, vector or virus particle according to any one of claims 1 to 6, wherein the peptide has low activity against one or more lactobacilli.

8. The expression construct, vector or virus particle according to any one of claims 1 to 7, wherein the peptide comprises an amino acid sequence selected from SEQ ID NOs: 59 to 83.

9. An isolated genetically-modified non-human cell comprising the expression construct or the expression vector or the virus particle according to any one of claims 1 to 8, preferably wherein the cell is a non-human mammalian cell.

10. An ex *vivo* method of producing a genetically-modified non-human mammalian cell, said method comprising introducing into a non-human mammalian cell the expression construct or the expression vector according to any one of claims 1 to 8.

11. The method of claim 10, wherein the non-human mammalian cell is an isolated fertilized oocyte or a somatic cell.

12. The method of claim 10 or 11, wherein the non-human mammalian cell is a somatic cell and said method further comprises injecting *ex vivo* or fusing *ex vivo* a somatic cell comprising the expression construct or vector or virus particle with an enucleated mature oocyte from the same species of non-human mammal as the somatic cell to thereby produce a genetically-modified non-human mammalian monocell embryo.

13. A genetically-modified non-human mammal comprising the expression construct or expression vector or virus particle according to any one of claims 1 to 8 or a zygote, embryo, offspring or reproductive material thereof comprising said expression construct or expression vector or virus particle.

14. A method for producing an antimicrobial peptide, said method comprising:
(i) producing or obtaining a genetically-modified non-human mammal capable of expressing the peptide and wherein said mammal comprises the expression construct, vector or viral particle according to any one of claims 1 to 8; and
(ii) maintaining the genetically-modified non-human mammal for a time and under conditions sufficient for the antimicrobial peptide to be expressed,
thereby producing the antimicrobial peptide.

15. The method according to claim 14 comprising obtaining or producing a genetically-modified non-human mammal that secretes the antimicrobial peptide into milk, and maintaining the genetically-modified non-human mammal for a time and under conditions sufficient for lactation to occur to thereby produce milk comprising said antimicrobial peptide, and preferably additionally comprising isolating the antimicrobial peptide from milk or a secretion thereof produced by the mammal.

16. The expression construct or the expression vector or a virus particle according to any one of claims 1 to 8, for use in the treatment or prevention of mastitis and/or an infection in a mammary gland or cell or tissue thereof by:
(i) *S. uberis;* or
(ii) *S. uberis* and at least one or more further organism(s) selected from the group consisting of *S*. *aureus*, *S. agalactiae* and *E. coli.*

## Patentansprüche

1. Expressionskonstrukt oder Expressionsvektor oder Viruspartikel, umfassend das Expressionskonstrukt, wobei das Expressionskonstrukt eine für ein Peptid codierende Nukleinsäure umfasst, wobei das Peptid eine aus SEQ ID NO: 7, 8, 10 bis 32 oder 36 bis 58 ausgewählte Aminosäuresequenz umfasst und wenigstens gegen *Streptococcus uberis* antimikrobielle Aktivität aufweist und wobei die Nukleinsäure in operativer Verknüpfung mit einem Promotor steht, der der Nukleinsäure Expression in einer Brustdrüse oder einer Zelle oder einem Gewebe davon verleiht, wobei der Promotor aus der aus einem Promotor des β-Casein-Gens, einem mit Prolactin induzierbaren brustspezifischen Promotor, einem Promotor des α-Lactalbumin-Gens, einem Promotor des WAP (Whey Acidic Protein)-Gens und einem auf nukleären Faktor κB (NF-κB) reagierenden Promotor bestehenden Gruppe ausgewählt ist.

2. Expressionskonstrukt, Vektor oder Viruspartikel nach Anspruch 1, wobei der Promotor der Nukleinsäure erhöhte Expression in einer Brustdrüse oder einer Zelle oder einem Gewebe davon während Involution und/oder Mastitis und/oder einer Infektion wenigstens mit *Streptococcus uberis* in einer Brustdrüse oder einer Zelle oder einem Gewebe davon verleiht und wobei es sich bei dem Promotor um einen auf NF-κB reagierenden Promotor handelt und vorzugsweise wobei der Promotor von einem Lactoferrin-Gen wie etwa einem Rinder-Lactoferrin-Gen stammt.

3. Expressionskonstrukt, Vektor oder Viruspartikel nach Anspruch 1 oder 2, wobei das Konstrukt ferner eine oder mehrere Sequenz(en) umfasst, die ausgewählt sind aus:
(i) einer Intron-Sequenz;
(ii) einer für ein Signalpeptid zur Verwendung bei der Steuerung der Sekretion des antimikrobiellen Peptids in Milch codierenden Sequenz; und
(iii) einer für eine Präprosequenz eines Cathelicidin-Proteins codierenden Sequenz.

4. Expressionskonstrukt, Vektor oder Viruspartikel nach Anspruch 1 oder 3, wobei das Konstrukt ferner eine für eine zwischen (i) einer für eine Präprosequenz eines Cathelicidin-Proteins codierenden Sequenz und einer für das Peptid codierenden Sequenz und/oder (ii) einer für ein Signalpeptid codierenden Sequenz und einer für das Peptid codierenden Sequenz liegende Protease-Erkennungssequenz codierende Sequenz umfasst.

5. Expressionskonstrukt, Vektor oder Viruspartikel nach Anspruch 1 oder 4, wobei das Konstrukt ferner eine für ein Fusionsprotein zwischen mehreren der Peptide codierende Sequenz umfasst.

6. Expressionskonstrukt, Vektor oder Viruspartikel nach einem der Ansprüche 1 bis 5, wobei das Peptid antimikrobielle Aktivität gegen *S. uberis* sowie einen weiteren, aus *S. suis, S. agalactiae, P. aeruginosa, E. coli, S. aureus, S. schleifen* subsp. coagulans, *S. schleiferi, S. epidermis, S. pseudointermedin, Mannheimia haemolytica (P. haemolytica), P. multocida, A. pleuropneumoniae* (APP), *H. somnus, Salmonella choleraesuis* und *B. bronchiseptica* und beliebigen Kombinationen davon ausgewählten Organismus aufweist.

7. Expressionskonstrukt, Vektor oder Viruspartikel nach einem der Ansprüche 1 bis 6, wobei das Peptid geringe Aktivität gegen ein oder mehrere Lactobacilli aufweist.

8. Expressionskonstrukt, Vektor oder Viruspartikel nach einem der Ansprüche 1 bis 7, wobei das Peptid eine aus SEQ ID NO: 59 bis 83 ausgewählte Aminosäuresequenz umfasst.

9. Isolierte gentechnisch veränderte nichtmenschliche Zelle, umfassend das Expressionskonstrukt, den Expressionsvektor oder das Viruspartikel nach einem der Ansprüche 1 bis 8, vorzugsweise wobei es sich bei der Zelle um eine nichtmenschliche Säugerzelle handelt.

10. Ex-vivo-Verfahren zur Erzeugung einer gentechnisch veränderten nichtmenschlichen Säugerzelle, bei dem man das Expressionskonstrukt oder den Expressionsvektor nach einem der Ansprüche 1 bis 8 in eine nichtmenschliche Säugerzelle einführt.

11. Verfahren gemäß Anspruch 10, wobei es sich bei der nichtmenschlichen Säugerzelle um eine isolierte befruchtete Eizelle oder eine somatische Zelle handelt.

12. Verfahren gemäß Anspruch 10 oder 11, wobei es sich bei der nichtmenschlichen Säugerzelle um eine somatische Zelle handelt und wobei man bei dem Verfahren ferner ex vivo eine das Expressionskonstrukt, den Vektor oder das Viruspartikel umfassende somatische Zelle mit einer entkernten reifen Eizelle aus der gleichen nichtmenschlichen Säugerspezies wie der somatischen Zelle injiziert oder fusioniert, um somit einen gentechnisch veränderten nichtmenschlichen einzelligen Säugerembryo zu erzeugen.

13. Gentechnisch veränderter nichtmenschlicher Säuger, umfassend das Expressionskonstrukt oder den Expressionsvektor oder das Viruspartikel nach einem der Ansprüche 1 bis 8 oder eine Zygote, ein Embryo, ein Nachkomme oder reproduktives Material davon, umfassend das Expressionskonstrukt oder den Expressionsvektor oder das Viruspartikel.

14. Verfahren zur Erzeugung eines antimikrobiellen Peptids, wobei man bei dem Verfahren:
(i) einen gentechnisch veränderten nichtmenschlichen Säuger, der zur Expression des Peptids fähig ist, erzeugt oder erhält und wobei der Säuger das Expressionskonstrukt, den Vektor oder das Viruspartikel nach einem der Ansprüche 1 bis 8 umfasst; und
(ii) den gentechnisch veränderten nichtmenschlichen Säuger über einen Zeitraum und unter Bedingungen hält, der bzw. die für die Expression des antimikrobiellen Peptids hinreichend ist bzw. sind,
und somit das antimikrobielle Peptid erzeugt.

15. Verfahren nach Anspruch 14, bei dem man einen gentechnisch veränderten nichtmenschlichen Säuger, bei dem das antimikrobielle Peptid in Milch sezerniert wird, erhält oder erzeugt und den gentechnisch veränderten nichtmenschlichen Säuger über einen Zeitraum und unter Bedingungen hält, der bzw. die für Auftreten von Laktation hinreichend ist bzw. sind, um somit das antimikrobielle Peptid enthaltende Milch zu produzieren, und bei dem man vorzugsweise zusätzlich das antimikrobielle Peptid aus Milch oder einem von dem Säuger produzierten Sekret davon isoliert.

16. Expressionskonstrukt oder Expressionsvektor oder Viruspartikel nach einem der Ansprüche 1 bis 8 zur Verwendung bei der Behandlung oder Vorbeugung von Mastitis und/oder einer Infektion in einer Brustdrüse oder einer Zelle oder einem Gewebe davon mit:
(i) *S. uberis;* oder
(ii) *S. uberis* und wenigstens einem weiteren Organismus oder mehreren weiteren Organismen, ausgewählt aus der aus *S. aureus, S. agalactiae* und *E. coli* bestehenden Gruppe.

## Revendications

1. Construit d'expression ou bien vecteur d'expression ou particule virale comprenant ledit construit d'expression, dans laquelle ledit construit d'expression comprend un acide nucléique codant pour un peptide, ledit peptide comprend une séquence d'acides aminés choisie parmi les SEQ ID n° : 7, 8, 10 à 32 ou 36 à 58 et ayant une activité antimicrobienne au moins contre *Streptococcus uberis,* et dans laquelle ledit acide nucléique est lié, de manière opérationnelle, à un promoteur qui confère une expression au dit acide nucléique dans une glande mammaire ou bien une cellule ou une tissu de celle-ci, dans laquelle le promoteur est choisi dans le groupe constitué par un promoteur du gène de la β-caséine, un promoteur spécifiquement mammaire inductible par la prolactine, un promoteur du gène de la α-lactalbumine, un promoteur du gène de la protéine de lactosérum acide (WAP) et un promoteur sensible au facteur nucléaire κB (NF-κB).

2. Construit d'expression, vecteur ou particule virale selon la revendication 1, dans laquelle le promoteur confère à l'acide nucléique une expression accrue dans une glande mammaire ou bien une cellule ou un tissu de celle-ci lors de l'involution et/ou d'une mastite et/ou d'une infection dans une glande mammaire ou bien une cellule ou un tissu de celle-ci au moins par *Streptococcus uberis,* et dans laquelle le promoteur est un promoteur sensible au NF-κB, et, de préférence, dans laquelle le promoteur est dérivé d'un gène de lactoferrine, tel qu'un gène de la lactoferrine bovine.

3. Construit d'expression, vecteur ou particule virale selon la revendication 1 ou la 2, dans laquelle ledit construit comprend en outre une ou plusieurs séquence(s) choisie(s) parmi :
(i) une séquence d'intron ;
(ii) une séquence codant pour un peptide signal destiné à être utilisé pour orienter la sécrétion du peptide antimicrobien dans le lait ; et
(iii) une séquence codant pour une séquence prépro d'une protéine de cathélicidine.

4. Construit d'expression, vecteur ou particule virale selon la revendication 1 ou la 3, dans laquelle ledit construit comprend en outre une séquence codant pour une séquence de reconnaissance d'une protéase, positionnée entre (i) une séquence qui code pour une séquence prépro d'une protéine de cathélicidine et une séquence codant pour le peptide, et/ou (ii) une séquence qui code pour un peptide signal et une séquence codant pour le peptide.

5. Construit d'expression, vecteur ou particule virale selon la revendication 1 ou la 4, dans laquelle ledit construit comprend en outre une séquence codant pour une protéine de fusion entre une pluralité de peptides.

6. Construit d'expression, vecteur ou particule virale selon l'une quelconque des revendications 1 à 5, dans laquelle le peptide a une activité antimicrobienne contre *S. uberis* et un organisme supplémentaire, choisi dans le groupe constitué par *S. suis, S. agalactiae, P. aeruginosa, E. coli, S. aureus, S. schleifen* subsp. coagulans, *S. schleiferi, S. epidermis, S. pseudointermedin, Mannheimia haemolytica (P. haemolytica), P. multocida, A. pleuropneumoniae (APP), H. somnus, Salmonella choleraesuis* et *B. bronchiseptica* ainsi que n'importe quelle combinaison de ceux-ci.

7. Construit d'expression, vecteur ou particule virale selon l'une quelconque des revendications 1 à 6, dans laquelle le peptide a une activité faible contre un ou plusieurs lactobacille(s).

8. Construit d'expression, vecteur ou particule virale selon l'une quelconque des revendications 1 à 7, dans laquelle le peptide comprend une séquence d'acides aminés choisie parmi les SEQ ID n° : 59 à 83.

9. Cellule non humaine isolée, modifiée par génie génétique comprenant le construit d'expression, le vecteur d'expression ou la particule virale, selon l'une quelconque des revendications 1 à 8, de préférence dans laquelle la cellule est une cellule de mammifère non humain.

10. Procédé *ex vivo* de production d'une cellule mammalienne non humaine, modifiée par génie génétique, ledit procédé comprenant l'introduction, dans une cellule mammalienne non humaine, du construit d'expression ou du vecteur d'expression selon l'une quelconque des revendications 1 à 8.

11. Procédé selon la revendication 10, dans laquelle la cellule mammalienne non humaine est un ovocyte fécondé isolé ou une cellule somatique.

12. Procédé selon la revendication 10 ou la 11, dans laquelle la cellule mammalienne non humaine est une cellule somatique et ledit procédé comprend en outre l'injection *ex vivo* ou la fusion *ex vivo* d'une cellule somatique, comprenant le construit ou bien le vecteur d'expression ou la particule virale, avec un ovocyte mature énucléé, provenant de la même espèce mammalienne non humaine que la cellule somatique, pour produire de ce fait un embryon monocellulaire mammalien non humain, génétiquement modifié.

13. Mammifère non humain génétiquement modifié comprenant le construit d'expression, le vecteur d'expression ou la particule virale, selon l'une quelconque des revendications 1 à 8, ou bien un zygote, un embryon ou bien une progéniture ou du matériel de reproduction de ceux-ci comprenant ledit construit d'expression ou bien ledit vecteur d'expression ou ladite particule virale.

14. Procédé de production d'un peptide antimicrobien, ledit procédé comprenant les étapes consistant à :
(i) produire ou obtenir un mammifère non humain génétiquement modifié étant capable d'exprimer le peptide et dans laquelle ledit mammifère comprend le construit d'expression, le vecteur ou la particule virale, selon l'une quelconque des revendications 1 à 8 ; et
(ii) maintenir le mammifère non humain génétiquement modifié pendant un laps de temps et dans des conditions qui sont suffisants pour que le peptide antimicrobien soit exprimé,
en produisant de ce fait le peptide antimicrobien.

15. Procédé, selon la revendication 14, comprenant les étapes consistant à obtenir ou produire un mammifère non humain génétiquement modifié qui sécrète le peptide antimicrobien dans le lait, et maintenir le mammifère non humain génétiquement modifié pendant un laps de temps et dans des conditions qui sont suffisants pour qu'ait lieu la lactation, pour produire de ce fait du lait qui comprend ledit peptide antimicrobien, et, de préférence, comprenant en outre l'isolement du peptide antimicrobien à partir du lait ou d'une sécrétion de celui-ci produit(e) par le mammifère.

16. Construit d'expression ou bien vecteur d'expression ou particule virale, selon l'une quelconque des revendications 1 à 8, destiné (e) à être utilisé (e) dans le traitement ou la prévention d'une mastite et/ou d'une infection dans une glande mammaire ou bien une cellule ou un tissu de celle-ci par :
(i) *S. uberis ;* ou
(ii) *S. uberis* et au moins un ou plusieurs organisme(s) en outre, choisi (s) dans le groupe constitué par *S. aureus, S. agalactiae* et *E. coli.*
